(19)

**Europäisches Patentamt**
**European Patent Office**
**Office européen des brevets**

(11) **EP 4 679 444 A2**

(12) **EUROPEAN PATENT APPLICATION**

(43) Date of publication:
**14.01.2026   Bulletin 2026/03**

(21) Application number: **25219215.8**

(22) Date of filing: **29.10.2021**

(51) International Patent Classification (IPC):
***G16H 20/40*** *(2018.01)*

(52) Cooperative Patent Classification (CPC):
**A61M 1/1603; A61M 1/3403; A61M 1/3612;**
**G16H 20/40;** A61M 1/342; A61M 1/3672;
A61M 2205/3334; A61M 2230/208

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **03.11.2020   IT 202000026212**

(62) Document number(s) of the earlier application(s) in
accordance with Art. 76 EPC:
**21801548.5 / 4 240 440**

(71) Applicant: **Gambro Lundia AB**
**226 43 Lund (SE)**

(72) Inventor: **POUCHOULIN, Dominique**
**F-01390 Tramoyes (FR)**

(74) Representative: **PGA S.p.A., Milano, Succursale**
**di Lugano**
**Via Castagnola, 21c**
**6900 Lugano (CH)**

Remarks:
This application was filed on 27.11.2025 as a
divisional application to the application mentioned
under INID code 62.

(54) **APPARATUS FOR EXTRACORPOREAL TREATMENT OF BLOOD CALCULATING FLOW RATES**

(57)     A CRRT apparatus comprising a control unit configured to execute a flow-rate setup procedure by receiving a patient prescription comprising clinical prescription parameters, by allowing entry of a set value for a prescribed dialysis dose ($D_{set}$) to be delivered, and of a target value for a parameter (nNBL; $Cp_{HCO3-pat}$) indicative of a steady state acid-base balance in the blood of the patient who has to undergo a CRRT blood treatment, and by determining operating parameters calculating a set value of relevant fluid flow rates including one or more of a fluid flow rate ($Q_{cit}$) through the anticoagulant infusion line, a fluid flow rate ($Q_{PBP}$) through the PBP infusion line, a fluid flow rate ($Q_{rep.pre}$) through the pre-dilution infusion line, a fluid flow rate ($Q_{rep.post}$) through the post-dilution infusion line, a fluid flow rate ($Q_{HCO3}$) through the post-dilution bicarbonate infusion line, a fluid flow rate ($Q_{ca}$) through the ion balancing infusion line, a blood fluid flow rate ($Q_b$) through the extracorporeal blood circuit, a fluid flow rate ($Q_{dial}$) through the dialysis liquid supply line, and a fluid flow rate ($Q_{eff}$) through the effluent fluid line, wherein calculating the set value of the fluid flow rates is based at least on the set value of the prescribed dialysis dose ($D_{set}$) and on the target value for the parameter (nNBL; $Cp_{HCO3\_pat}$) indicative of a steady state acid-base balance in the blood.

FIG.7

**Description**

**Technical Field**

The present invention relates to a medical apparatus for extracorporeal treatment of blood. The present invention also relates to a process of calculating set flow rates in a medical apparatus for extracorporeal fluid processing.

In more detail, the present invention is applicable in the context of continuous renal replacement therapies (CRRT) with or without anticoagulation, for example CRRT with or without systemic anticoagulation (e.g., heparin) / with or without regional anticoagulation (e.g., citrate). In particular, the present invention may be advantageously used for administering regional citrate anticoagulation (RCA) during continuous renal replacement therapies (CRRT).

**Background of the Invention**

The kidneys fulfil many functions, including the removal of water, the excretion of catabolites (or waste from the metabolism, for example urea and creatinine), the regulation of the concentration of the electrolytes in the blood (e.g. sodium, potassium, magnesium, calcium, bicarbonates, phosphates, chlorides) and the regulation of the acid/base equilibrium within the body, which is obtained in particular by the removal of weak acids and by the production of ammonium salts. In individuals who have lost (temporarily or permanently) the use of their kidneys, since these excretion and regulation mechanisms no longer work, the body accumulates water and waste from the metabolism and exhibits an excess of electrolytes, as well as, in general, acidosis, the pH of the blood plasma shifting downwards, below 7.35 (the blood pH normally varies within narrow limits of between 7.35 and 7.45). As mentioned, in order to overcome renal dysfunction, resort is conventionally made to a blood treatment involving extracorporeal circulation through an exchanger having a semipermeable membrane (dialyzer) in which the patient's blood is circulated on one side of the membrane and a dialysis liquid, comprising the main electrolytes of the blood in concentrations close to those in the blood of a healthy subject, is circulated on the other side. Furthermore, a pressure difference is created between the two compartments of the dialyzer which are delimited by the semipermeable membrane, so that a fraction of the plasma fluid passes by ultrafiltration through the membrane into the compartment containing the dialysis liquid. The blood treatment which takes place in a dialyzer as regards waste from the metabolism and electrolytes results from two mechanisms of molecular transport through the membrane. On the one hand, the molecules migrate from the liquid where their concentration is higher to the liquid where their concentration is lower. This is diffusive transport. On the other hand, certain catabolites and certain electrolytes are entrained by the plasma fluid which filters through the membrane under the effect of the pressure difference created between the two compartments of the exchanger. This is convective transport. Three of the abovementioned functions of the kidney, namely the removal of water, the excretion of catabolites and the regulation of the electrolytic concentration of the blood, are therefore performed in a conventional blood treatment device by the combination of dialysis and blood filtration (this combination is referred to as hemodiafiltration). To perform one or more of the above described treatments, extracorporeal blood treatment equipment may comprise a plurality of lines for delivering fluid directly to the patient or into the extracorporeal blood circuit.

When setting up the machine, an operator usually imposes the blood pump flow rate, the individual flow rates for each of the infusion lines, the flow rate for the dialysis line and for the effluent line (in reality this latter may alternatively be calculated based on the set patient fluid removal rate). The set values for the flow rates on each line are used to control respective pumps: in other words, a plurality of pumps are used where each pump draws fluid from or supplies fluid to a respective fluid container according to the set flow rate value for the respective line. Setting up of the machine is therefore cumbersome as it entails the definition and entry on the part of the operator of a relatively high number of flow rates. Moreover, the independent setting of each one of the flow rates does not provide the operator with intuitive information in terms of medically relevant prescription parameters. Finally, the need for independently setting a plurality of parameters may be source of errors and does not allow to optimize fluid consumption.

WO 2013/030642 deals with a blood treatment apparatus and a process of setting up a medical apparatus for the delivery or collection of fluids, wherein a control unit is configured calculate set values of two or more of the fluid flow rates based on a fluid flow rate set by the operator and on a prescribed dose value. However, WO 2013/030642 does not focus on the acid-base balance management aspects and does not specifically address the issue of acid base balance in regional anticoagulation in the context of CRRT.

As regards the regulation of the acid/base equilibrium inside the body, the approach adopted to overcome renal deficiency is to act on a mechanism by which the acid/base equilibrium inside the body is regulated, this mechanism consisting of the buffer systems of the blood, the main one of which comprises carbonic acid, as a weak acid, associated with its alkali salt, bicarbonate. This is why, in order to correct acidosis in a patient suffering from renal insufficiency, he/she is administered with bicarbonate via the vascular route, directly or indirectly, during a hemodialysis session. In the field of renal treatment, continuous renal replacement therapy (CRRT) has been widely used in critically ill patients with acute kidney injury and anticoagulation of the extracorporeal blood is necessary to maintain the patency of the circuit. In recent decades, different anticoagulation strategies have been used in clinical settings and heparin is the most commonly used anticoagulant. Although heparin has the advantages of low cost, easy monitoring and simple reversal, it may increase bleeding. Additionally, there is the risk of heparin-induced thrombocytopenia type II that can result in life-threatening complications. Regional citrate anticoagulation (RCA), which was first introduced into clinical use in the early 1980s, has been recommended as the most suitable form of CRRT regional circuit anticoagulation and has been safely used even in patients with severe liver dysfunction. However, citrate infusion in critically ill patients impacts a variety of metabolic systems, which can lead to metabolic alkalosis, hypocalcaemia and citrate excessive load/toxicity. These potential disturbances may be partially resolved by careful monitoring, adherence to treatment protocols, and oversight by trained staff in clinical practice. Notwithstanding the above criticalities, citrate anticoagulation has become the preferred anticoagulation choice for continuous renal replacement therapies (CRRT) as minimizing patient bleeding risks (regional anticoagulation effect) and increasing extracorporeal blood circuit life time. While RCA has some limitations with respect to compatibility with 'large' blood flow rates, this is not a problem in CRRT where efficiency is primarily driven by the fluid exchange rate and where the vast majority of treatments are delivered at blood flow rate below 200 ml/min.

Fast metabolism of the citrate infused to the patient is part of the key mechanisms making RCA successful. Citrate metabolism produces energy, as well as bicarbonate and $CO_2$ while releasing complexed calcium. In the situation where large amounts of citrate are infused to the patient, large amounts of bicarbonate are produced, up to the point where metabolic alkalosis is generated. Citrate accumulation matches with the scenario where systemic citrate concentration is significantly increased. It can develop in two circumstances 'normal' citrate load combined to poor citrate metabolism, and 'normal' citrate metabolism combined to large citrate load. The first scenario is likely to lead to metabolic acidosis due to a low production rate of bicarbonate from citrate. The second scenario is considered and addressed in the present application, specifically in respect to CRRT therapies in the RCA context. Consequence of citrate accumulation is a need to increase total calcium concentration as to keep (systemic) ionised calcium within the physiological range. This can be achieved by increasing the calcium infusion rate. This problem is a transitory problem during initiation of the therapy, as a safe steady state can be reached after stabilization of systemic citrate concentration (6-8 hours). Discontinuation of the therapy may however lead to an episode of hypercalcemia (as citrate is metabolized and complex-bound calcium is released). In the clinical setting, citrate accumulation is diagnosed via the monitoring of total to ionised systemic calcium ratio (ratio > 2.5 indicating probable citrate accumulation). Further, once citrate enters the patient systemic circulation, it is metabolized to bicarbonate on a 1:3 basis; thus, 1 mmol citrate yields 3 mmol bicarbonate. In presence of high citrate load, large amounts of bicarbonate are produced with the risk of metabolic alkalosis. Therefore, though regional anticoagulation may highly alleviate the adverse effects of heparin, RCA imposes the need of proper monitoring the acid-base balance in the patient blood, to avoid severe risks of alkalosis.

EP0678301 relates to an artificial kidney for intensive care particularly adapted to treating people suffering temporarily from kidney failure following an accident or a surgical operation. As clarified in the prior art document, in addition to purifying plasma wastes (e.g. urea) and to remove excess water, the kidneys play an important role in maintaining the acid-base equilibrium of the blood. Since the final concentration of bicarbonate in the blood depends on the concentration of bicarbonate in the perfusion solution or in the dialysis liquid, on the respective flow rates thereof, and on the flow rate of the patient's blood through the membrane exchanger, the main problem at the basis of document EP0678301 is that the concentration of bicarbonate in the blood of the patient corresponds rarely exactly to the desired concentration. EP0678301 describes a blood treatment device including a dialyzer with two chambers separated by a membrane. A dialysis liquid container (that does not contain any bicarbonate) is connected to a fluid pump via a duct which runs to the second chamber of the dialyzer. Electromagnetic clamps are provided for connecting the container to either the dialyzer or to the blood circuit. A bubble trap is provided in the return line of the blood circuit. The bubble trap is linked to an infusion container containing a solution of bicarbonate. In accordance with EP0678301, the flow rate $Q_{HCO3}$ of the circulation pump is controlled as a function of the flow rate $Q_{OUT}$ of the dialysate pump regardless of the type of treatment being delivered to the patient either by the equation:

$$Q_{HCO3} = Q_{OUT} * [HCO_3]_{DES} / [HCO_3]_{SOL}$$

or by the equation:

$$Q_{HCO3} = Cl * [HCO_3]_{DES} / [HCO_3]_{SOL}$$

wherein:

$Q_{HCO3}$ is flow rate of the circulation pump;
$Q_{OUT}$ is the flow rate $Q_{OUT}$ of the dialysate pump;
$[HCO_3]_{DES}$ is the desired concentration of bicarbonate in the blood of the patient;

$[HCO_3]_{SOL}$ is the concentration of the solution in the container;
**CI** is the clearance of the dialyzer for bicarbonate.

Notably, this prior art is directed to proper adjustment of patient blood acid-base equilibrium, by using a specific control of post infusion of bicarbonate solution based on clearance/dialysate flow rate which exclusively works in the following dialysis machine configurations: HF with post dilution and HD(F) with post dilution. Therefore, the problem of proper acid-base management in configuration wherein citrate is infused pre-blood pump (e.g., regional anticoagulation systems) and/or bicarbonate containing solution is pre-infused remains unsolved.

As to adapting to specific patient condition, protocols may (by far not systematically) include guidelines for adjusting citrate infusion or dialysis fluid/replacement flow rates in case patient monitoring data evidence alkalosis or acidosis problems. When present, these guidelines appear largely empirical. In the case of acidosis, some literature report for the infusion of bicarbonate 'bolus'. Although some published protocols are derived from some upstream modelling, no parameter representative of the expected buffer balance from the therapy is made explicitly available, whatever 'original' protocol parameters are used or after these have been tuned further to patient monitoring data. As to date, anticipation and control of acid base-balance during CRRT, particularly when using regional citrate anticoagulation (RCA) remains a difficult question especially when intending to use non-proven prescriptions.

**SUMMARY**

In this situation, it is a general object of the present embodiments to offer a technical solution capable overcoming one or more of the above drawbacks.

More in detail, it is an object of the present embodiments to render available a CRRT medical apparatus for the extracorporeal treatment of blood and a process configured for providing assisted prescription, at the same time taking into consideration the proper acid-base blood balance, particularly in the context of regional anticoagulation. In detail, it is an aim of the present embodiments to allow for acid-balance control/management, wherein the system is also designed to vary buffer balance of the extracorporeal blood circuit in an easy, but safe and controlled way. It is an aim to provide an apparatus and a method allowing to properly calculate set flow rates keeping an eye towards a proper buffer balance and capable of reducing as much possible the actions required for setting up the apparatus.

Is a further object of aspects of the invention to define an apparatus and a process allowing the operator to set up a CRRT blood treatment apparatus using medically meaningful parameters, which may result in an easier to use user interface. In particular, it is an aim to provide an assisted prescription in a situation where prescription includes a parameter specifying a steady state acid-base balance target, parameter which is of specific importance in the case of citrate anticoagulation

It is an auxiliary object of the invention to offer a medical apparatus for the treatment of fluid and a process for calculating set flow rates in said apparatus which may facilitate flow rate setting before and during the treatmentand optimize the consumption of fluid with respect to the prescription target and the system constraints.

Another auxiliary object is an apparatus capable of controlling operating parameters in a safe manner.

At least one of the above objects is substantially reached by an apparatus according to one or more of the appended apparatus claims. One or more of the above objects is also substantially reached by a process according to any one of the appended process claims.

Apparatus and processes according to aspects of the invention are here below described.

A 1st independent aspect concerns a Continuous Renal Replacement Therapy (CRRT) apparatus comprising:

a filtration unit (2) having a primary chamber (3) and a secondary chamber (4) separated by a semi-permeable membrane (5);
an extracorporeal blood circuit (17) having a blood withdrawal line (6) connected to an inlet of the primary chamber (3), and a blood return line (7) connected to an outlet of the primary chamber (3), said extracorporeal blood circuit (17) being configured for connection to a patient cardiovascular system;
a blood pump (21) configured to control the flow of blood through the extracorporeal blood circuit (17);
an effluent fluid line (13) connected to an outlet of the secondary chamber (4);
one or more of further fluid lines selected in the group comprising:

a pre-dilution infusion line (29) connected at one end thereof to the blood withdrawal line (6),
a post-dilution infusion line (63) connected at one end thereof to the blood return line (7),
a post-dilution bicarbonate infusion line (23) connected at one end thereof to the blood return line (7),
a ion balancing infusion line (74) connected at one end thereof either to the blood return line (7) or to a patient catheter,
a dialysis liquid supply line (8) connected at one end thereof to the inlet of the secondary chamber (4),

a pre-blood pump - PBP - infusion line (52) connected at one end thereof to the blood withdrawal line in a region of the blood withdrawal line which is positioned in use upstream from the blood pump (21), an anticoagulant infusion line (51) connected at one end thereof to the blood withdrawal line in a region of the blood withdrawal line which is positioned in use upstream from the blood pump (21), and a syringe fluid line (22) connected at one end thereof to the blood withdrawal line,

actuators for regulating the flow of fluid (24, 25, 26, 31, 53, 54, 65, 75) through said fluid lines (23, 8, 13, 29, 52, 51, 63, 74);
a memory (16) storing one or more mathematical relations; and
a control unit (12) connected to the memory (16) and to the actuators, the control unit being configured to execute a flow-rate setup procedure comprising:

- receiving a patient prescription comprising clinical prescription parameters, the receiving step including:

  ◦ allowing entry of a target value for a parameter (nNBL; $Cp_{HCO3\_pat}$) indicative of a steady state acid-base balance in the blood of the patient who has to undergo a CRRT blood treatment,

- determining one or more operating parameters using said one or more mathematical relations, the determining of the operating parameters comprises calculating a set value of one or more fluid flow rates selected in the group including:

  • a fluid flow rate ($Q_{cit}$) through the anticoagulant infusion line (51),
  • a fluid flow rate ($Q_{PBP}$) through the PBP infusion line (52),
  • a fluid flow rate ($Q_{rep.pre}$) through the pre-dilution infusion line (29),
  • a fluid flow rate ($Q_{rep.post}$) through the post-dilution infusion line (63),
  • a fluid flow rate ($Q_{HCO3}$) through the post-dilution bicarbonate infusion line (23),
  • a fluid flow rate ($Q_{ca}$) through the ion balancing infusion line (74),
  • a blood fluid flow rate ($Q_b$) through the extracorporeal blood circuit (17),
  • a fluid flow rate ($Q_{syr}$) through a syringe fluid line (22),
  • a fluid flow rate ($Q_{dial}$) through the dialysis liquid supply line (8), and
  • a fluid flow rate ($Q_{eff}$) through the effluent fluid line (13),

wherein calculating the set value of one or more fluid flow rates is based at least on said target value for the parameter (nNBL; $Cp_{HCO3\_pat}$) indicative of a steady state acid-base balance in the blood.

In an aspect according to the previous aspect, receiving a patient prescription comprises allowing entry of a set value for a prescribed dialysis dose ($D_{set}$) to be delivered, and determining one or more operating parameters comprises determining at least two operating parameters, the determining of the operating parameters comprises calculating a set value of at least two fluid flow rates selected in the group, calculating the set value of at least two fluid flow rates is based at least on said set value of the prescribed dialysis dose ($D_{set}$) and on said target value for the parameter (nNBL; $Cp_{HCO3\_pat}$) indicative of a steady state acid-base balance in the blood.

A further independent aspect is directed to a process of setting up a Continuous Renal Replacement Therapy (CRRT) apparatus, the apparatus comprising:

a filtration unit (2) having a primary chamber (3) and a secondary chamber (4) separated by a semi-permeable membrane (5);
an extracorporeal blood circuit (17) having a blood withdrawal line (6) connected to an inlet of the primary chamber (3), and a blood return line (7) connected to an outlet of the primary chamber (3), said extracorporeal blood circuit (17) being configured for connection to a patient cardiovascular system;
a blood pump (21) configured to control the flow of blood through the extracorporeal blood circuit (17);
an effluent fluid line (13) connected to an outlet of the secondary chamber (4);
one or more of further fluid lines selected in the group comprising:

• a pre-dilution infusion line (29) connected at one end thereof to the blood withdrawal line (6),
• a post-dilution infusion line (63) connected at one end thereof to the blood return line (7),
• a post-dilution bicarbonate infusion line (23) connected at one end thereof to the blood return line (7),
• a ion balancing infusion line (74) connected at one end thereof either to the blood return line (7) or to a patient catheter,

- a dialysis liquid supply line (8) connected at one end thereof to the inlet of the secondary chamber (4),
- a pre-blood pump - PBP - infusion line (52) connected at one end thereof to the blood withdrawal line in a region of the blood withdrawal line which is positioned in use upstream from the blood pump (21),
- an anticoagulant infusion line (51) connected at one end thereof to the blood withdrawal line in a region of the blood withdrawal line which is positioned in use upstream from the blood pump (21), and
- a syringe fluid line (22) connected at one end thereof to the blood withdrawal line,

actuators for regulating the flow of fluid (24, 25, 26, 31, 53, 54, 65, 75) through said fluid lines (23, 8, 13, 29, 52, 51, 63, 74);

a memory (16) storing one or more mathematical relations; and

a control unit (12) connected to the memory (16) and to the actuators,

the method comprising the following steps executable by the control unit:

- receiving a patient prescription comprising clinical prescription parameters, the receiving step including:

  ○ allowing entry of a set value for a prescribed dialysis dose ($D_{set}$) to be delivered,
  ○ allowing entry of a target value for a parameter (nNBL; $Cp_{HCO3\_pat}$) indicative of a steady state acid-base balance in the blood of the patient who has to undergo a CRRT blood treatment,

- determining operating parameters using said one or more mathematical relations, the determining of the operating parameters comprises calculating a set value at least two fluid flow rates selected in the group including:

  - a fluid flow rate ($Q_{cit}$) through the anticoagulant infusion line (51),
  - a fluid flow rate ($Q_{PBP}$) through the PBP infusion line (52),
  - a fluid flow rate ($Q_{rep.pre}$) through the pre-dilution infusion line (29),
  - a fluid flow rate ($Q_{rep.post}$) through the post-dilution infusion line (63),
  - a fluid flow rate ($Q_{HCO3}$) through the post-dilution bicarbonate infusion line (23),
  - a fluid flow rate ($Q_{ca}$) through the ion balancing infusion line (74),
  - a blood fluid flow rate ($Q_b$) through the extracorporeal blood circuit (17),
  - a fluid flow rate ($Q_{dial}$) through the dialysis liquid supply line (8), and
  - a fluid flow rate ($Q_{eff}$) through the effluent fluid line (13),

wherein calculating the set value of at least two fluid flow rates is based at least on said set value of the prescribed dialysis dose ($D_{set}$) and on said target value for the parameter (nNBL; $Cp_{HCO3\_pat}$) indicative of a steady state acid-base balance in the blood.

In another independent aspect, a Continuous Renal Replacement Therapy (CRRT) apparatus is provided comprising:

a filtration unit (2) having a primary chamber (3) and a secondary chamber (4) separated by a semi-permeable membrane (5);

an extracorporeal blood circuit (17) having a blood withdrawal line (6) connected to an inlet of the primary chamber (3), and a blood return line (7) connected to an outlet of the primary chamber (3), said extracorporeal blood circuit (17) being configured for connection to a patient cardiovascular system;

a blood pump (21) configured to control the flow of blood through the extracorporeal blood circuit (17);

an effluent fluid line (13) connected to an outlet of the secondary chamber (4);

one or more of further fluid lines selected in the group comprising:

a pre-dilution infusion line (29) connected at one end thereof to the blood withdrawal line (6),

a post-dilution infusion line (63) connected at one end thereof to the blood return line (7),

a post-dilution bicarbonate infusion line (23) connected at one end thereof to the blood return line (7),

a ion balancing infusion line (74) connected at one end thereof either to the blood return line (7) or to a patient catheter,

a dialysis liquid supply line (8) connected at one end thereof to the inlet of the secondary chamber (4),

a pre-blood pump - PBP - infusion line (52) connected at one end thereof to the blood withdrawal line in a region of the blood withdrawal line which is positioned in use upstream from the blood pump (21),

an anticoagulant infusion line (51) connected at one end thereof to the blood withdrawal line in a region of the blood withdrawal line which is positioned in use upstream from the blood pump (21), and

a syringe fluid line (22) connected at one end thereof to the blood withdrawal line,

actuators for regulating the flow of fluid (24, 25, 26, 31, 53, 54, 65, 75) through said fluid lines (23, 8, 13, 29, 52, 51, 63, 74);
a memory (16) storing one or more mathematical relations; and
a control unit (12) connected to the memory (16) and to the actuators, the control unit being configured to execute a flow-rate setup procedure comprising:

- receiving a patient prescription comprising clinical prescription parameters, the receiving step including:

  ◦ allowing entry of a set value for a prescribed dialysis dose ($D_{set}$) to be delivered,
  ◦ allowing entry of a target value for a parameter (nNBL; $Cp_{HCO3\_pat}$) indicative of a steady state acid-base balance in the blood of the patient who has to undergo a CRRT blood treatment,

- determining operating parameters using said one or more mathematical relations, the determining of the operating parameters comprises calculating a set value at least two fluid flow rates selected in the group including:

  • a fluid flow rate ($Q_{cit}$) through the anticoagulant infusion line (51),
  • a fluid flow rate ($Q_{PBP}$) through the PBP infusion line (52),
  • a fluid flow rate ($Q_{rep.pre}$) through the pre-dilution infusion line (29),
  • a fluid flow rate ($Q_{rep.post}$) through the post-dilution infusion line (63),
  • a fluid flow rate ($Q_{HCO3}$) through the post-dilution bicarbonate infusion line (23),
  • a fluid flow rate ($Q_{ca}$) through the ion balancing infusion line (74),
  • a blood fluid flow rate ($Q_b$) through the extracorporeal blood circuit (17),
  • a fluid flow rate ($Q_{dial}$) through the dialysis liquid supply line (8), and
  • a fluid flow rate ($Q_{eff}$) through the effluent fluid line (13),

wherein calculating the set value of at least two fluid flow rates is based at least on said set value of the prescribed dialysis dose ($D_{set}$) and on said target value for the parameter (nNBL; $Cp_{HCO3\_pat}$) indicative of a steady state acid-base balance in the blood.

Notably and in accordance with the previous aspects, calculating each set value of the at least two fluid flow rates is based at least on both said set value of the prescribed dialysis dose ($D_{set}$) and on said target value for the parameter (nNBL; $Cp_{HCO3\_pat}$) indicative of a steady state acid-base balance in the blood. For example, a first fluid flow rate from the list (e.g., the fluid flow rate ($Q_{rep.post}$) through the post-dilution infusion line (63)) is calculated based on both the set value of the prescribed dialysis dose ($D_{set}$) and on the target value for the parameter (nNBL; $Cp_{HCO3\_pat}$) indicative of a steady state acid-base balance in the blood and a second fluid flow rate from the list (e.g., the fluid flow rate ($Q_{dial}$) through the dialysis liquid supply line (8)) is calculated based on both the set value of the prescribed dialysis dose ($_{Dset}$) and on the target value for the parameter (nNBL; $Cp_{HCO3\_pat}$) indicative of a steady state acid-base balance in the blood.

In a further independent aspect, a Continuous Renal Replacement Therapy (CRRT) apparatus is provided comprising:

a filtration unit (2) having a primary chamber (3) and a secondary chamber (4) separated by a semi-permeable membrane (5);
an extracorporeal blood circuit (17) having a blood withdrawal line (6) connected to an inlet of the primary chamber (3), and a blood return line (7) connected to an outlet of the primary chamber (3), said extracorporeal blood circuit (17) being configured for connection to a patient cardiovascular system;
a blood pump (21) configured to control the flow of blood through the extracorporeal blood circuit (17);
an effluent fluid line (13) connected to an outlet of the secondary chamber (4);
one or more of further fluid lines selected in the group comprising:

a pre-dilution infusion line (29) connected at one end thereof to the blood withdrawal line (6),
a post-dilution infusion line (63) connected at one end thereof to the blood return line (7),
a post-dilution bicarbonate infusion line (23) connected at one end thereof to the blood return line (7),
a ion balancing infusion line (74) connected at one end thereof either to the blood return line (7) or to a patient catheter,
a dialysis liquid supply line (8) connected at one end thereof to the inlet of the secondary chamber (4),
a pre-blood pump - PBP - infusion line (52) connected at one end thereof to the blood withdrawal line in a region of the blood withdrawal line which is positioned in use upstream from the blood pump (21),

an anticoagulant infusion line (51) connected at one end thereof to the blood withdrawal line in a region of the blood withdrawal line which is positioned in use upstream from the blood pump (21), and

a syringe fluid line (22) connected at one end thereof to the blood withdrawal line,

actuators for regulating the flow of fluid (24, 25, 26, 31, 53, 54, 65, 75) through said fluid lines (23, 8, 13, 29, 52, 51, 63, 74);

a memory (16) storing one or more mathematical relations; and

a control unit (12) connected to the memory (16) and to the actuators, the control unit being configured to execute a flow-rate setup procedure comprising:

- receiving a patient prescription comprising clinical prescription parameters, the receiving step including:

  ○ allowing entry of a set value for a prescribed dialysis dose ($_{Dset}$) to be delivered,
  ○ allowing entry of a target value for a parameter (nNBL; $Cp_{HCO3\_pat}$) indicative of a steady state acid-base balance in the blood of the patient who has to undergo a CRRT blood treatment,

- determining one or more operating parameters using said one or more mathematical relations, the determining of the operating parameters comprises calculating a set value of at least two fluid flow rates selected in the group including:

  • a fluid flow rate ($Q_{cit}$) through the anticoagulant infusion line (51),
  • a fluid flow rate ($Q_{PBP}$) through the PBP infusion line (52),
  • a fluid flow rate ($Q_{rep.pre}$) through the pre-dilution infusion line (29),
  • a fluid flow rate ($Q_{rep.post}$) through the post-dilution infusion line (63),
  • a fluid flow rate ($Q_{HCO3}$) through the post-dilution bicarbonate infusion line (23),
  • a fluid flow rate ($Q_{ca}$) through the ion balancing infusion line (74),
  • a blood fluid flow rate ($Q_b$) through the extracorporeal blood circuit (17),
  • a fluid flow rate ($Q_{syr}$) through a syringe fluid line (22),
  • a fluid flow rate ($Q_{dial}$) through the dialysis liquid supply line (8), and
  • a fluid flow rate ($Q_{eff}$) through the effluent fluid line (13),

  wherein calculating the set value of either:

  ➢ the fluid flow rate ($Q_{cit}$) through the anticoagulant infusion line (51),
  ➢ the fluid flow rate ($Q_{PBP}$) through the PBP infusion line (52),
  ➢ the fluid flow rate ($Q_{rep.pre}$) through the pre-dilution infusion line (29),

  is based at least on both said set value of the prescribed dialysis dose ($_{Dset}$) and on said target value for the parameter (nNBL; $Cp_{HCO3\_pat}$) indicative of a steady state acid-base balance in the blood (it is noted that this flow rate is one of the at least two flow rates mentioned in previous aspects), and

  wherein calculating the set value of at least another fluid flow rate is based at least on both said set value of the prescribed dialysis dose ($_{Dset}$) and on said target value for the parameter (nNBL; $Cp_{HCO3\_pat}$) indicative of a steady state acid-base balance in the blood.

In a further aspect in accordance with any one of the previous aspects, said at least another fluid flow rate (or one of the at least two flow rates) is either:

➢ the fluid flow rate ($Q_{dial}$) through the dialysis liquid supply line (8); or
➢ the fluid flow rate ($Q_{eff}$) through the effluent fluid line (13),

and wherein calculating the set value of said another fluid flow rate is based at least on both said target value for the parameter (nNBL; $Cp_{HCO3\_pat}$) indicative of a steady state acid-base balance in the blood and on said set value of the prescribed dialysis dose ($D_{set}$).

In a 2nd aspect according to any one of the previous aspects the anticoagulant infusion line (51) connected at one end thereof to the blood withdrawal line in a region of the blood withdrawal line which is positioned in use upstream from the blood pump (21) and an anticoagulant source (10) connected at an opposite end of the anticoagulant infusion line (51), wherein receiving a patient prescription further comprises allowing entry of a set value for a regional anticoagulation dose

($D_{cit}$), in particular a citrate dose, indicative of an intensity of the regional anticoagulation.

In a 3$^{rd}$ aspect according to the preceding aspect, the regional anticoagulation dose ($D_{cit}$) has the same unit as a concentration and is in more detail the injected amount of anticoagulant per liter of treated blood (mmol/L blood). In a 4$^{th}$ aspect according to the preceding two aspects, the control unit (12) is configured to mathematically relate, using a citrate flow rate mathematical relation, the set value for the fluid flow rate ($Q_{cit}$) through the anticoagulant infusion line (51) with the set value for the regional anticoagulation dose ($D_{cit}$), in particular to calculate the set value for the fluid flow rate ($Q_{cit}$) as a function of the set value for the regional anticoagulation dose ($D_{cit}$).

In a 5$^{th}$ aspect according to any one of the preceding three aspects, the control unit (12) is configured to mathematically relate, using a citrate flow rate mathematical relation, the set value for the fluid flow rate ($Q_{cit}$) through the anticoagulant infusion line (51) with a blood flow rate ($Q_b$) and/or with an anticoagulant, particularly citrate, concentration in the anticoagulant source (10), in particular to calculate the set value for the fluid flow rate ($Q_{cit}$) as a function of the blood flow rate ($Q_b$) and/or the anticoagulant, particularly citrate, concentration in the anticoagulant source (10).

In a 6$^{th}$ aspect according to any one of the preceding four aspects, said one or more mathematical relations include a citrate flow rate mathematical relation and the fluid flow rate ($Q_{cit}$) through the anticoagulant infusion line (51) is calculated based on the citrate flow rate mathematical relation stored in said memory (16):

$$Q_{cit} = \frac{D_{cit}}{C_{cit\_PBP}} \cdot Q_b$$

wherein the significance of the denotations is given in the Glossary.

In a 7$^{th}$ aspect according to any one of the preceding aspects, the control unit (12) is configured to drive an anticoagulant pump (54) to infuse anticoagulant in the blood withdrawal line at the calculated fluid flow rate ($Q_{cit}$) for the anticoagulant.

In a 8$^{th}$ aspect according to any one of the preceding aspects 2 or 3, the regional anticoagulation dose ($D_{cit}$) is the fluid flow rate ($Q_{cit}$) through the anticoagulant infusion line (51).

In a 9$^{th}$ aspect according to any one of the preceding seven aspects, calculating the set value comprises calculating at least three fluid flow rates and is further based at least on said set value of the regional anticoagulation dose ($D_{cit}$), in particular using at least one of said one or more mathematical relations.

In a 10$^{th}$ aspect according to any one of the preceding aspects, the CRRT apparatus comprises the ion balancing infusion line (74) connected at one end thereof either to the blood return line (7) or to a patient catheter and a source of ion balancing solution (11) connected at an opposite end of ion balancing infusion line (74), wherein receiving a patient prescription further comprises allowing entry of a set value for an ion re-establishing solution parameter (CaComp; $D_{ca}$), in particular a calcium compensating parameter, indicative of the intensity of ion balancing, in particular of calcium ions balancing.

In an 11$^{th}$ aspect according to the preceding aspect, the ion re-establishing solution parameter (CaComp) represents a compensation value or compensation percentage of the calcium removed in the filtration unit (2), for example included between 0.05 and 2 or between 5% and 200%.

In a 12$^{th}$ aspect according to the preceding aspect 10, the ion re-establishing solution parameter ($D_{ca}$) is a, e.g. calcium, dose in terms of an ion, e.g., calcium, concentration, in particular the calcium dose being total calcium concentration in the effluent.

In a 13$^{th}$ aspect according to any one of the preceding three aspects, the control unit (12) is configured to mathematically relate, using a calcium flow rate mathematical relation, the set value for the fluid flow rate ($Q_{ca}$) through the ion balancing infusion line (74) with the set value for the ion re-establishing solution parameter (CaComp; $D_{ca}$), in particular to calculate the set value for the fluid flow rate ($Q_{ca}$) as a function of the set value for the ion re-establishing solution parameter (CaComp; $D_{ca}$).

In a 14$^{th}$ aspect according to any one of the preceding four aspects, the control unit (12) is configured to mathematically relate, using a calcium flow rate mathematical relation, the set value for the fluid flow rate ($Q_{ca}$) through the ion balancing infusion line (74) with an effluent flow rate ($Q_{eff}$), in particular to calculate the set value for the fluid flow rate ($Q_{ca}$) as a function of the effluent flow rate ($Q_{eff}$).

In a 15$^{th}$ aspect according to any one of the preceding five aspects, the control unit (12) is configured to mathematically relate, using a calcium flow rate mathematical relation, the set value for the fluid flow rate ($Q_{ca}$) through the ion balancing infusion line (74) with a calcium concentration ($C_{ca}$) in a source of ion balancing solution (11) and/or with a calcium concentration ($C_{ca rep.post}$) in an auxiliary container (64) connected to the post-dilution infusion line (63), and/or with a calcium concentration ($C_{caHCO3}$) in a bicarbonate container (28) connected to the post-dilution bicarbonate infusion line (23), in particular to calculate the set value for the fluid flow rate ($Q_{ca}$) as a function of the calcium concentration ($C_{ca}$) in the source of ion balancing solution (11) and/or as a function of the calcium concentration ($C_{ca rep.post}$) in the auxiliary container (64) connected to the post-dilution infusion line (63) and/or as a function of the calcium concentration ($C_{caHCO3}$) in a

bicarbonate container (28) connected to the post-dilution bicarbonate infusion line (23).

In a 16th aspect according to any one of the preceding six aspects, said one or more mathematical relations include a calcium flow rate mathematical relation and wherein the set value for the fluid flow rate ($Q_{ca}$) through the ion balancing infusion line (74) is calculated based on the calcium flow rate mathematical relation stored in the memory (16):

$$Q_{ca} = \frac{CaComp \cdot J_{Ca}}{C_{ca}} - \frac{Q_{rep.post} \cdot C_{ca_{rep.post}}}{C_{ca}} - \frac{Q_{HCO3} \cdot C_{ca_{HCO3}}}{C_{ca}}$$

wherein the significance of the denotations is given in the Glossary.

In a 17th aspect according to the preceding aspect 12, said one or more mathematical relations include a calcium flow rate mathematical relation and wherein the set value for the fluid flow rate ($Q_{ca}$) through the ion balancing infusion line (74) is calculated based on the calcium flow rate mathematical relation stored in the memory (16):

$$Q_{ca} = \frac{D_{ca} \cdot Q_{eff}}{C_{ca}} - \frac{Q_{rep.post} \cdot C_{ca_{rep.post}}}{C_{ca}} - \frac{Q_{HCO3} \cdot C_{ca_{HCO3}}}{C_{ca}}$$

wherein the significance of the denotations is given in the Glossary; if any of the infusion lines is missing, the corresponding flow rate is absent from the mathematical relation, or the corresponding flow rate is set to zero.

In a 18th aspect according to any one of the preceding aspects, the control unit (12) is configured to drive an ion balancing pump (75) to infuse an ion re-establishing solution in the blood return line (7) or into the patient (P) at the calculated fluid flow rate ($Q_{ca}$) for ion re-establishing solution.

In a 19th aspect according to any one of the preceding nine aspects, calculating the set value comprises calculating at least three fluid flow rates and is further based at least on said set value for the ion re-establishing solution parameter ($CaComp$; $D_{ca}$), in particular using at least one of said one or more mathematical relations.

In a 20th aspect according to any one of the preceding aspects, receiving a patient prescription further comprises allowing entry of a set value for the blood flow rate ($Q_b$) and calculating the set value comprises calculating at least three fluid flow rates and calculating one or more (two or all) set values out of the at least three fluid flow rates is further based at least on said set value for the blood flow rate ($Q_b$), in particular using at least one of said one or more mathematical relations.

In a 21st aspect according to any one of the preceding aspects, receiving a patient prescription further comprises allowing entry of a set value for at least a fluid removal rate ($Q_{PFR}$) from the patient and calculating the set value comprises calculating at least three fluid flow rates and calculating one or more (two or all) set values out of the at least three fluid flow rates is further based at least on said set value for the fluid removal rate ($Q_{PFR}$) from the patient, in particular using at least one of said one or more mathematical relations.

In a 22nd aspect according to any one of the preceding aspects, the control unit (12) is configured to mathematically relate, using an effluent flow rate mathematical relation, the fluid flow rate ($Q_{eff}$) through the effluent fluid line (13) with the set value for the fluid removal rate ($Q_{PFR}$), in particular to calculate the fluid flow rate ($Q_{eff}$) through the effluent fluid line (13) based on the set value for the fluid removal rate ($Q_{PFR}$).

In a 23rd aspect according to any one of the preceding two aspects, the control unit (12) is configured to mathematically relate, in particular to calculate, the fluid flow rate ($Q_{eff}$) through the effluent fluid line (13) based on an effluent flow rate mathematical relation comprised in said one or more mathematical relations:

$$Q_{eff} = Q_{PBP} + Q_{cit} + Q_{syr} + Q_{dial} + Q_{rep.pre} + Q_{rep.post} + Q_{HCO3} + Q_{PFR} + Q_{ca}$$

wherein the significance of the denotations is given in the Glossary; if any of the infusion lines or the dialysis liquid supply line is missing, the corresponding flow rate is absent from the mathematical relation, or the corresponding flow rate is set to zero.

In a 24th aspect according to any one of the preceding aspects, the CRRT apparatus comprises a fluid source of a solution supplying fluid to one of said one or more further fluid lines, said solution comprising at least one buffer agent in the form of bicarbonate or bicarbonate precursor.

In a 25th aspect according to the preceding aspect, the bicarbonate precursor includes citrate, lactate and/or acetate.

In a 26th aspect according to any one of the preceding aspects, the parameter (nNBL) indicative of a steady state acid-base balance in the blood of the patient undergoing a CRRT treatment is a parameter function of a net buffer load (NBL) in the patient expected at a steady state, wherein the net buffer load is a sum of bicarbonate generation from bicarbonate precursor metabolism, such as citrate or lactate metabolism, bicarbonate balance in the extracorporeal blood circuit (17) and/or of bicarbonate infusions into the patient and/or of acid infusion in the extracorporeal blood circuit (17), such as citric acid infusion.

In a 27th aspect according to the preceding aspect, the net buffer load is a normalized net buffer (nNBL) load in the patient expected at a steady state, particularly normalized over a patient body weight (BW), in more detail the parameter (nNBL) indicative of a steady state acid-base balance in the blood of the patient undergoing a CRRT treatment being the net buffer load (NBL) or the normalized net buffer load (nNBL):

$$\frac{J_{buffer\_load}}{BW} = nNBL = \frac{NBL}{BW}$$

In a 28th aspect according to any one of the preceding aspects, the parameter (nNBL) indicative of a steady state acid-base balance in the blood of the patient is based on one or more of, and in particular the four of:

- an estimation of an amount per unit of time of bicarbonate generated from metabolism of the bicarbonate precursor infused to the patient, in particular of citrate $(J_{met\_cit})$ and/or lactate $(J_{met\_lact})$;
- a bicarbonate balance $(J_{HCO3\_bal})$ from the CRRT blood treatment to be delivered in terms of an amount per unit of time;
- a lactate balance $(J_{lact\_bal})$ from the CRRT blood treatment to be delivered in terms of an amount per unit of time;
- an acid infusion $(J_{H+})$ from citric acid contained in the fluid source in terms of an amount per unit of time. In a 29th aspect according to the preceding aspect, the parameter (nNBL) indicative of a steady state acid-base balance in the blood of the patient is an algebraic sum of the estimation of bicarbonate form precursor metabolism $(J_{met\_cit};\ J_{lact})$, the bicarbonate balance $(J_{HCO3\_bal})$, and the acid infusion $(J_{H+})$, in particular the acid infusion $(J_{H+})$ being a negative term providing a loss in patient buffer.

In a 30th aspect according to the preceding aspect, the parameter (nNBL) indicative of a steady state acid-base balance in the blood of the patient is defined as follows:

$$nNBL = \frac{J_{buffer\_load}}{BW} = \frac{J_{met\_cit} + J_{HCO3\_bal} - J_{H+}}{BW}$$

alternatively, when also lactate balance is considered, the parameter (nNBL) indicative of a steady state acid-base balance in the blood of the patient is defined as follows:

$$nNBL = \frac{J_{buffer\_load}}{BW} = \frac{J_{met\_cit} + J_{HCO3\_bal} + J_{lact\_bal} - J_{H+}}{BW}$$

In a 31st aspect according to any one of the preceding aspects, the parameter (nNBL; $Cp_{HCO3\_pat}$) indicative of a steady state acid-base balance in the blood of the patient is based on an acid infusion $(J_{H+})$ from citric acid contained in the fluid source in terms of an amount per unit of time, wherein the acid infusion $(J_{H+})$ is a function of a citric acid concentration $(C_{citric\_pbp})$ and of an infusion rate $(Q_{cit})$ of citric acid, in particular the acid infusion $(J_{H+})$ being equal to 3 times the citric acid concentration $(C_{citric\_pbp})$ times the infusion rate $(Q_{cit})$ of citric acid. In a 32nd aspect according to any one of the preceding aspects, the parameter (nNBL) indicative of a steady state acid-base balance in the blood of the patient undergoing a CRRT treatment is a parameter defined assuming a constant value for patient plasma bicarbonate concentration $(Cp_{HCO3\_pat}0)$, said constant value being for example 25 mM.

In a 33rd aspect according to any one of the preceding aspects, the parameter $(Cp_{HCO3\_pat})$ indicative of a steady state bicarbonate concentration in the blood of the patient is based on an estimated net buffer load $(J_{buffer\_load}/BW)$ indicative of a steady state acid-base balance in the blood of the patient.

In a further aspect according to any one of the preceding aspects, the parameter $(Cp_{HCO3\_pat})$ indicative of a steady state bicarbonate concentration in the blood of the patient is defined imposing a constant value for a normalized net buffer load (NBL) for the patient at steady state, said constant value being for example NBL=nNBL0·BW= 0,1 mmol/h/kg.

In a 34th aspect according to any one of the preceding aspects, the parameter $(Cp_{HCO3\_pat})$ indicative of a steady state bicarbonate concentration in the blood of the patient is defined imposing a constant value for the normalized net buffer load (NBL) for the patient at steady state, said constant value being for example nNBL0·BW= 0,1 mmol/h/kg.

In a 35th aspect according to any one of the preceding aspects, said one or more mathematical relations include steady state bicarbonate indicator mathematical relations, and wherein the steady state bicarbonate indicator mathematical relations links the parameter $(Cp_{HCO3\_pat})$ indicative of a steady state bicarbonate concentration in the blood of the patient with said at least two flow rates in the group.

In a 36th aspect according to any one of the preceding aspects, said one or more mathematical relations include steady state bicarbonate indicator mathematical relations, and wherein the steady state bicarbonate indicator mathematical relations links the parameter ($Cp_{HCO3\_pat}$) indicative of a steady state bicarbonate concentration in the blood of the patient with one or more of the fluid flow rate ($Q_{rep.pre}$) through the pre-dilution infusion line (29), the fluid flow rate ($Q_{dial}$) through the dialysis liquid supply line (8), the fluid flow rate ($Q_{cit}$) through the anticoagulant infusion line (51), the fluid flow rate ($Q_{PBP}$) through the PBP infusion line (52), in particular links the parameter ($Cp_{HCO3\_pat}$) with the fluid flow rate ($Q_{rep.pre}$; $Q_{dial}$; $Q_{cit}$; $Q_{PBP}$) times the respective bicarbonate concentration ($C_{HCO3rep.pre}$; $C_{HCO3dial}$; $C_{HCO3cit}$; $C_{HCO3PBP}$) in the fluid flowing in the respective line.

In a 37th aspect according to any one of the preceding aspects, said one or more mathematical relations include steady state bicarbonate indicator mathematical relations, and wherein the steady state bicarbonate indicator mathematical relations links the parameter ($Cp_{HCO3\_pat}$) indicative of a steady state bicarbonate concentration in the blood of the patient with the blood flow rate ($Q_b$), in particular a blood water flow rate ($Q_{bw}$) and/or a blood water flow rate ($Q_{bw\_inlet}$) at the inlet of the filtration unit (2).

In a 38th aspect according to any one of the preceding aspects, said one or more mathematical relations include steady state bicarbonate indicator mathematical relations, and wherein the steady state bicarbonate indicator mathematical relations links the parameter ($Cp_{HCO3\_pat}$) indicative of a steady state bicarbonate concentration in the blood of the patient with a bicarbonate concentration ($C_{HCO3dial}$) in a fluid flowing in the dialysis liquid supply line (8) and/or with a bicarbonate clearance ($K_{HCO3}$) of the filtration unit (2).

In a 39th aspect according to any one of the preceding aspects, said one or more mathematical relations include steady state bicarbonate indicator mathematical relations, and wherein the steady state bicarbonate indicator mathematical relations links the parameter ($Cp_{HCO3\_pat}$) indicative of a steady state bicarbonate concentration in the blood of the patient with a bicarbonate plasma water concentration $Cpw_{HCO3\_inlet}$ at filtration unit inlet.

In a 40th aspect according to any one of the preceding aspects, said one or more mathematical relations include steady state bicarbonate indicator mathematical relations, and wherein the steady state bicarbonate indicator mathematical relations links the parameter ($Cp_{HCO3\_pat}$) indicative of a steady state bicarbonate concentration in the blood of the patient with an ultrafiltration flow rate ($Q_{fil}$) in the filtration unit (2), in particular with the ultrafiltration flow rate ($Q_{fil}$) times a bicarbonate concentration ($C_{HCO3dial}$) in the fluid flowing in the dialysis liquid supply line (8). In a 41st aspect according to any one of the preceding aspects, said one or more mathematical relations include steady state bicarbonate indicator mathematical relations, and wherein the steady state bicarbonate indicator mathematical relations links the parameter ($Cp_{HCO3\_pat}$) indicative of a steady state bicarbonate concentration in the blood of the patient with a bicarbonate removal ($J_{HCO3\_eff}$) into spent dialysate flowing in the effluent line (13). In a 42nd aspect according to any one of the preceding aspects, said one or more mathematical relations include steady state bicarbonate indicator mathematical relations, and wherein the steady state bicarbonate indicator mathematical relations are the following:

$$Cp_{HCO3\_pat} = \frac{Fp}{Qbw}$$
$$\cdot \left( Cpw_{HCO3_{inlet}} \cdot Qbw_{inlet} - Q_{rep.pre} \cdot C_{HCO3rep.pre} - Q_{cit} \cdot C_{HCO3_{cit}} \right.$$
$$\left. - Q_{PBP} \cdot C_{HCO3_{PBP}} \right)$$

$$Cpw_{HCO3\_inlet} = C_{HCO3\_dial} + \frac{J_{HCO3\_eff} - Q_{dial} \times C_{HCO3_{dial}} - Q_{fil} \times C_{HCO3\_dial}}{K_{HCO3}}$$

$$J_{HCO3\_eff} = J_{HCO3\_inf} - J_{HCO3_{bal}} = J_{HCO3\_inf} + J_{met\_cit} - J_{H+} - J_{buffer\_load}$$

$$J_{buffer\_load} = nNBL \times BW$$

wherein the significance of the denotations is given in the Glossary; if any of the infusion lines or the dialysis liquid supply line is missing, the corresponding flow rate is absent from the mathematical relation, or the corresponding flow rate is set to zero.

Note that if $Cp_{HCO3\_pat}$ is taken as the parameter indicative of a steady state acid-base balance in the blood of the patient, then nNBL will have to be (implicitly) taken as a constant, namely nNBL0; if vice versa, nNBL is taken as the parameter indicative of a steady state acid-base balance in the blood of the patient, then $Cp_{HCO3\_pat}$ will have to be (implicitly) taken as a constant, namely $Cp_{HCO3\_pat}0$.

In a 43rd aspect according to any one of the preceding aspects, the target value for the parameter (nNBL; $Cp_{HCO3\_pat}$) indicative of a steady state acid-base balance in the blood of the patient affects the set value of the fluid flow rate ($Q_{rep.pre}$) through the pre-dilution infusion line (29), the set value of the fluid flow rate ($Q_{rep.pre}$) through the pre-dilution infusion line (29) being an operating parameter calculated by the control unit (12). In particular, this is one of the at least two flow rates calculated based on the set value of the prescribed dialysis dose ($_{Dset}$) and on said target value for the parameter (nNBL; $Cp_{HCO3\_pat}$) indicative of a steady state acid-base balance in the blood. In a 44th aspect, according to any one of the preceding aspects, the target value for the parameter (nNBL; $Cp_{HCO3\_pat}$) indicative of a steady state acid-base balance in the blood of the patient affects the set value of the fluid flow rate ($Q_{rep.post}$) through the post-dilution infusion line (63), affects the set value of the fluid flow rate ($Q_{rep.post}$) through the post-dilution infusion line (63) being an operating parameter calculated by the control unit (12). In particular, this is one of the at least two flow rates calculated based on the set value of the prescribed dialysis dose ($_{Dset}$) and on said target value for the parameter (nNBL; $Cp_{HCO3\_pat}$) indicative of a steady state acid-base balance in the blood.

In a 45th aspect according to any one of the preceding aspects, the target value for the parameter (nNBL; $Cp_{HCO3\_pat}$) indicative of a steady state acid-base balance in the blood of the patient affects the set value of the fluid flow rate ($Q_{HCO3}$) through the post-dilution bicarbonate infusion line (23), the set value of the fluid flow rate ($Q_{HCO3}$) through the post-dilution bicarbonate infusion line (23) being an operating parameter calculated by the control unit (12). In particular, this is one of the at least two flow rates calculated based on the set value of the prescribed dialysis dose ($_{Dset}$) and on said target value for the parameter (nNBL; $Cp_{HCO3\_pat}$) indicative of a steady state acid-base balance in the blood.

In a 46th aspect according to any one of the preceding aspects, the target value for the parameter (nNBL; $Cp_{HCO3\_pat}$) indicative of a steady state acid-base balance in the blood of the patient affects the set value of the fluid flow rate ($Q_{ca}$) through the ion balancing infusion line (74), the set value of the fluid flow rate ($Q_{ca}$) through the ion balancing infusion line (74) being an operating parameter calculated by the control unit (12). In particular, this is one of the at least two flow rates calculated based on the set value of the prescribed dialysis dose ($_{Dset}$) and on said target value for the parameter (nNBL; $Cp_{HCO3\_pat}$) indicative of a steady state acid-base balance in the blood. In a 47th aspect according to any one of the preceding aspects, the target value for the parameter (nNBL; $Cp_{HCO3\_pat}$) indicative of a steady state acid-base balance in the blood of the patient affects the set value of the fluid flow rate ($Q_{dial}$) through the dialysis liquid supply line (8), the set value of the fluid flow rate ($Q_{dial}$) through the dialysis liquid supply line (8) being an operating parameter calculated by the control unit (12). In particular, this is one of the at least two flow rates calculated based on the set value of the prescribed dialysis dose ($_{Dset}$) and on said target value for the parameter (nNBL; $Cp_{HCO3\_pat}$) indicative of a steady state acid-base balance in the blood. In a 48th aspect according to any one of the preceding aspects, the target value for the parameter (nNBL; $Cp_{HCO3\_pat}$) indicative of a steady state acid-base balance in the blood of the patient affects the set value of the fluid flow rate ($Q_{cit}$) through the anticoagulant infusion line (51), particularly in case the set value for the blood fluid flow rate ($Q_b$) through the extracorporeal blood circuit (17) is determined by the control unit (12) as an operating parameter, the set value of the fluid flow rate ($Q_{cit}$) through the anticoagulant infusion line (51) being an operating parameter calculated by the control unit (12). In particular, this is one of the at least two flow rates calculated based on the set value of the prescribed dialysis dose ($_{Dset}$) and on said target value for the parameter (nNBL; $Cp_{HCO3\_pat}$) indicative of a steady state acid-base balance in the blood.

In a 49th aspect according to any one of the preceding aspects, the target value for the parameter (nNBL; $Cp_{HCO3\_pat}$) indicative of a steady state acid-base balance in the blood of the patient affects the set value of the fluid flow rate ($Q_{PBP}$) through the PBP infusion line (52), the set value of the fluid flow rate ($Q_{PBP}$) through the PBP infusion line (52) being an operating parameter calculated by the control unit (12). In particular, this is one of the at least two flow rates calculated based on the set value of the prescribed dialysis dose ($_{Dset}$) and on said target value for the parameter (nNBL; $Cp_{HCO3\_pat}$) indicative of a steady state acid-base balance in the blood.

In a 50th aspect according to any one of the preceding aspects, the target value for the parameter (nNBL; $Cp_{HCO3\_pat}$) indicative of a steady state acid-base balance in the blood of the patient affects the set value of the blood fluid flow rate ($Q_b$) through the extracorporeal blood circuit (17), the set value of the blood fluid flow rate ($Q_b$) through the extracorporeal blood circuit (17) being an operating parameter calculated by the control unit (12). In particular, this is one of the at least two flow rates calculated based on the set value of the prescribed dialysis dose ($_{Dset}$) and on said target value for the parameter (nNBL; $Cp_{HCO3\_pat}$) indicative of a steady state acid-base balance in the blood.

In a 51st aspect according to any one of the preceding aspects, the target value for the parameter (nNBL; $Cp_{HCO3\_pat}$) indicative of a steady state acid-base balance in the blood of the patient affects the set value of the fluid flow rate ($Q_{eff}$) through the effluent fluid line (13), the set value of the fluid flow rate ($Q_{eff}$) through the effluent fluid line (13) being an operating parameter calculated by the control unit (12). In particular, this is one of the at least two flow rates calculated based on the set value of the prescribed dialysis dose ($_{Dset}$) and on said target value for the parameter (nNBL; $Cp_{HCO3\_pat}$) indicative of a steady state acid-base balance in the blood.

Notably, the target value for the parameter (nNBL; $Cp_{HCO3\_pat}$) indicative of a steady state acid-base balance in the blood of the patient is used for the calculation of the majority and specifically all the above indicated fluid flow rates, when relevant (i.e., if the lines is present and the applied treatment requires use of the line with a set flow rate different from

zero).

In a 52nd aspect according to any one of the preceding aspects, at least a number of said one or more mathematical relations correlates the parameter (nNBL; $Cp_{HCO3\_pat}$) indicative of a steady state acid-base balance in the blood of the patient who has to undergo a CRRT blood treatment with the at least two fluid flow rates selected in said group.

In a 53rd aspect according to any one of the preceding aspects, calculating the set value of the fluid flow rate ($Q_{cit}$) through the anticoagulant infusion line (51) is based at least on said target value for the parameter (nNBL; $Cp_{HCO3\_pat}$) indicative of a steady state acid-base balance in the blood and/or on said set value of the prescribed dialysis dose ($D_{set}$), particularly in case the set value for the blood fluid flow rate ($Q_b$) through the extracorporeal blood circuit (17) is determined by the control unit (12) as an operating parameter,
in particular wherein the control unit (12) calculates the set value of the fluid flow rate ($Q_{cit}$) through the anticoagulant infusion line (51) using said mathematical relations.

In a 54th aspect according to any one of the preceding aspects, calculating the set value of the fluid flow rate ($Q_{PBP}$) through the PBP infusion line (52) is based at least on said target value for the parameter (nNBL; $Cp_{HCO3\_pat}$) indicative of a steady state acid-base balance in the blood and/or on said set value of the prescribed dialysis dose ($D_{set}$), in particular wherein the control unit (12) calculates the set value of the fluid flow rate ($Q_{PBP}$) through the PBP infusion line (52) using said mathematical relations.

In a 55th aspect according to any one of the preceding aspects, calculating the set value of the fluid flow rate ($Q_{rep.pre}$) through the pre-dilution infusion line (29) is based at least on said target value for the parameter (nNBL; $Cp_{HCO3\_pat}$) indicative of a steady state acid-base balance in the blood and/or on said set value of the prescribed dialysis dose ($D_{set}$), in particular wherein the control unit (12) calculates the set value of the fluid flow rate ($Q_{rep.pre}$) through the pre-dilution infusion line (29) using said mathematical relations.

In a 56th aspect according to any one of the preceding aspects, calculating the set value of the fluid flow rate ($Q_{rep.post}$) through the post-dilution infusion line (63) is based at least on said target value for the parameter (nNBL; $Cp_{HCO3\_pat}$) indicative of a steady state acid-base balance in the blood and/or on said set value of the prescribed dialysis dose ($D_{set}$), in particular wherein the control unit (12) calculates the set value of the fluid flow rate ($Q_{rep.post}$) through the post-dilution infusion line (63) using said mathematical relations.

In a 57th aspect according to any one of the preceding aspects, calculating the set value of the fluid flow rate ($Q_{HCO3}$) through the post-dilution bicarbonate infusion line (23) is based at least on said target value for the parameter (nNBL; $Cp_{HCO3\_pat}$) indicative of a steady state acid-base balance in the blood and/or on said set value of the prescribed dialysis dose ($D_{set}$), in particular wherein the control unit (12) calculates the set value of the fluid flow rate ($Q_{HCO3}$) through the post-dilution bicarbonate infusion line (23) using said mathematical relations.

In a 58th aspect according to any one of the preceding aspects, calculating the set value of the fluid flow rate ($Q_{ca}$) through the ion balancing infusion line (74) is based at least on said target value for the parameter (nNBL; $Cp_{HCO3\_pat}$) indicative of a steady state acid-base balance in the blood and/or on said set value of the prescribed dialysis dose ($D_{set}$), in particular wherein the control unit (12) calculates the set value of the fluid flow rate ($Q_{ca}$) through the ion balancing infusion line (74) using said mathematical relations.

In a 59th aspect according to any one of the preceding aspects, calculating the set value of the blood fluid flow rate ($Q_b$) through the extracorporeal blood circuit (17) is based at least on said target value for the parameter (nNBL; $Cp_{HCO3\_pat}$) indicative of a steady state acid-base balance in the blood and/or on said set value of the prescribed dialysis dose ($D_{set}$), in particular wherein the control unit (12) calculates the set value of the fluid flow rate ($Q_b$) through the extracorporeal blood circuit (17) using said mathematical relations.

In a 60th aspect according to any one of the preceding aspects, calculating the set value of the fluid flow rate ($Q_{dial}$) through the dialysis liquid supply line (8) is based at least on said target value for the parameter (nNBL; $Cp_{HCO3\_pat}$) indicative of a steady state acid-base balance in the blood and/or on said set value of the prescribed dialysis dose ($D_{set}$), in particular wherein the control unit (12) calculates the set value of the fluid flow rate ($Q_{dial}$) through the dialysis liquid supply line (8) using said mathematical relations.

In a 61st aspect according to any one of the preceding aspects, calculating the set value of the fluid flow rate ($Q_{eff}$) through the effluent fluid line (13) is based at least on said target value for the parameter (nNBL; $Cp_{HCO3\_pat}$) indicative of a steady state acid-base balance in the blood and/or on said set value of the prescribed dialysis dose ($D_{set}$), in particular wherein the control unit (12) calculates the set value of the fluid flow rate ($Q_{eff}$) through the effluent fluid line (13) using said mathematical relations.

In a 62nd aspect according to any one of the preceding aspects, wherein said one or more mathematical relations include a net buffer load mathematical relation linking the the parameter (nNBL; $Cp_{HCO3\_pat}$) indicative of a steady state acid-base balance in the blood with at least one, and particularly all, of:

    ◦ a bicarbonate balance ($J_{HCO3\_bal}$),
    ◦ a bicarbonate generated from metabolism of citrate ($J_{met\_cit}$), or a citrate load ($J_{cit\_load}$), in particular wherein the metabolism of citrate load leads to 3 moles of bicarbonate per mole of citrate at steady state, namely $J_{met\_cit}$ = 3 $\cdot J_{cit\_load}$,
  ○ a bicarbonate generated from metabolism of lactate ($J_{met\_lact}$), or a lactate balance ($J_{lact\_bal}$), in particular wherein the metabolism of lactate leads to 1 mole of bicarbonate per mole of lactate at steady state, namely $J_{met\_lact} = J_{lact\_bal}$,
  ○ an acid infusion $J_{H+}$,

wherein optionally the net buffer load mathematical relation is:

$$J_{buffer\_load} = J_{met\_cit} + J_{HCO3_{bal}} + J_{met\_lact} - J_{H+}$$

wherein the significance of the denotations is given in the Glossary; if any of the mass transfer rate is missing, the corresponding mass transfer term is absent from the mathematical relation, or the corresponding value is set to zero.

In a 63$^{rd}$ aspect according to any one of the preceding aspects, said one or more mathematical relations include a bicarbonate balance mathematical relation based on a bicarbonate balance ($J_{HCO3\_bal}$) from the CRRT blood treatment to be delivered in terms of an amount per unit of time, in particular wherein the bicarbonate balance ($J_{HCO3\_bal}$) is the difference between a bicarbonate infusion rate from the dialysis liquid supply line (8) and/or the infusion lines ($J_{HCO3\_inj}$) and the bicarbonate removal into dialysate ($J_{HCO3\_dial}$), in particular the bicarbonate balance mathematical relation is:

$$J_{HCO3\_bal} = J_{HCO3\_inf} - J_{HCO3\_eff}$$

wherein the significance of the denotations is given in the Glossary; if any of the mass transfer rate is missing, the corresponding mass transfer term is absent from the mathematical relation, or the corresponding value is set to zero.

In a 64$^{th}$ aspect according to the preceding aspect, bicarbonate infusion rate ($J_{HCO3\_inf}$) from the dialysis liquid supply line (8) is a function of the fluid flow rate ($Q_{dial}$) through the dialysis liquid supply line (8) and bicarbonate concentration ($C_{HCO3dial}$) in the dialysis fluid flowing in the dialysis liquid supply line (8), in particular the fluid flow rate ($Q_{dial}$) times the bicarbonate concentration ($C_{HCO3dial}$).

In a 65$^{th}$ aspect according to any one of the preceding two aspects, bicarbonate infusion rate $J_{HCO3\_inf}$) from the infusion lines is a function of the fluid flow rate/s ($Q_{rep.pre}$; $Q_{rep.post}$; $Q_{cit}$; $Q_{PBP}$; $Q_{HCO3}$; $Q_{ca}$) and the respective bicarbonate concentration ($C_{HCO3rep.pre}$; $C_{HCO3rep.post}$; $C_{HCO3cit}$; $C_{HCO3PBP}$; $C_{HCO3HCO3}$; $C_{HCO3ca}$), in particular the fluid flow rate/s ($Q_{rep.pre}$; $Q_{rep.post}$; $Q_{cit}$; $Q_{PBP}$; $Q_{HCO3}$; $Q_{ca}$) times the respective bicarbonate concentration ($C_{HCO3rep.pre}$; $C_{HCO3rep.post}$; $C_{HCO3cit}$; $C_{HCO3PBP}$; $C_{HCO3HCO3}$; $C_{HCO3ca}$).

In a 66$^{th}$ aspect according to any one of the preceding three aspects, said one or more mathematical relations include a bicarbonate infusion mathematical relation and wherein the bicarbonate infusion rate from the dialysis liquid supply line (8) and the infusion lines ($J_{HCO3\_inf}$) is particularly according to the bicarbonate infusion mathematical relation:

$$J_{HCO3\_inf} = Q_{dial} \cdot C_{HCO3_{dial}} + Q_{rep.pre} \cdot C_{HCO3_{rep.pre}} + Q_{rep.post} \cdot C_{HCO3_{rep.post}} + Q_{HCO3}$$
$$\cdot C_{HCO3_{HCO3post}} + Q_{cit} \cdot C_{HCO3_{cit}} + Q_{PBP} \cdot C_{HCO3_{PBP}} + Q_{ca} \cdot C_{HCO3_{ca}}$$

wherein the significance of the denotations is given in the Glossary; if any of the infusion lines or the dialysis liquid supply line is missing, the corresponding flow rate is absent from the mathematical relation, or the corresponding flow rate is set to zero.

In a 67$^{th}$ aspect according to any one of the preceding four aspects, the bicarbonate removal into spent dialysate ($J_{HCO3\_eff}$) in the effluent line (13) is a function of the fluid flow rate ($Q_{dial}$) through the dialysis liquid supply line (8) and bicarbonate concentration ($C_{HCO3dial}$) in the dialysis fluid flowing in the dialysis liquid supply line (8), in particular the fluid flow rate ($Q_{dial}$) times the bicarbonate concentration ($C_{HCO3dial}$).

In a 68$^{th}$ aspect according to any one of the preceding five aspects, the bicarbonate removal into spent dialysate ($J_{HCO3\_eff}$) in the effluent line (13) is a function of bicarbonate concentration in the dialysis fluid ($C_{HCO3\_dial}$), in particular as a function of a difference between bicarbonate plasma water concentration at filter inlet ($Cpw_{HCO3\_inlet}$) and bicarbonate concentration in the dialysis fluid ($C_{HCO3\_dial}$).

In a 69$^{th}$ aspect according to any one of the preceding six aspects, the bicarbonate removal into spent dialysate ($J_{HCO3\_eff}$) in the effluent line (13) is a function of an ultrafiltration flow rate ($Q_{fil}$) in the filtration unit (2) and bicarbonate concentration ($C_{HCO3dial}$) in the dialysis fluid flowing in the dialysis liquid supply line (8), in particular the ultrafiltration flow rate ($Q_{fil}$) times the bicarbonate concentration ($C_{HCO3dial}$).

In a 70$^{th}$ aspect according to any one of the preceding seven aspects, the bicarbonate removal into spent dialysate ($J_{HCO3\_eff}$) in the effluent line (13) is a function of a bicarbonate clearance ($K_{HCO3}$).

In a 71$^{st}$ aspect according to any one of the preceding eight aspects, the bicarbonate removal into spent dialysate ($J_{HCO3\_eff}$) in the effluent line (13) is a function of the fluid flow rate ($Q_{eff}$) through the effluent fluid line (13), in particular wherein a bicarbonate clearance ($K_{HCO3}$) is estimated equal to said effluent fluid flow rate ($Q_{eff}$).

In a 72nd aspect according to any one of the preceding aspects, the bicarbonate removal into spent dialysate ($J_{HCO3\_eff}$) in the effluent line (13) is a function of a bicarbonate clearance ($K_{HCO3}$) and the bicarbonate clearance ($K_{HCO3}$) is a function of one or more flow rates, particularly including one or more of the fluid flow rate ($Q_{dial}$) through the dialysis liquid supply line (8), an ultrafiltration rate ($Q_{fil}$) in filtration unit (2), and a blood water flow rate ($Qbw_{inlet}$).

In a 73rd aspect according to the preceding aspect, the bicarbonate clearance ($K_{HCO3}$) is a function of the filtration unit (2) intended for CRRT treatment, in particular as a function of a sieving coefficient ($SC_{HCO3}$) for bicarbonate and/or a ratio of filtration unit surface area to diffusive mass transfer resistance ($S/RT_{HCO3}$) for bicarbonate.

In a 74th aspect according to any one of the preceding aspects, said one or more mathematical relations include bicarbonate clearance mathematical relations as follows:

$$K_{HCO3} = \frac{Qbw_{inlet} \cdot Q_{dial} - f_{HCO3} \cdot (Qbw_{inlet} - SC_{HCO3} \cdot Q_{fil}) \cdot (Q_{dial} + SC_{HCO3} \cdot Q_{fil})}{Q_{dial} - f_{HCO3} \cdot (Qbw_{inlet} - SC_{HCO3} \cdot Q_{fil})}$$

$$f_{HCO3} = \left( \frac{Qbw_{inlet} - SC_{HCO3} \cdot Q_{fil}}{Qbw_{inlet}} \cdot \frac{Q_{dial} + SC_{HCO3} \cdot Q_{fil}}{Q_{dial}} \right)^{\frac{1}{\gamma_{HCO3}}}$$

$$\gamma_{HCO3} = e^{\left( \frac{SC_{HCO3} \cdot Q_{fil}}{\left(\frac{S}{RT}\right)_{HCO3}} \right)} - 1$$

wherein the significance of the denotations is given in the Glossary; if any of the infusion lines or the dialysis liquid supply line is missing, the corresponding flow rate is absent from the mathematical relation, or the corresponding flow rate is set to zero.

It is noted that above equations do not work with $Q_{dial}=0$ or $Q_{fil}=0$; in such situations it is possible to remove the infinite value by (artificially) replacing 0 value for either or both $Q_{dial}$ and/or $Q_{fil}$ by e.g., 1 or 0.1 ml/h. Otherwise specific equations for pure CVVHD or pure CVVH may be alternatively used.

In a 75th aspect according to any one of the preceding aspects, said one or more mathematical relations include an ultrafiltration flow rate mathematical relation linking an ultrafiltration flow rate ($Q_{fil}$) through the filtration unit (2) with a fluid removal rate ($Q_{PFR}$) from the patient and infusion flow rate/s ($Q_{PBP}$, $Q_{cit}$, $Q_{rep.pre}$, $Q_{rep.post}$, $Q_{HCO3}$, $Q_{ca}$) in the extracorporeal blood circuit(17), in particular according to:

$$Q_{fil} = Q_{PBP} + Q_{cit} + Q_{syr} + Q_{rep.pre} + Q_{rep.post} + Q_{HCO3} + Q_{PFR} + Q_{ca}$$

wherein the significance of the denotations is given in the Glossary; if any of the infusion lines is missing, the corresponding flow rate is absent from the mathematical relation, or the corresponding flow rate is set to zero.

In a 76th aspect according to any one of the preceding aspects, said one or more mathematical relations include a blood water flow rate mathematical relation linking a blood water flow rate ($Q_{bw}$) to the blood fluid flow rate ($Q_b$) and haematocrit (Hct), in particular according to:

$$Qbw = Q_b \cdot [(1 - Hct) \cdot F_p + Hct \cdot Frbc]$$

wherein the significance of the denotations is given in the Glossary.

In a 77th aspect according to any one of the preceding aspects, said one or more mathematical relations include a blood water flow rate at filtration unit inlet mathematical relation linking blood water flow rate $Qbw_{inlet}$ at the filtration unit inlet to one or more of a blood water flow rate ($Q_{bw}$), the fluid flow rate through the anticoagulant infusion line (51), the fluid flow rate ($Q_{PBP}$) through the PBP infusion line (52), and the fluid flow rate ($Q_{rep.pre}$) through the pre-dilution infusion line (29), in particular blood water flow rate at filtration unit inlet mathematical relation being as follows:

$$Qbw_{inlet} = Q_{bw} + Q_{cit} + Q_{PBP} + Q_{rep.pre} + Q_{syr}$$

$$= Q_b \cdot [(1 - Hct) \cdot Fp + Hct \cdot Frbc] + Q_{cit} + Q_{PBP} + Q_{rep.pre} + Q_{syr}$$

wherein the significance of the denotations is given in the Glossary.

In a 78th aspect according to any one of the preceding aspects, said one or more mathematical relations include a bicarbonate removal mathematical relation, in particular wherein the bicarbonate removal mathematical relation is:

$$J_{HCO3\_eff} = Q_{dial} \cdot C_{HCO3_{dial}} + K_{HCO3} \cdot \left(Cpw_{HCO3\_inlet} - C_{HCO3\_dial}\right) + Q_{fil} \cdot C_{HCO3\_dial}$$

wherein the significance of the denotations is given in the Glossary.

In a 79th aspect according to any one of the preceding aspects, said one or more mathematical relations include a plasma water concentration at filter inlet mathematical relation correlating the bicarbonate plasma water concentration at filter inlet ($Cpw_{HCO3\_inlet}$) as a function of flow rates including one or more of blood water flow rate ($Q_{bw}$), blood water flow rate at filter inlet ($Qbw_{inlet}$), the fluid flow rate through the anticoagulant infusion line (51), the fluid flow rate ($Q_{PBP}$) through the PBP infusion line (52) and the fluid flow rate ($Q_{rep.pre}$) through the predilution infusion line (29) and/or as a function of bicarbonate concentrations ($C_{HCO3cit}$; $C_{HCO3PBP}$; $C_{HCO3rep.pre}$) in the fluid flowing in the corresponding infusion lines and patient plasma bicarbonate concentration ($Cp_{HCO3pat}$). In an 80th aspect according to the preceding aspect, said plasma water concentration at filter inlet mathematical relation is:

$$Cpw_{HCO3\_inlet}$$

$$= \frac{Q_{bw} \cdot \dfrac{Cp_{HCO3_{pat}}}{Fp} + Q_{cit} \cdot C_{HCO3_{cit}} + Q_{PBP} \cdot C_{HCO3_{PBP}} + Q_{rep.pre} \cdot C_{HCO3_{rep.pre}}}{Qbw_{inlet}}$$

wherein the significance of the denotations is given in the Glossary; if any of the infusion lines is missing, the corresponding flow rate is absent from the mathematical relation, or the corresponding flow rate is set to zero.

In an 81st aspect according to any one of the preceding aspects, said one or more mathematical relations include a bicarbonate balance mathematical relation based on a bicarbonate balance ($J_{HCO3\_bal}$) from the CRRT blood treatment to be delivered in terms of an amount per unit of time, in particular the bicarbonate balance mathematical relation is:

$$J_{HCO3\_bal} = Q_{rep.pre} \cdot C_{HCO3_{rep.pre}} + Q_{cit} \cdot C_{HCO3_{cit}} + Q_{PBP} \cdot C_{HCO3_{PBP}} + Q_{rep.post}$$
$$\cdot C_{HCO3_{rep.post}} + Q_{HCO3} \cdot C_{HCO3_{HCO3post}} - K_{HCO3}$$
$$\cdot \left(Cpw_{HCO3_{inlet}} - C_{HCO3_{dial}}\right) - Q_{fil} \cdot C_{HCO3\_dial}$$

wherein the significance of the denotations is given in the Glossary; if any of the infusion lines or the dialysis liquid supply line is missing, the corresponding flow rate is absent from the mathematical relation, or the corresponding flow rate is set to zero.

Note that the above equation assumes that no bicarbonate is present in the ion replacement (Ca) solution.

In an 82nd aspect according to any one of the preceding aspects, the net buffer load mathematical relation of aspect 62 and the bicarbonate balance mathematical relation of aspect 81 (or alternatively aspects 63 + 66 + 78) link the the parameter (nNBL; $Cp_{HCO3\_pat}$) indicative of a steady state acid-base balance in the blood with the at least two fluid flow rates selected in the group, the control unit (12) using the net buffer load mathematical relation of aspect 62 and the bicarbonate balance mathematical relation of aspect 81 (or alternatively aspects 63 + 66 + 78) to calculate the set value of said at least two fluid flow rates.

In an 83rd aspect according to the preceding aspect, the mathematical relations of aspects 75, 76, 77 and 78 are further used by the control unit (12) to calculate the set value of said at least two flow rates.

In an 84th aspect according to any one of the preceding aspects, said one or more mathematical relations include a citrate load mathematical relation based on a citrate load ($J_{cit\_load}$) to the patient in terms of an amount per unit of time, in particular wherein the citrate load ($J_{cit\_load}$) is the difference between a citrate infusion rate ($J_{cit\_PBP}$) through the anticoagulant infusion line (51) and a citrate removal rate to effluent ($J_{cit\_dial}$) in the effluent line (13), in particular the citrate load mathematical relation is:

$$J_{citrate\_load} = J_{cit\_PBP} - J_{cit\_dial}$$

wherein the significance of the denotations is given in the Glossary; if any of the mass transfer rate is missing, the corresponding mass transfer term is absent from the mathematical relation, or the corresponding value is set to zero.

In an 85<sup>th</sup> aspect according to the preceding aspect, the citrate load ($J_{cit\_load}$), in particular the citrate removal rate to effluent ($J_{cit\_dial}$), is a function of patient citrate metabolic clearance ($K_{cit\_met}$), in particular the metabolic clearance being based on, e.g., directly proportional to, a patient body weight (BW), for example being determined as follows:

$$K_{cit\_met} = 700 \cdot \frac{BW}{72}$$

wherein patient citrate metabolic clearance ($K_{cit\_met}$) is measured as [ml/min] and body weight (BW) is measured as [kg].

In an 86<sup>th</sup> aspect according to any one of the preceding two aspects, the citrate load ($J_{cit\_load}$) is a function of citrate clearance ($K_{cit}$).

An 87<sup>th</sup> aspect according to any one of the preceding three aspects, the citrate load ($J_{cit\_load}$) is a function of the fluid flow rate ($Q_{eff}$) through the effluent fluid line (13), in particular wherein a citrate clearance ($K_{cit}$) is estimated equal to said effluent fluid flow rate ($Q_{eff}$).

In an 88<sup>th</sup> aspect according to any one of the preceding aspects, the citrate load ($J_{cit\_load}$) is a function of a citrate clearance ($K_{cit}$) and, according to citrate clearance mathematical relations of said one or more mathematical relations, the citrate clearance ($K_{cit}$) is a function of one or more flow rates, particularly including one or more of the fluid flow rate ($Q_{dial}$) through the dialysis liquid supply line (8), an ultrafiltration rate ($Q_{fil}$) in filtration unit (2), and a plasma water flow rate ($Qpw_{inlet}$) at the filtration unit inlet.

In an 89<sup>th</sup> aspect according to the preceding aspect, the citrate load ($J_{cit\_load}$) is a function of the filtration unit (2) intended for CRRT treatment, in particular as a function of a sieving coefficient ($SC_{cit}$) for citrate and/or a ratio of filtration unit surface area to diffusive mass transfer resistance ($S/RT_{cit}$) for citrate.

In a 90<sup>th</sup> aspect according to the one of the preceding aspects, said one or more mathematical relations include citrate clearance mathematical relations as follows:

$$K_{cit} = \frac{Qpw_{inlet} \cdot Q_{dial} - f_{cit} \cdot (Qpw_{inlet} - SC_{cit} \cdot Q_{fil}) \cdot (Q_{dial} + SC_{cit} \cdot Q_{fil})}{Q_{dial} - f_{cit} \cdot (Qpw_{inlet} - SC_{cit} \cdot Q_{fil})}$$

$$f_{cit} = \left( \frac{Qpw_{inlet} - SC_{cit} \cdot Q_{fil}}{Qpw_{inlet}} \cdot \frac{Q_{dial} + SC_{cit} \cdot Q_{fil}}{Q_{dial}} \right)^{\frac{1}{\gamma_{cit}}}$$

$$\gamma_{cit} = e^{\left( \frac{SC_{cit} \cdot Q_{fil}}{\frac{S}{RT_{cit}}} \right)} - 1$$

wherein the significance of the denotations is given in the Glossary; if any of the infusion lines or the dialysis liquid supply line is missing, the corresponding flow rate is absent from the mathematical relation, or the corresponding flow rate is set to zero.

In a 91<sup>st</sup> aspect according to any one of the preceding aspects, said one or more mathematical relations include a plasma flow rate mathematical relation linking a plasma flow rate ($Q_p$) to the blood fluid flow rate ($Q_b$) and haematocrit (Hct), in particular according to:

$$Qp = Q_b \cdot (1 - Hct)$$

wherein the significance of the denotations is given in the Glossary.

In a 92<sup>nd</sup> aspect according to any one of the preceding aspects, said one or more mathematical relations include a plasma water flow rate mathematical relation linking a plasma water flow rate ($Q_{pw}$) to the blood fluid flow rate ($Q_b$) and haematocrit (Hct), in particular according to:

$$Qpw = Q_b \cdot (1 - Hct) \cdot F_p$$

wherein the significance of the denotations is given in the Glossary.

In a 93<sup>rd</sup> aspect according to any one of the preceding aspects, said one or more mathematical relations include a plasma water flow rate at filtration unit inlet mathematical relation linking plasma water flow rate $Qpw_{inlet}$ at the filtration unit inlet to one or more of a plasma water flow rate ($Q_{pw}$), the fluid flow rate through the anticoagulant infusion line (51), the fluid flow rate ($Q_{PBP}$) through the PBP infusion line (52), and the fluid flow rate ($Q_{rep.pre}$) through the pre-dilution infusion line (29), in particular blood water flow rate at filtration unit inlet mathematical relation being as follows:

$$Qpw_{inlet} = Qpw + Q_{cit} + Q_{syr} + Q_{PBP} + Q_{rep.pre}$$

$$= Q_b \cdot (1 - Hct) \cdot Fp + Q_{cit} + Q_{PBP} + Q_{rep.pre}$$

wherein the significance of the denotations is given in the Glossary; if any of the infusion lines or the dialysis liquid supply line is missing, the corresponding flow rate is absent from the mathematical relation, or the corresponding flow rate is set to zero.

In a 94th aspect according to any one of the preceding aspects, a citrate load ($J_{cit\_load}$) is alternatively a function of a citrate dose ($D_{cit}$) and the blood flow ($Qb$), namely according to $D_{cit} \cdot Q_b$, or a function of the citrate flow rate in the anticoagulant line (51) and a total citrate concentration ($C_{cit\_pbp}$), namely according to $Q_{cit} \cdot C_{citPBP}$. In a 95th aspect according to any one of the preceding aspects, said one or more mathematical relations include a citrate load mathematical relation linking a citrate load ($J_{cit\_load}$) with a citrate dose ($D_{cit}$) and/or the blood flow rate ($Q_b$) and/or a citrate clearance ($K_{cit}$) and/or plasma water flow rate ($Q_{pw\_inlet}$) at the inlet of the filtration unit (2), in particular wherein the citrate load mathematical relation is either:

$$J_{citrate\_load} = D_{cit} \cdot Q_b \cdot \left( 1 - \frac{K_{cit}}{Qpw_{inlet}} \right) \cdot \left( 1 - \frac{1}{1 + \frac{K_{cit\_met}}{K_{cit}} \cdot \frac{Qpw_{inlet}}{Qp}} \right)$$

or:

$$J_{citrate\_load} = D_{cit} \times Qb \times \left( 1 - \frac{K_{cit}}{Qpw_{inlet}} \right)$$

wherein the significance of the denotations is given in the Glossary.

In a 96th aspect according to any one of the preceding aspects, the net buffer load mathematical relation of aspect 67 and the citrate load mathematical relation of aspect 95 link the the parameter (nNBL; $Cp_{HCO3\_pat}$) indicative of a steady state acid-base balance in the blood with the at least two fluid flow rates selected in the group, the control unit (12) uses the net buffer load mathematical relation of aspect 67 and the citrate load mathematical relation of aspect 95 to calculate the set value of said at least two fluid flow rates.

In a 97th aspect according to the preceding aspect, the mathematical relations of aspects 85, 90, 91, 92 and 83 are further used by the control unit (12) to calculate the set value of said at least two fluid flow rates.

In a 98th aspect according to any one of the preceding aspects, the control unit (12) is configured to calculate two or more fluid flow rates selected in the group.

In a 99th aspect according to any one of the preceding aspects, the control unit (12) is configured to calculate three or more fluid flow rates selected in the group.

In a 100th aspect according to any one of the preceding aspects, the control unit (12) is configured to calculate four or more fluid flow rates selected in the group.

In a 101st aspect according to any one of the preceding aspects, the control unit (12) is configured to receive a number 'n' of clinical prescription parameters and to calculate a plurality 'm' of fluid flow rates selected in the group, wherein the plurality of fluid flow rates is higher or equal to the number clinical prescription parameters, namely m≥n.

In a 102nd aspect according to any one of the preceding aspects, the control unit (12) is configured to receive a number 'n' of clinical prescription parameters comprising said prescribed dialysis dose ($_{Dset}$) and said parameter (nNBL; $Cp_{HCO3\_pat}$) indicative of a steady state acid-base balance in the blood of the patient and one or more of:

  ○ a regional anticoagulation, e.g., citrate, dose ($D_{cit}$) plus a ion, e.g., calcium, re-establishing solution parameter (CaComp; $D_{ca}$);
  ○ a fluid removal rate ($Q_{pfr}$) from the patient; and
  ○ a blood flow rate ($Q_b$);

wherein the control unit (12) is further configured to calculate a plurality 'm' of fluid flow rates selected in the group, wherein the plurality of fluid flow rates is higher or equal to the number clinical prescription parameters, namely m≥n.

In a 103rd aspect according to any one of the preceding two aspects, if m>n, the control unit (12) provides for additional therapy configuration parameters to be selectable, said additional therapy configuration parameters adding further mathematical relations to be used for calculating the 'm' fluid flow rates, in particular, if m=n+1, one additional therapy configuration parameter is selectable, if m=n+2, two additional therapy configuration parameters are selectable.

In a 104th aspect according to any one of the preceding aspects, said further mathematical relations include one or more of a convection-diffusion relation, a blood pre-dilution relation, and a pre-post infusion relation.

In a 105th aspect according to any one of the preceding aspects, the apparatus is configured for performing regional anticoagulation treatments and comprises:

- the anticoagulant infusion line (51) connected at one end thereof to the blood withdrawal line in a region of the blood withdrawal line which is positioned in use upstream from the blood pump (21);
- a source of regional anticoagulant (10) providing an anticoagulant to the anticoagulant infusion line (51), said source including in particular citrate;
- the ion balancing infusion line (74) connected at one end thereof either to the blood return line (7) or to the patient catheter, and
- a source of ion balancing solution (11) providing an ion balancing solution to the ion balancing infusion line (74), said source including in particular calcium ions;

wherein the control unit (12) is configured to execute the flow-rate setup procedure comprising:

- receiving the patient prescription, the receiving step further including:

  ○ allowing entry of a set value for a prescribed anticoagulant dose ($D_{cit}$) to be delivered,

- determining of the operating parameters comprising calculating the at least two fluid flow rates, the set value for at least the fluid flow rate through the anticoagulant infusion line (51) and the fluid flow rate ($Q_{ca}$) through the ion balancing infusion line (74) using said one or more mathematical relations.

In a 106th aspect according to the preceding aspect, calculating the set value for at least the fluid flow rate through the anticoagulant infusion line (51) and/or the fluid flow rate ($Q_{ca}$) through the ion balancing infusion line (74), is based at least on said set value of the prescribed dialysis dose ($D_{set}$), particularly in case the set value for the blood fluid flow rate ($Q_b$) through the extracorporeal blood circuit (17) is determined by the control unit (12) as an operating parameter.

In a 107th aspect according to the preceding aspect, calculating the set value for at least the fluid flow rate through the anticoagulant infusion line (51) and/or the fluid flow rate ($Q_{ca}$) through the ion balancing infusion line (74), is based at least on said target value for the parameter (nNBL; $Cp_{HCO3\_pat}$) indicative of a steady state acid-base balance in the blood, particularly when the set value for the blood fluid flow rate ($Q_b$) through the extracorporeal blood circuit (17) is determined by the control unit (12) as an operating parameter.

In a 108th aspect according to any one of the preceding aspects, one or more of said mathematical relations correlate the parameter (nNBL; $Cp_{HCO3\_pat}$) indicative of a steady state acid-base balance in the blood of the patient to on an estimation of an amount per unit of time of bicarbonate generated from metabolism of citrate ($J_{met\_cit}$) infused to the patient, in particular wherein the metabolism of citrate load leads to 3 moles of bicarbonate per mole of citrate at steady state, namely:

$$J_{met\_cit} = 3 \cdot J_{cit\_load}.$$

In a 109th aspect according to any one of the preceding aspects, the control unit (12) is further configured to control said actuators for regulating the flow of fluid based on said set value and calculated value/s of the fluid flow rates. In a 110th aspect according to any one of the preceding aspects, the actuators for regulating the flow of fluid (24, 25, 26, 31, 53, 54, 65, 75) comprises pumps, e.g., rotational and/or peristaltic pumps, and/or valves.

In a 111th aspect according to any one of the preceding aspects, said one or more mathematical relations correlates the parameter (nNBL; $Cp_{HCO3\_pat}$) indicative of a steady state acid-base balance in the blood of the patient who has to undergo a CRRT blood treatment with the at least two fluid flow rates selected in said group.

In a 112th aspect according to any one of the preceding aspects, the control unit (12) is further configured to calculate the set values of the at least two fluid flow rates by applying said prescribed dose value ($_{Dset}$) and the target value for the parameter indicative of a steady state acid-base balance in the blood to said mathematical relations.

In a 113th aspect according to any one of the preceding two aspects, the control unit (12) is further configured to calculate the set values of the at least two fluid flow rates by applying the clinical prescription parameters to said mathematical relations.

In a 114th aspect according to any one of the preceding three aspects, the clinical prescription parameters further comprises a fluid removal rate ($Q_{pfr}$) from the patient to said mathematical relations.

In a 115th aspect according to any one of the preceding four aspects, the clinical prescription parameters further comprises a blood flow rate ($Q_b$) in the extracorporeal blood circuit (17).

In a 116th aspect according to any one of the preceding five aspects, the clinical prescription parameters further comprises the prescribed dialysis dose ($_{Dset}$) and the parameter (nNBL; $Cp_{HCO3\_pat}$) indicative of a steady state acid-base balance in the blood of the patient.

In a 117th aspect according to any one of the preceding six aspects, the clinical prescription parameters further comprises the regional anticoagulation dose ($D_{cit}$) and/or the the ion re-establishing solution parameter (CaComp; $D_{ca}$).

In a 118th aspect according to any one of the preceding aspects, the memory (16) stores a plurality of further mathematical relations correlating the at least two fluid flow rates selected in said group with each other.

In a 119th aspect according to the preceding aspect, the CRRT apparatus comprises - a preset number of predilution lines selected in the group including said pre-dilution infusion line (29) connected at one end thereof to the blood withdrawal line (6), said pre-blood pump - PBP - infusion line (52) connected at one end thereof to the blood withdrawal line in a region of the blood withdrawal line which is positioned in use upstream from the blood pump (21), and said anticoagulant infusion line (51) connected at one end thereof to the blood withdrawal line in a region of the blood withdrawal line which is positioned in use upstream from the blood pump (21);

- a preset number of post-dilution lines selected in the group including said post-dilution infusion line (63) connected at one end thereof to the blood return line (7), said post-dilution bicarbonate infusion line (23) connected at one end thereof to the blood return line (7), and said ion balancing infusion line (74) connected at one end thereof either to the blood return line (7) or to a patient catheter;

wherein said further mathematical relations stored in said memory comprise one or more of the following:

- a convection-diffusion relation, relating the total fluid flow rate ($Q_{rep.pre} + Q_{rep.post} + Q_{pbp} + Q_{HCO3} + Q_{ca} + Q_{cit}$) through said preset number of pre- and post-dilution lines with the fluid flow rate ($Q_{dial}$) through said dialysis liquid supply line (8),
- a blood pre-dilution relation, relating the flow rate of blood ($Q_b$) or of plasma ($Q_p$) and a total fluid flow rate ($Q_{rep.pre} + Q_{pbp} + Q_{cit}$) infused in the blood withdrawal line through said preset number of pre-dilution lines,
- a pre-post relation, relating a total fluid flow rate ($Q_{rep.pre} + Q_{pbp} + Q_{cit}$) through said preset number of pre-dilution lines with a total fluid flow rate ($Q_{rep.post} + Q_{HCO3} + Q_{ca}$) through said preset number of post-dilution lines.

In a 120th aspect according to the preceding aspect, the convection-diffusion relation defines a first split between the total fluid flow rate ($Q_{rep.pre} + Q_{rep.post} + Q_{pbp} + Q_{HCO3} + Q_{ca} + Q_{cit}$) through said preset number of pre- and post-dilution lines and the fluid flow rate ($Q_{dial}$) through said dialysis liquid supply line (8), in particular the convection-diffusion relation defines a first percentage split or a first ratio R1 dividing the total fluid flow rate ($Q_{rep.pre} + Q_{rep.post} + Q_{pbp} + Q_{HCO3} + Q_{ca} + Q_{cit}$) through said preset number of pre- and post-dilution lines by the fluid flow rate ($Q_{dial}$) through said dialysis liquid supply line (8).

In a 121st aspect according to any one of the preceding two aspects, the blood pre-dilution relation defines a second ratio R2 dividing the total fluid flow rate ($Q_{rep.pre} + Q_{pbp} + Q_{cit}$) infused in the blood withdrawal line (6) through said preset number of pre-dilution lines by the flow rate of blood ($Q_b$) or of plasma ($Q_p$).

In a 122nd aspect according to any one of the preceding three aspects, the pre-post relation defines a third split between the total fluid flow rate ($Q_{rep.pre} + Q_{pbp} + Q_{cit}$) through said preset number of pre-dilution lines and total fluid flow rate ($Q_{rep.post} + Q_{HCO3} + Q_{ca}$) through said preset number of post-dilution lines, in particular the pre-post relation defines a third percentage split or a third ratio R3 dividing the total fluid flow rate ($Q_{rep.pre} + Q_{pbp} + Q_{cit}$) through said preset number of pre-dilution lines by total fluid flow rate ($Q_{rep.post} + Q_{HCO3} + Q_{ca}$) through said preset number of post-dilution lines.

In a 123rd aspect according to any one of the preceding three aspects, the control (12) unit is further configured to: store a preset value or preset range for each one of said first, second and third splits/ratios R1, R2, R3.

In a 124th aspect according to any one of the preceding four aspects, the control unit (12) is further configured to allow entry by an operator of a set value or a set range for each one of said first, second and third splits/ratios R1, R2, R3.

In a 125th aspect according to any one of the preceding seven aspects, the control unit (12) is further configured to allow the user to select one or more therapy configurations, said therapy configurations comprising at least one of said further mathematical relations, and optionally two or three of said further mathematical relations, and to calculate the set values of at least two of said fluid flow rates by applying the set value of the prescribed dose ($_{Dset}$) and the target value of the parameter (nNBL; $Cp_{HCO3\_pat}$) indicative of a steady state acid-base balance in the blood of the patient entered by the operator to both the mathematical relations and the further mathematical relation/s selected by the user.

In a 126th aspect according to any one of the preceding aspects, the control unit (12) is further configured to allow the user to select one or more therapy configurations, wherein selecting said therapy configurations comprises choosing the parameter (nNBL; $Cp_{HCO3\_pat}$) indicative of a steady state acid-base balance in the blood of the patient who has to undergo a CRRT blood treatment between a net buffer load (nNBL; NBL) and a steady state patient bicarbonate ($Cp_{HCO3\_pat}$).

In a 127th aspect according to any one of the preceding aspects, the control unit (12) is further configured to allow the user to select one or more therapy configurations, wherein selecting said therapy configurations comprises choosing a definition for the prescribed dialysis dose ($D_{set}$).

In a 128th aspect according to any one of the preceding aspects, the control unit (12) is further configured to allow the user to select one or more therapy configurations, wherein selecting said therapy configurations comprises choosing blood flow rate as clinical prescription parameter or as operating parameter.

In a 129th aspect according to any one of the preceding aspects, the control unit (12) is further configured to allow the user to enter treatment configuration values including receiving entries for one or more of:

- patient body weight (BW);
- patient hematocrit (Hct);
- filtration unit parameters, such as filter mass transfer coefficient by surface area (K0.A), sieving coefficient (SC), solute clearance ($K_{sol}$), filtration unit surface (S), diffusive mass transfer resistance (RT) or any combination thereof;
- substance concentration for one or more containers feeding said lines (29, 63, 23, 74, 8, 52, 51), in particular bicarbonate concentration and/or lactate concentration and/or citrate concentration and/or calcium concentration. In a 130th aspect according to the preceding aspect, the control unit (12) is further configured to calculate the set values of the at least two fluid flow rates by applying the treatment configuration values to said mathematical relations.

In a 131st aspect according to any one of the preceding two aspects, said substance concentration for one or more containers is entered by an operator for each respective container through a user interface (15) connected to the control unit (12), and/or said substance concentration in the fluid is determined by the control unit (12) associating an identification code on each respective container to a respective substance concentration.

In a 132nd aspect according to any one of the preceding three aspects, said filtration unit parameters for the filtration unit are entered by an operator, and/or said filtration unit parameters are determined by the control unit (12) associating an identification code on the filtration unit (2) to said filtration unit parameters.

In a 133rd aspect according to any one of the preceding aspects, the apparatus comprises the dialysis liquid supply line (8) connected to the inlet of the secondary chamber, wherein the control unit is configured to calculate the set value for the fluid flow rate ($Q_{dial}$) in the dialysis liquid supply line (8), which is not set by the operator, based on at least a part of the clinical prescription parameters, in particular the target value for the parameter (nNBL; $Cp_{HCO3\_pat}$) indicative of a steady state acid-base balance set by the operator and/or on said prescribed dialysis dose value ($D_{set}$).

In a 134th aspect according to any one of the preceding aspects, the apparatus comprises the pre-dilution infusion line (29) connected at one end thereof to the blood withdrawal line (6), in particular the blood pump (21) being active in correspondence of a segment of the blood withdrawal line (6) and the pre-dilution infusion line (29) being connected to the blood withdrawal line (6) downstream the blood pump (21), wherein the control unit is configured to calculate the set value for the fluid flow rate ($Q_{rep.pre}$) in the pre-dilution infusion line (29), which is not set by the operator, based on at least a part of the clinical prescription parameters, in particular the target value for the parameter (nNBL; $Cp_{HCO3\_pat}$) indicative of a steady state acid-base balance set by the operator and/or on said prescribed dialysis dose value ($D_{set}$).

In a 135th aspect according to any one of the preceding aspects, the apparatus comprises the post-dilution infusion line (63) connected at one end thereof to the blood return line (7), wherein the control unit is configured to calculate the set value for the fluid flow rate ($Q_{rep.post}$) in the post-dilution infusion line (63), which is not set by the operator, based on at least a part of the clinical prescription parameters, in particular the target value for the parameter (nNBL; $Cp_{HCO3\_pat}$) indicative of a steady state acid-base balance set by the operator and/or on said prescribed dialysis dose value ($D_{set}$).

In a 136th aspect according to any one of the preceding aspects, the apparatus comprises the post-dilution bicarbonate infusion line (23) connected at one end thereof to the blood return line (7), wherein the control unit is configured to calculate the set value for the fluid flow rate ($Q_{HCO3}$) in the post-dilution bicarbonate infusion line (23), which is not set by the operator, based on at least a part of the clinical prescription parameters, in particular the target value for the parameter (nNBL; $Cp_{HCO3\_pat}$) indicative of a steady state acid-base balance set by the operator and/or on said prescribed dialysis dose value ($D_{set}$).

In a 137th aspect according to any one of the preceding aspects, the apparatus comprises the ion balancing infusion line (74) connected at one end thereof either to the blood return line (7) or to a patient catheter, wherein the control unit is configured to calculate the set value for the fluid flow rate ($Q_{ca}$) in the ion balancing infusion line (74), which is not set by the operator, based on at least a part of the clinical prescription parameters, in particular the target value for the parameter (nNBL; $Cp_{HCO3\_pat}$) indicative of a steady state acid-base balance set by the operator and/or on said prescribed dialysis dose value ($D_{set}$).

In a 138th aspect according to any one of the preceding aspects, the apparatus comprises the pre-blood pump - PBP - infusion line (52) connected at one end thereof to the blood withdrawal line in a region of the blood withdrawal line which is positioned in use upstream from the blood pump (21), in particular the blood pump (21) being active in correspondence of a segment of the blood withdrawal line (6), wherein the control unit is configured to calculate the set value for the fluid flow rate ($Q_{PBP}$) in the pre-blood pump - PBP - infusion line (52), which is not set by the operator, based on at least a part of the clinical prescription parameters, in particular the target value for the parameter (nNBL; $Cp_{HCO3\_pat}$) indicative of a steady state acid-base balance set by the operator and/or on said prescribed dialysis dose value ($D_{set}$).

In a 139th aspect according to any one of the preceding aspects, the apparatus comprises the anticoagulant infusion line (51) connected at one end thereof to the blood withdrawal line in a region of the blood withdrawal line which is positioned in use upstream from the blood pump (21), in particular the blood pump (21) being active in correspondence of a segment of the blood withdrawal line (6), wherein the control unit is configured to calculate the set value for the fluid flow rate in the anticoagulant infusion line (51), which is not set by the operator, based on at least a part of the clinical prescription parameters, in particular the target value for the parameter (nNBL; $Cp_{HCO3\_pat}$) indicative of a steady state acid-base balance set by the operator and/or on said prescribed dialysis dose value ($D_{set}$).

In a 140th aspect according to any one of the preceding aspects, the operating parameters further comprises a blood flow rate ($Q_b$) in the extracorporeal blood circuit (17), wherein the control unit is configured to calculate the set value for the blood flow rate ($Q_b$) in the extracorporeal blood circuit (17), which is not set by the operator, based on at least a part of the clinical prescription parameters, in particular the target value for the parameter (nNBL; $Cp_{HCO3\_pat}$) indicative of a steady state acid-base balance set by the operator and/or on said prescribed dialysis dose value ($D_{set}$).

In a 141st aspect according to any one of the preceding aspects, the control unit (12) is configured to calculate three or more, and in particular four or more, set values of the fluid flow rates selected in the group.

In a 142nd aspect according to any one of the preceding aspects, the control unit (12) is configured to calculate the set value for a patient fluid removal rate ($Q_{PFR}$) from the patient.

In a 143rd aspect according to any one of the preceding aspects, said prescribed dialysis dose value ($D_{set}$) comprises a prescribed value for a flow rate or a combination of flow rates.

In a 144th aspect according to any one of the preceding aspects, said prescribed dialysis dose value ($D_{set}$) comprises a prescribed value for one selected in the group including:

- an effluent dose flow rate ($D_{eff\_set}$), which is the prescribed value of the flow rate through the effluent line (13),
- a convective dose flow rate ($D_{conv\_set}$), which is the prescribed value of the sum of the flow rates through all infusion lines ($Q_{rep.pre}$, $Q_{rep.post}$, $Q_{PBP}$, $Q_{cit}$, $Q_{ca}$, $Q_{HCO3}$) and the patient fluid removal rate ($Q_{pfr}$), optionally wherein the prescribed convective dose flow rate value is corrected for predilution,
- a diffusive dose flow rate ($D_{dial\_set}$), which is the prescribed value of the flow rate through the dialysis fluid line ($Q_{dial}$),
- an urea dose ($D_{urea\_set}$), which is a prescribed value for an estimated urea clearance,
- a clearance dose ($K_{solute\_set}$), which is a prescribed value for an estimated clearance for a given solute.

In a 145th aspect according to the preceding aspect, the control unit (12) is configured to correct the selected one of the above defined doses to take into account a predilution effect, when a infusion line (29; 51; 52) is present and delivers fluid upstream from the treatment unit (2), by multiplying the dose value times a dilution factor ($F_{dilution}$), which is < then 1, as per the following formula:

$$Dose_{corr\_xxx} = F_{dilution} \times Dose_{\_xxx}$$

(with xxx = eff, conv, dial).

In a 146th aspect according to any one of the preceding aspects, the control unit (12) is configured to control the blood pump (21) using either the entered or the calculated set value for the blood flow ($Q_b$).

In a 147th aspect according to any one of the preceding aspects, the CRRT apparatus further comprises a graphic user interface (15) connected to said control unit (12), said control unit being configured to:

- display on the graphic user interface an indicium prompting a user to enter or select the set value for said prescribed dialysis dose ($D_{set}$),
- display on the graphic user interface an indicium prompting a user to enter or select the target value for said parameter (nNBL; $Cp_{HCO3\_pat}$) indicative of a steady state acid-base balance in the blood of the patient,
- display on the graphic user interface an indicium allowing selection of one or more further mathematical relations the user intends to select, including displaying an indicium allowing a user to enter or select a set value for the mathematical relation/s, in particular to enter or select one or more of said first, second and third ratios,

- detecting selection of the further mathematical relation, and
- using said further mathematical relation to calculate the set value of the at least two fluid flow rates selected in the group.

In a 148<sup>th</sup> aspect according to any one of the preceding aspects, the actuators for regulating the flow of fluid through said fluid lines comprises a pre-infusion pump (31) for regulating the flow through said pre-dilution infusion line (29) and/or a post-infusion pump (65) for regulating the flow through said post-dilution infusion line (63).

In a 149<sup>th</sup> aspect according to any one of the preceding aspects, a dialysis liquid supply line (8) is connected to the inlet of the secondary chamber (4), and the actuators for regulating the flow of fluid through said fluid lines comprises at least a dialysis fluid pump (25) for regulating the flow through said dialysis liquid supply line (8).

In a 150<sup>th</sup> aspect according to any one of the preceding aspects, the actuators for regulating the flow of fluid through said fluid lines comprises PBP infusion pump (53) for regulating the flow through said pre-blood pump - PBP - infusion line (52) and/or an anticoagulant pump (54) for regulating the flow through said anticoagulant infusion line (51).

In a 151<sup>st</sup> aspect according to any one of the preceding aspects, the actuators for regulating the flow of fluid through said fluid lines comprises a bicarbonate pump (24) for regulating the flow through said post-dilution bicarbonate infusion line (23) and/or an ion balancing pump (75) for regulating the flow through said ion balancing infusion line (74).

In a 152<sup>nd</sup> aspect according to any one of the preceding aspects, the CRRT apparatus further comprises a collection container (62) connected to an end of the effluent fluid line (13) to collect spent dialysate.

In a 153<sup>rd</sup> aspect according to any one of the preceding aspects, the CRRT apparatus further comprises a replacement container (30) of fresh fluid connected to an end of the pre-dilution infusion line (29) ), in particular said container (30) being a bag containing a replacement solution.

In a 154<sup>th</sup> aspect according to any one of the preceding aspects, the CRRT apparatus further comprises an auxiliary container (64) of fresh fluid connected to an end of the post-infusion line (63), in particular said container (64) being a bag containing a replacement solution.

In a 155<sup>th</sup> aspect according to any one of the preceding aspects, the CRRT apparatus further comprises a liquid container (14) of fresh fluid connected to an end of the dialysis liquid supply line (8), in particular said container (14) being a bag containing fresh dialysis fluid.

In a 156<sup>th</sup> aspect according to any one of the preceding aspects, the CRRT apparatus further comprises a PBP container (18) of fresh fluid connected to an end of the pre-blood pump infusion line (52), in particular said container (18) being a bag containing a replacement solution.

In a 157<sup>th</sup> aspect according to any one of the preceding aspects, the CRRT apparatus further comprises a container (10) for regional anticoagulant connected to an end of the anticoagulant infusion line (51), in particular wherein said container (10) is a bag and contains citrate, and optionally citric acid.

In a 158<sup>th</sup> aspect according to any one of the preceding aspects, the CRRT apparatus further comprises a bicarbonate container (24) connected to an end of the post-dilution bicarbonate infusion line (23), in particular said container (24) being a bag containing a bicarbonate solution, optionally a concentrated bicarbonate solution.

In a 159<sup>th</sup> aspect according to any one of the preceding aspects, the CRRT apparatus further comprises an ion balancing solution container (11) connected to an end of the ion balancing infusion line (74), in particular said container (11) being a bag or a syringe containing a calcium concentrated solution.

In a 160<sup>th</sup> aspect according to any one of the preceding aspects, the CRRT apparatus further comprises one or more scales weighing one or more of said containers, in particular the apparatus comprising a corresponding scale for each respective of said containers, said one or more scales being connected to the control unit and sending to the control unit corresponding weight signals.

In a 161<sup>st</sup> aspect according to any one of the preceding aspects, the memory (16) stores one or a plurality of optimization criteria, said control unit (12) being configured to calculate the set value for the fluid flow rate/s by applying the optimization criteria.

In a 162<sup>nd</sup> aspect according to the preceding aspect, the optimization criteria comprises a first optimization criterion imposing that an emptying time of at least one among the containers (10; 18; 30; 64; 28; 11; 14) of fresh fluid and/or a filling time of the collection container (62) is substantially same as or multiple of the emptying time of one or more of the other containers of fresh fluid.

In a 163<sup>rd</sup> aspect according to any one of the preceding two aspects, the optimization criteria comprises a second optimization criterion imposing that fluid consumption through said fluid lines is minimized.

In a 164<sup>th</sup> aspect according to any one of the preceding three aspects, the optimization criteria comprises a third optimization criterion imposing that a life time of said filtration unit (2) is maximized.

In a 165<sup>th</sup> aspect according to any one of the preceding four aspects, the optimization criteria comprises a fourth optimization criterion imposes that urea clearance or dialysance of a given solute is maximized.

In a 166<sup>th</sup> aspect according to any one of the preceding five aspects, the control unit (12) is configured to allow the user selecting one or more of said criteria and calculate said at least one flow rate using said selected criteria.

In a 167th aspect according to any one of the preceding six aspects, the control unit (12) is configured to allow the user selecting one or more of said criteria and one or more of said further mathematical relations and to calculate at least three or more flow rates using said selected criteria and said selected further mathematical relations.

In a 168th aspect according to any one of the preceding aspects, the control unit (12) is configured to determine if said selected further mathematical relations are compatible or conflicting with each other and then:

> in case the selected further mathematical relations are compatible, calculate the flow rate/s based on the selected further mathematical relations and the clinical prescription parameters,
> in case one or more of the selected further mathematical relations are conflicting with each other, execute one or more of the following sub-steps:

- inform the user,
- allow the user to assign a priority to each of the selected further mathematical relations,
- assign a priority ranking to the selected further mathematical relations, said priority ranking being either predetermined or user adjustable, and then ignore further mathematical relations as soon as flow rates have been calculated from the prioritized further mathematical relations,
- define a compromise between further mathematical relations using preset rules.

In a 169th aspect according to any one of the preceding aspects, the control unit (12) is configured to store in the memory (16) the volume or weight of fluid which is contained in each container (10; 18; 30; 64; 28; 11; 14) of fresh fluid and optionally in the collection container (62).

In a 170th aspect according to the preceding aspect, said volume or weight of fluid is detected by a sensor associated to each respective container and connected to the control unit (12).

In a 171st aspect according to the preceding aspect, wherein said volume of fluid is detected based on signals associated to the corresponding pump acting on the infusion line fluidly connected to the respective container, for example based on signals from a Hall sensor associated to a peristaltic pump or based on plunger position in a syringe pump.

In a 172nd aspect according to the preceding aspect, wherein said volume of fluid is detected based on signals associated to the corresponding pump in one or more of:

- the ion balancing infusion line (74);
- the syringe fluid line (22);
- the anticoagulant infusion line (51).

In a 173rd aspect according to the preceding aspect 170, wherein said sensor is a weight scale weighing at least one of said containers.

In a 174th aspect according to the preceding aspect 170, wherein said sensor is a droplet counter determining weight and/or volume based on drops count.

In a 175th aspect according to any one of the preceding aspects, a data carrier is provided comprising instructions which when executed by the control unit of an apparatus according to any one of the preceding apparatus aspects render said control unit configured to execute the respective steps described in the preceding aspects.

## DESCRIPTION OF THE DRAWINGS

Aspects of the invention are shown in the attached drawings, which are provided by way of non-limiting example, wherein:

> Figure 1 shows a CRRT extracorporeal blood treatment apparatus according to aspects of the disclosure;
> Figures 2-6 show schematic representations of blood treatment apparatuses according to aspects of the disclosure;
> Figure 7 is a flowchart showing calculation of set flow rates in a CRRT extracorporeal blood treatment apparatus, e.g. of the type of figures 1-6, according to aspects of the invention.

## DETAILED DESCRIPTION

As mentioned, extracorporeal blood treatment (dialysis) may be used in patients with rapidly developing loss of kidney function, called acute renal failure or slowly worsening kidney function, called Stage 5 chronic kidney disease (or end-stage renal disease). In the following description, some embodiments of extracorporeal blood treatment apparatuses will be firstly described being suitable, or designed, principally for intensive care treatments. Citrate regional anticoagulation will be thereafter introduced followed by definition of steady state acid-base balance parameter/s. The methods to simplify and/or assist CRRT prescription on the basis of meaningful clinical parameters are thereafter described and may be implemented in any of the described embodiments as it is apparent from the following description.

**Definitions**

Unless defined otherwise, all technical and scientific terms used have the same meaning as commonly understood by one of ordinary skill in the art.

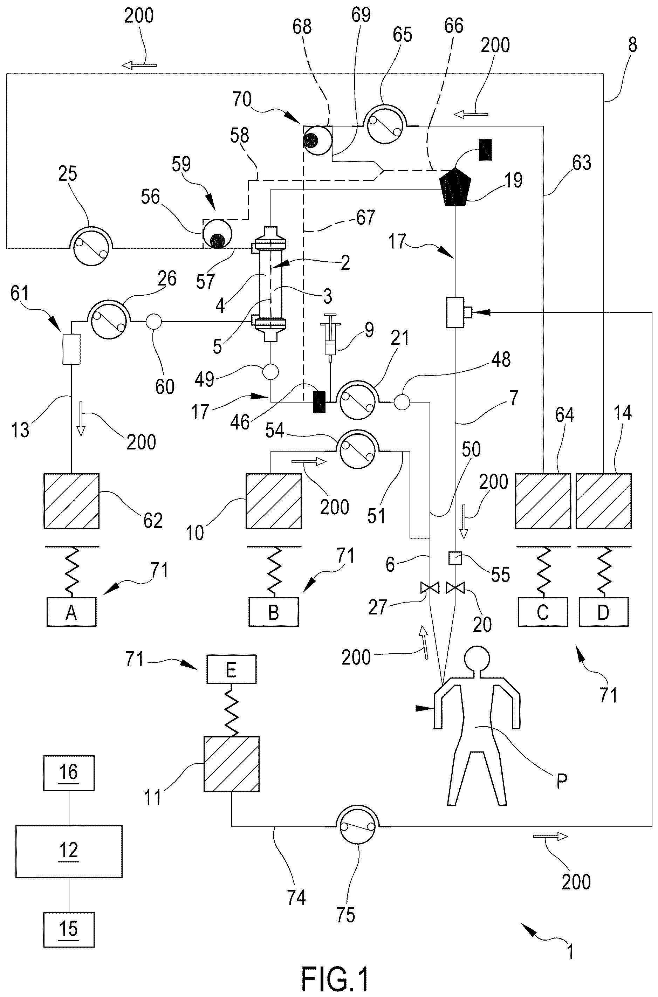

The term "downstream" refers to a position of a first component in a flow path relative to a second component wherein fluid will pass by the second component prior to the first component during normal operation. The first component can be said to be "downstream" of the second component, while the second component is "upstream" of the first component. Figure 1 shows the fluid circulation directions (indicated with reference 200) during normal operation of the apparatus 1.

We define the "dialysis fluid" as the treatment fluid introduced to the second chamber of the filtration unit 2. The dialysis fluid may be on-line prepared or pre-packaged in sterile bags. Usually in CRRT apparatuses/applications the dialysis fluid, but also the replacement fluids (possibly also regional anticoagulant fluid and/or ion re-establishing solution fluid) are contained in (disposable) bags.

We define the "dialysate" or "effluent" as the fluid from the outlet from the second chamber of the filtration unit 2. Dialysate or effluent is the spent dialysis fluid, comprising the uremic toxins removed from the blood and may include ultrafiltrate fluid.

We define "regional anticoagulant" as a substance which, once mixed with extracorporeal blood, substantially prevents blood coagulation in the extracorporeal blood circuit and which is quickly metabolized by the patient, thus avoiding systemic anticoagulation.

We define "net buffer load" during the extracorporeal blood treatment (e.g., CRRT) the combination of bicarbonate generated from the metabolism of bicarbonate precursors, such as citrate and/or lactate infused into the patient ($J_{met\_cit}$; $J_{met\_lact}$), bicarbonate balance from the extracorporeal blood therapy ($J_{HCO3\_bal}$) which may match with net loss or net gain for the patient, and acid infusion, e.g., from citric acid content of the anticoagulant solution, when relevant. From the mathematical point of view, the general definition of net buffer load (mmol/h) used hereinafter is:

$$J_{buffer\_load} = J_{met\_cit} + J_{HCO3\_bal} + J_{met\_lact} - J_{H+}$$

We define "citrate dose" as the injected amount of citrate per liter of blood treated (mmol/L blood); it defines the intensity of citrate anticoagulation.

We define patient "citrate load" as the rate at which citrate is returned to the patient (mmol/h).

$$J_{citrate\_load} = J_{cit\_PBP} - J_{cit\_eff}$$

We define "bicarbonate balance" as the net infusion or loss rate of bicarbonate in the extracorporeal blood treatment matching with the difference between the infusion rate from the dialysate and/or replacement fluids and the bicarbonate removal rate into dialysate.

$$J_{HCO3\_bal} = J_{HCO3\_inf} - J_{HCO3\_eff}$$

We define "calcium compensation" (or calcium compensation parameter) as the relative dosage of calcium infusion to compensate for the estimated calcium loss in dialysate, expressed in percentage.

We define "total calcium concentration", particularly in the effluent as the total amount of calcium per unit of liquid volume in the effluent, including both ionized and bound calcium.

We define "$K_0A$" as the mass transfer-area coefficient of a filtration unit, wherein $K_0$ is the clearance at infinite blood and dialysis fluid flow rates and A is the filtration unit surface area. "$K_0A$" is specific of a given solute and thereby changes according to the solute which is specifically considered.

In this application the term "citrate" means that the component is in form of a salt of citric acid, such as sodium, magnesium, calcium, or potassium salt thereof. The citric acid (denoted $C_6H_8O_7$) is deprotonated stepwise, therefore the "citrate" include all the different forms, citrate (denoted $C_6H_5O_7^{3-}$), hydrogen citrate (denoted $C_6H_6O_7^{2-}$), and dihydrogen citrate (denoted $C_6H_7O_7^{-}$).

The term "citrate" or "total citrate" means the total amount of citric acid and any salts thereof, such as its sodium, magnesium, calcium, or potassium salt thereof. In other terms, "total citrate" is the sum of free citrate ions and citrate containing complexes and ion pairs.

The term "buffer agent" means bicarbonate or bicarbonate precursors such as lactate, citrate or acetate.

We define "number of degrees of freedom" as the difference between the numbers of operating flow rates to be computed minus the number of prescription parameters.

**Glossary**

The following terms/parameters are consistently used throughout the equations provided in the following description of the detailed working of the extracorporeal blood treatment apparatus.

| Parameters | |
|---|---|
| $BW$ | patient body weight (kg) |
| $C$ | solute concentration (mM) |
| $C_{cit\_PBP}$ | total citrate concentration in citrate anticoagulation solution (sodium citrate + citric acid) - (mM) |
| $C_{ca}$ | calcium concentration of the ion balancing solution (mmol/l) |
| $C_{ca_{rep.post}}$ | calcium concentration of the replacement solution in post-dilution (mmol/l) |
| $C_{ca_{HCO3}}$ | calcium concentration in the bicarbonate container/infusion line (mmol/l) |
| $C_p$ | plasma concentration (mM) |
| $C_{pw}$ | plasma water concentration (mM) |
| $Cpw_{cit\_inlet}$ | citrate concentration in plasma water at the filter inlet (mM) |
| $Cp_{cit\_pat}$ | patient systemic citrate concentration (mM) |
| $C_{HCO3_{cit}}$ | bicabonate concentration in citrate solution (mM) |
| $C_{HCO3_{PBP}}$ | bicarbonate concentration in PBP solution (mM) |
| $C_{HCO3_{dial}}$ | bicarbonate concentration in dialysis fluid (mM) |
| $C_{HCO3_{rep.pre}}$ | bicarbonate concentration in pre-dilution line (mM) |
| $C_{HCO3_{rep.post}}$ | bicarbonate concentration in post-dilution line (mM) |
| $C_{HCO3_{HCO3post}}$ | bicarbonate concentration in bicarbonate post-dilution line (mM) |
| $Cp_{HCO3\_pat}$ | patient systemic bicarbonate plasma concentration (mM) ($\leftrightarrow$ [HCO$_3^-$]$_{pat.eq}$) |
| $Cpw_{HCO3\_put}$ | patient systemic bicarbonate plasma water concentration (mM) |
| $Cpw_{HCO3\_inlet}$ | bicarbonate plasma water concentration at filter inlet (mM) |
| $C_{lact\_dial}$ | lactate concentration in dialysis fluid (mM) |
| $C_{lact_{rep.pre}}$ | lactate concentration in pre-dilution line (mM) |
| $C_{lact_{rep.post}}$ | lactate concentration in post-dilution line (mM) |
| $Cp_{lact\_pat}$ | patient systemic lactate plasma concentration (mM) |
| $Cpw_{lact\_inlet}$ | lactate plasma water concentration at filter inlet (mM) |
| $D_{CRRT}$ | CRRT dialysis dose (ml/h) |
| $D_{eff}$ | Effluent dose (ml/h) |
| $D_{conv}$ | convective dose (ml/h) |
| $D_{dial}$ | diffusive dose (ml/h) |
| $D_{urea}$ | urea dose (ml/h) |
| $D_{sol}$ | clearance dose (ml/h) |
| $NDose_{xxx}$ | Normalized dose (ml/kg/h) |
| $D_{cit}$ | citrate dose (mmol/L of blood) |
| $D_{ca}$ | calcium dose; calcium concentration in the effluent (mmol/l) |
| $Hct$ | haematocrit (dimensionless, $\in$ [0; 1]) |

(continued)

| Parameters | |
|---|---|
| $J_{xxx}$ | mass transfer rate of 'xxx' solute (amount per unit of time) - (mmol/h) |
| $J_{HCO3\_bal}$ | Bicarbonate balance from the extracorporeal blood therapy - (mmol/h) |
| $J_{HCO3\_inf}$ | Bicarbonate infusion rate from the dialysis and/or all replacement fluids - (mmol/h) |
| $J_{HCO3\_eff}$ | Bicarbonate removal rate into effluent - (mmol/h) |
| $J_{H+}$ | Acid infusion in the extracorporeal blood therapy - (mmol/h) |
| $J_{citrate\_load}$ | Citrate load or the net infusion rate of citrate to the patient - (mmol/h) |
| $J_{cit\_PBP}$ | Citrate infusion rate from the pre-blood-pump anticoagulant circuit - (mmol/h) |
| $J_{cit\_dial}$ | Citrate losses in the effluent - (mmol/h) |
| $J_{met\_cit}$ | Bicarbonate generated from the metabolism of citrate infused to the patient - (mmol/h) |
| $J_{lact\_bal}$ | Lactate balance from the extracorporeal blood therapy - (mmol/h) |
| $J_{lact\_inf}$ | Lactate infusion rate from the dialysis and/or all replacement fluids - (mmol/h) |
| $J_{lact\_eff}$ | Lactate removal rate into effluent - (mmol/h) |
| $J_{met\_lact}$ | Bicarbonate generated from the metabolism of lactate infused to the patient - (mmol/h) |
| $J_{buffer\_load}$ | Net buffer load during extracorporeal therapies - (mmol/h) |
| $SC_{urea}$ | filter sieving coefficient for urea |
| $SC_{cit}$ | filter sieving coefficient for citrate |
| $SC_{HCO3}$ | filter sieving coefficient for bicarbonate |
| PRE | pre-infusion ratio of replacement fluid flow (dimensionless, $\in [0; 1]$) |
| Q | flow rate (ml/min) |
| $Q_b$ | blood flow rate (ml/min) |
| $Q_{PBP}$ | Pre-Blood-Pump flow rate (ml/min) |
| $Q_{cit}$ | citrate solution flow rate (ml/min) |
| $Q_{HCO3}$ | bicarbonate post infusion solution flow rate (ml/min) |
| $Q_{rep.pre}$ | replacement flow rate / pre-filter (ml/min) |
| $Q_{rep.post}$ | replacement flow rate / post-filter (ml/min) |
| $Q_{PFR}$ | Patient Fluid Removal (PFR) rate (ml/min) |
| $Q_{rep}$ | total replacement flow rate ($Q_{rep.pre}+Q_{rep.post}$) (ml/min) |
| $Q_{pre}$ | total predilution infusion flow rate (ml/min) |
| $Q_{syr}$ | syringe flow rate (ml/min) |
| $Q_{bw}$ | blood water flow rate (ml/min) |
| $Q_{bw-inlet}$ | blood water flow rate at the filter inlet (ml/min) |
| $Q_p$ | plasma flow rate (ml/min) |
| $Q_{pw}$ | plasma water flow rate (ml/min) |
| $Q_{pw-inlet}$ | plasma water flow rate at filter inlet (ml/min) |
| $Q_{eff}$ | effluent flow rate (ml/min) |
| $Q_{fil}$ | ultrafiltration rate in CRRT filter (ml/min) |
| $Q_{dial}$ | dialysis flow rate (ml/min) |
| $Q_{ca}$ | calcium solution flow rate (ml/min) |
| $S/RT_{xxx}$ | ratio of filter surface area to diffusive mass transfer resistance (ml/min) for solute 'xxx' ($\leftrightarrow$'$K0.A_{xxx}$') |

(continued)

| Parameters | |
|---|---|
| CaComp | calcium compensation parameter (dimensionless, $\in$ [5%; 200%]) |
| $F_{dilution_{blood}}$ | blood dilution factor (dimensionless) |
| $F_{dilution_{plasma}}$ | plasma dilution factor (dimensionless) |
| $F_{dilution_{pw}}$ | plasma water dilution factor (dimensionless) |
| | |
| **Indices** | |
| cit | citrate |
| $HCO_3$ | bicarbonate |
| lact | lactate |
| Ca | calcium |
| inlet | filter blood inlet |
| PBP | Pre Blood-Pump |
| dial | dialysis fluid |
| rep | replacement |
| rep.pre | pre-dilution replacement infusion |
| rep.post | post-dilution replacement infusion |
| eff | Effluent or dialysate |
| | |
| **Constants** | Below indicated fixed value may be changed based on specific needs |
| $Cp_{HCO_3}0=25$ mM | patient plasma bicarbonate concentration (mM) |
| $Cp_{ilact}0=1.5$mM | patient plasma lactate concentration (mM) |
| nNBL0=0,1 mmol/h/kg | Set normalized net buffer load at patient steady state |
| $F_{cal*}=0.92$ | fraction of patient plasma total calcium made available in plasma for transfer through filter membrane after citrate infusion (dimensionless) |
| $F_p=0.95$ | plasma water volume fraction |
| $F_{rbc}=0.85$ | intra erythrocyte water volume fraction |
| $F_{cal}=F_{cal*}/F_p$ | fraction of patient plasma total calcium made available in plasma water for transfer through filter membrane after citrate infusion (dimensionless) |

It is noted that values for the above constants are exemplificative. For example, patient plasma bicarbonate concentration may be set at a different level based on medical staff decision e.g., 27 mM; the same occurs with normalized net buffer load that may assume different values such as 0.15 mmol/h/kg. Further, other constants may be more (or less) precisely estimated without affecting the other part of the present description.

**Extracorporeal blood treatment apparatus particularly for CRRT treatments**

With reference to figure 1, the numeral 1 globally refers to the extracorporeal blood treatment apparatus, in particular for intensive care therapies. The extracorporeal blood treatment apparatus 1 is designed for delivering any one of treatments like hemodialysis, hemofiltration, hemodiafiltration, ultrafiltration. The apparatus according to figure 1 is particularly designed for continuous renal replacement therapies (CRRT). CRRT systems are configured for delivering very specific treatments designed for patients versing in acute states of illness and who have temporarily lost their kidney function in its entirety. In this respect, CRRT systems may be structurally and/or operationally different from extracorporeal blood treatment systems designed for chronic patient care. In contrast to chronic patients, acute patients temporarily experience complete loss of their kidney function typically due to a contemporaneous state of severe injury or during recovery from surgery. Consequently, acute patients are often extremely weak and typically not in a condition to be submitted to regular dialysis treatment, which could further deteriorate their state and lead to serious and possibly life-threatening complications. Under circumstances as described, CRRT systems are designed to individually treat a patient exhibiting very poor health, without inducing further stress to the patient body, in particular without allowing vital parameters pertaining to the patient's blood to deviate from ideal or near-ideal values. Within the scope of this document CRRT systems are, thus, inherently characterized by one or more of the following features. CRRT involves renal replacement therapy, meaning an adjuvant therapy aimed firstly at facilitating continuous fluid removal in diuretic-resistant or acute renal failure patients. Therefore, CRRT systems inherently require a continuous net fluid removal from the patient. In other words, a CRRT system requires a fluid balance control system, such as a weight loss control system, configured to generate a continuous net weight loss rate (as opposed to merely controlling parameters to enable achieving a desired target weight loss as typically found in chronic patient care). Furthermore, acute patients experience extravascular fluid overload, which cannot be safely removed within a short period of time (e.g. within a few hours of chronic treatment) without causing potentially severe consequences (e.g. hypovolemic shock, arrhythmia, hypoxemia, hypoventilation, etc.). Therefore, a CRRT system must inherently include a much more accurate control over system parameters, in particular flow rates, in order to ensure that the required low flow rates of both blood circulating extra-corporeally and of treatment fluid (infused in the extracorporeal circuit or diffused through the dialyzer) are used. Moreover, CRRT treatment is performed continuously (e.g. for days or even weeks, without interruption/with minimal interruptions e.g., downtimes to change bags). Therefore, treatment settings in CRRT are based on flow rate settings, rather than settings pertaining to some specified treatment time (which would be unknown as acute patients may require treatment for an unknown time). Consequently, operation of CRRT systems cannot be based on some pre-defined absolute weight loss to be achieved, but rather on a meticulously controlled fluid balance in the patient, requiring continuous adjustments to a number of operating parameters, which have to be controlled and maintained during the entire (and a priori unknown) treatment time, based on a set weight-loss rate. Additionally, CRRT renal replacement therapy involves therapy substituting kidney functions for a relatively long time period and, thus, a CRRT system further requires at least either fresh dialysis liquid exchange in the dialyzer (in order to remove unwanted substances from blood and to add desired substances to the blood by diffusion) and/or fresh infusion fluid in combination with ultrafiltration (in order to remove unwanted substances from blood and to add desired substances to the blood by convection).

At least for the reasons set forth above, CRRT systems need to exhibit specific technical features enabling the system to:

- Allow setting of a weight loss rate,
- Continuously remove excess water in accordance with a set weight loss rate,
- Operate continuously at comparably low flow rates compatible with CRRT, and
- Balance ion equilibrium by means of proper dialysis being performed and/or by means of substitution fluid continuously being delivered at controlled flow rates.

Finally, in order to ready a CRRT apparatus as soon as possible given the acute situation of a patient requiring a treatment and given the absence of advance notice of the emergency situation requiring treatment, the CRRT machine is dressed using an integrated disposable set, wherein all the lines and the filtration unit are grouped together and already properly connected in the disposable set. Further, all the fluids are contained in pre-packaged bags (dialysis fluid or replacement fluids in bags of e.g., 2, 5 or 10 litres each) or pre-packaged syringes (heparin and/or concentrated calcium replacement solution).

The apparatus 1 of figure 1 has an extracorporeal blood circuit 17, which takes blood from a patient P, for instance by means of a needle or a catheter or an implanted port or other access device (not shown), introduced into a vein or artery of said patient, and through the blood withdrawal line 6 takes said blood, for instance continuously, to the filtration unit 2. The filtration unit 2 having a primary chamber 3 and a secondary chamber 4 separated by a semipermeable membrane 5; depending upon the treatment the membrane of the filtration unit may be selected to have different properties and performances.

The blood passes through the primary chamber 3 of the filtration unit 2 and, through the blood return line 7, the treated blood is carried back to the patient. In the example of figure 1, the connection with an anticoagulant line 51 is provided immediately downstream from the blood collecting zone on the blood withdrawal line 6. In particular, the machine is equipped with source of regional anticoagulant 10, such as at least a secondary fluid container or bag for supplying the anticoagulant line 51; by using corresponding actuator/s for conveying fluid, in the example shown comprising an anticoagulant pump 54, for instance a peristaltic pump, it is possible to control the fluid flow within said line by introducing the regional anticoagulant directly into the blood with a direct connection to the blood withdrawal line 6. After defining a direction of fluid (blood) circulation 200 (during normal use of the apparatus) from the blood withdrawal line 6 towards the filtration unit 2 and from the latter through the blood return line 7 towards the patient P, a known blood pressure sensor 48, which shall not be described in further detail, is placed immediately downstream the anticoagulant line 51. The blood circuit 17 comprises actuator/s for conveying fluid, i.e. in this particular case at least a blood pump 21 for controlling and managing the suitable blood flow $Q_b$ in the circuit. Also the blood pump 21 is generally a peristaltic pump acting either on the blood withdrawal line (as shown e.g. in figure 1) or on the blood return line. An operator may enter a set value for the blood flow rate $Q_b$ through a user interface 15 and the control unit 12, during treatment, is configured to control the blood pump based on the set blood flow rate. Note that, alternatively, the blood pump 21 may be automatically controlled with no need of user input/prescription: in that case control unit 12 may control the blood pump 21 at a prefixed flow rate or at a flow rate calculated based on other parameters such as, for instance, other flow rates and constraints set by the medical operator (as apparent from the following description). In addition, or alternatively, the blood pump may also be controlled based on pressure; if the blood pump 21 is controlled based on the pressure signal detected upstream the blood pump then a pressure sensor 48 is present in the tract of bloodline upstream the blood pump 21: for instance the control unit 12 may be designed to drive the blood pump in a manner to keep the pressure detected by pressure sensor 48 within a prefixed range, or below a prefixed threshold.

Following the direction of blood circulation, a gas exchanger 46 for removing $CO_2$ from circulating blood may be connected to the blood circuit. The gas exchanger 46 is in fluid communication with the blood circuit 17 to receive extracorporeal blood, allow $CO_2$ removal from blood and returning blood to the blood circuit at a downstream point. Figure 1 shows a gas exchanger 46 placed upstream the filtration unit 2; however, the gas exchanger may be alternatively positioned downstream the filtration unit along blood circulation direction. As mentioned, blood circulation direction during normal use of the apparatus is indicated in figure 1 with an arrow 200 which also represent the blood flow rate $Q_b$ direction during treatment. The gas exchanger 46 is connected in series with the filtration unit 2 and is placed downstream the injection point 50 where the regional anticoagulation solution is delivered to extracorporeal blood. The gas exchanger 46 has a blood chamber and a gas chamber separated by a membrane permeable to gases, in particular $CO_2$; the gas exchanger comprise a gas inlet, which can be connected to a gas source, such as the medical gas supply system in a hospital to receive pressurized air or oxygen for example, and a gas outlet in fluid communication with the gas chamber to discharge exhausted gas having removed $CO_2$ from extracorporeal blood. The blood inlet and the blood outlet put the extracorporeal blood circuit 17 in fluid communication with the gas exchanger blood chamber.

Then, the blood passes through another pressure sensor 49 controlling the correct flow within the blood circuit. After passing through the primary chamber 3 of the filtration unit 2, where the suitable exchanges of substances, molecules and fluids occur by means of a semipermeable membrane, the treated blood enters the blood return line 7, first passing through the air separator 19, commonly known as "bubble trap", designed so as to ensure the detection and removal of air bubbles present in the blood. The treated blood getting out of the air separator 19, before being returned to the patient P passes through an air bubble sensor 55 verifying the absence of said dangerous formations within the treated blood that has to be re-introduced in the patient's blood circulation. Immediately downstream from the bubble sensor 55, the safety valve 20 (or venous clamp) is placed which, in case of alarm, can block the blood flow towards the patient. In particular, should the bubble sensor 55 detect the presence of anomalies in the blood flow, the machine through safety valve 20 would be able to block immediately the passage of blood so as to avoid any consequence to the patient. A corresponding safety valve 27 (or arterial clamp) is present on the blood withdrawal line close the patient vascular access to fully isolate the patient from the extracorporeal blood circuit in case of need. Downstream from the safety valve 20, the treated blood is then carried back to the patient P undergoing therapy. The extracorporeal blood treatment apparatus of figure 1 is equipped with a dialysis fluid circuit 32, which is also provided with at least a dialysis supply line 8 leading into the filtration unit 2 and with an effluent (or dialysate) line 13 from the filtration unit. At least a primary fluid container, defining said dialysis liquid source 14, is designed to supply the supply line 8 of the dialysis fluid circuit 32 (generally the primary fluid container shall consist of one or more bags containing a suitable dialysis liquid). The supply line 8 includes actuator/s for conveying fluid such as at least a dialysis fluid pump 25 (in the embodiment of figure 1 a peristaltic pump) for controlling the flow rate $Q_{dial}$ of dialysis liquid from the bag and for defining a direction 200 of dialysis fluid circulation. Downstream from the dialysis fluid pump 25 in the direction of circulation 200 there is a branching 56 splitting the dialysis supply line 8 up into an intake branch 57 and an infusion branch 58. In particular, the infusion branch 58 is connected to the blood return line 7 of the blood circuit 17. In other words, by means of said infusion branch 58 it is possible to obtain a post-infusion directly in the blood line 17 using the content of the primary fluid container. Conversely, the intake branch 57 conveys the fluid directly to the filtration unit 2 and in particular to the secondary chamber of said unit. The dialysis fluid circuit 32 is further equipped with a selector 59 for determining the percentages of fluid flow within the infusion branch 58 and the intake branch 57. Generally said selector 59, usually placed near the branching 56, can be positioned at least between a first operating condition in which it allows the passage of fluid in the intake branch 57 and blocks the passage in the infusion branch 58, and a second operating condition in which it allows the passage of fluid in the infusion branch 58 and blocks the passage in the intake branch 57. In other words, said selector 59 may consist of a valve element operating on the dialysis fluid circuit 32 by alternatively blocking the passage of fluid in either branch. Suitable selectors may be alternatively provided, which are able to establish a priori the amount of liquid that has to pass through both branches simultaneously. It will also be possible to vary the percentages of fluid in either branch as a function of time and of the pre-established therapies. The dialysis liquid through the intake branch 57 gets into the secondary chamber 4 of the filtration unit 2. In particular, the primary chamber 3 through which the blood flow passes is separated from the secondary chamber 4 through which the dialysis liquid passes by means of the semipermeable membrane 5 ensuring the suitable passage of the dangerous substances/molecules and of fluid from the blood towards the dialysis liquid mainly by means of convection and diffusion processes, and also ensuring through the same principles the passage of substances/molecules from the dialysis liquid towards the blood. The dialysis fluid then gets into the effluent line 13 and passes through a suitable effluent pressure sensor 60. An actuator is provided for conveying fluid, for instance a dialysate pump 26 controlling the flow rate $Q_{eff}$ in the effluent line 13 within the fluid circuit 32. Also said pump will generally be a peristaltic pump. The fluid to be eliminated then passes through a blood detector 61 and is conveyed into a collection container or bag 62. The hydraulic circuit of the apparatus according to figure 1 includes at least another infusion line 63 for feeding fluid into the blood return line 7 of the blood circuit 17. In particular, the infusion fluid is taken from at least an auxiliary container 64 and is sent directly to the blood return line 7 of the blood circuit 17 through actuator/s for conveying fluid, generally an infusion pump 65 (in the example a peristaltic pump) controlling its flow rate $Q_{rep}$ - total replacement flow rate. In particular, the infusion liquid can be introduced directly into the air separator 19. As can also be inferred, the infusion branch 58 of the dialysis fluid circuit 32 and the infusion line 63 are equipped with a common end length 66 letting fluid to enter into the blood circuit 17. Said intake end length 66 is placed downstream from the infusion pump 65 with respect to a direction of infusion and carries the fluid directly into the air separator 19. Further, referring to the diagram in figure 1, the infusion line 63 comprises at least a pre-infusion branch 67 connected to the blood withdrawal line 6 of the blood circuit 17. In further detail, downstream from the infusion pump 65 with respect to the direction of infusion, there is an infusion branching 68 splitting the infusion line 63 up into the pre-infusion branch 67 and post-infusion branch 69. The pre-infusion branch 67, in particular, carries the fluid taken from the bag 64 into the blood withdrawal line 6 of the blood circuit 17 downstream from the blood pump 21 and downstream the gas exchanger 46 with respect to the direction of blood circulation. Conversely, the post-infusion branch 69 is connected directly to the common end length 66. The infusion line 63 further comprises a selector 70 for determining the percentage of liquid flow to be sent to the post-infusion branch 69 and to the pre-infusion branch 67. The selector 70 placed near the branching 68 may be switched between at least a first operating condition in which it allows the passage of fluid in the pre-infusion branch 67 and blocks the passage in the post-infusion branch 69, and at least a second operating condition in which it allows the passage of fluid in the post-infusion branch 69 and blocks the passage in the pre-infusion branch 67. Obviously, as in the case of the selector 59 present on the dialysis fluid circuit 32, also the other selector 70 will be able to determine the percentage of fluid that has to pass in each of the two branches and to possibly vary it in time in accordance with the planned therapies. Moreover, the selector 59 and the other selector 70 will generally, though not necessarily, be of the same nature. Notably, the flow rate through the pre-infusion branch/line 67 may be determined by proper control of the infusion pump 65 and other selector 70; the control unit 12 may receive a pre-infusion ratio of replacement fluid flow PRE (a value between 0 and 1) and determine the pre-infusion flow rate $Q_{rep.pre}$ based on the pre-infusion ratio PRE and on total replacement flow rate $Q_{rep}$. In particular, $Q_{rep.pre}=PRE \cdot Q_{rep}$. Alternatively, post-infusion ratio may be used symmetrically, or a ratio between pre- and pos- infusion rate may be used as well ($R3=Q_{re.pre}/Q_{rep.post}$).

The apparatus is equipped with means 71 for determining at least the weight of the primary fluid container 14 and/or of the auxiliary fluid container 64 and/or of the regional anticoagulant container 10 and/or of the collection container 62. In particular, said means 71 comprises weight sensors, for instance respective scales A, B, C, D and E (for example at least an independent sensor for each fluid bag associated to the machine). In particular, there will be at least four of said scales, each pair being independent from the other, and each one measuring the respective weight of a bag. It should then be pointed out that there is a control unit or CPU 12 active (at least) on the blood circuit 17 and in particular active on the pressure sensor 48 for reading pressure values, on the blood pump 21, on the gas exchanger 46, on the other pressure sensor 49, and on the device for detecting the presence of air bubbles 55 and on the respective safety valves 20, 27. The control unit 12 has also to control the dialysis fluid circuit 32 and, in particular, shall be input with the data detected by the scales A, B, C, D and (possibly) E and, concerning the weight of the bag 14, and shall act on the pump 25, on the selector 59, on the pressure sensor 60, then on the dialysate pump 26 and shall eventually receive the data detected by the scale A whose function is to determine the weight of the collection container 62. The control unit 12 shall also act on the infusion line 63 checking the weight of the auxiliary container 64 (checked by the scale C) and will be able to control both the infusion pump 65 and the other selector 70. The control unit 12 shall also act on the anticoagulant line 51 detecting the weight of the anticoagulant fluid container 10 by means of the scale B and suitably controlling the anticoagulant pump 54 according to the treatments to be carried out as below detailed and explained. As apparent, a regional anticoagulation system is implemented in the apparatus 1 of figure 1 to provide anticoagulation restricted to the extracorporeal blood circuit 17. The regional anticoagulation system is described in detail in the respective description paragraph. However, the apparatus of figure 1 may be alternatively (or additionally) provided with a systemic anticoagulation system, such as a syringe pump 9 for injecting heparin downstream the blood pump 21. Indeed, the algorithm of embodiments the invention as described in the subsequent detailed description works both in CRRT treatment configurations with RCA and in CRRT treatment configurations with systemic (or no) anticoagulation without RCA.

The control unit 12 is also connected to a memory 16 and to user interface 15, for instance a graphic user interface, which receives operator's inputs and displays the apparatus outputs. For instance, the graphic user interface 15 may include a touch screen, a display screen and/or hard keys for entering user's inputs or a combination thereof.

**The regional anticoagulation system**

A regional anticoagulation system comprises a source of regional anticoagulant 10, e.g., a container or a bag containing at least a substance having an anticoagulant effect. For example, citrate, in the form of pure sodium citrate ($Na_3$citrate) or mixture of sodium citrate and citric acid are used for blood anticoagulation purpose.

Alternatively pure citric acid may be used as anticoagulant. Indeed, citrate has a high affinity for calcium in creating complexes and several steps of the coagulation cascade are dependent on blood (ionized) calcium. A proper decrease of ionized calcium concentration in the presence of citrate inactivates the coagulation cascade.

Normal plasma includes about 1.1 to 1.3 mmol/l of ionized calcium, 0.1-0.2 mmol/l of complexed calcium and 0.9 to 1.2 mmol/l of protein-bound calcium. In order to achieve proper anticoagulation effects, general guidelines are to adjust citrate amount/dose as to reach an ionized calcium concentration of 0.20 to 0.35 mmol/l in the extracorporeal blood circuit after citrate infusion. Plasma with citrate addition for anticoagulation purposes would include (as an average) about 0.3 mmol/l of ionized calcium, 1.8 mmol/l of complexed calcium (mainly $Ca_3$citrate$_2$) and 0.2 mmol/l of protein-bound calcium. During RCA, intensity of anticoagulation can be adjusted via the amount of infused citrate. Post-filtration unit ionized calcium concentration is commonly used as key parameter (target in the 0.20-0.35 mmol/l range) and is measured e.g., with blood gas analyzer.

The regional anticoagulation system is arranged to deliver the regional anticoagulant at a delivery point 50 in the extracorporeal blood circuit 17. Citrate infusion is preferably administered close to an access end of the blood withdrawal line 6 to get full anticoagulation of the extracorporeal blood circuit 17. In general the delivery point 50 is located upstream the blood pump 21; however, it is not excluded that the delivery point 50 is located in the blood withdrawal line 6 downstream the blood pump. Alternatively, or in combination, the delivery point 50 for citrate may be the inlet of the filtration unit 2. In this latter configuration, the dialysis fluid contains citrate in an amount sufficient to achieve ionized calcium level around 0.25-0.35 mmol/l in blood circuit downstream the dialyzer. Citrate may be added to the treatment fluid flowing along the supply line 8 using a corresponding concentrate bag/container in case the dialysis fluid is on-line prepared as in current apparatuses for chronic treatment. Alternatively, particularly in case of CRRT apparatuses, the source 14 for dialysis fluid is a container/bag including the proper citrate concentration or content.

Commercial citrate solutions are generally packed in respective plastic bags (sources 10) and can be split between physiologic and concentrated solutions. Physiologic citrate solutions are solutions having sodium concentration about 140 mmol/l, such as Baxter PrismoCitrate 10/2 (with 10 mmol/l Nacitrate and 2 mmol/l citric acid) and Baxter RegioCit 18/0 (with 18 mmol/l Nacitrate). Concentrated citrate solutions are for example, ACD-A (Anticoagulant Citrate Dextrose Solution) from Biomet: mix of sodium citrate (75 mmol/l), citric acid (38 mmol/l) and glucose; and Citrate 4% from Fresenius: citrate 136 mmol/l.

When citrate is infused into the blood withdrawal line 6 close to the patient vascular access, blood pump speed is automatically adjusted as to take the operator set blood flow rate from access site (blood pump speed=k*($Q_b$+$Q_{cit}$), wherein $Q_b$ is the set (or calculated) blood flow rate - desired at the access site and $Q_{cit}$ is the citrate infusion flow rate).

Citrate amount is prescribed through the 'Citrate Dose' parameter ($D_{citrate}$) which is the amount of citrate per liter of blood treated (mmol/l blood). Notably, citrate dose does not match with citrate concentration in the diluted blood reaching the filtration unit. The concept is rather to provide for an amount of citrate in proportion to the amount of calcium to be chelated. The set of the citrate pump 54 is:

$$Q_{cit} = \frac{D_{cit}}{C_{cit\_PBP}} \cdot Q_b$$

(Eq. 1)

wherein

$Q_{cit}$ is the citrate infusion flow rate;
$Q_b$ is the set blood flow rate;
$D_{cit}$ is the citrate dose; and
$C_{cit\_pbp}$ is the citrate concentration in the anticoagulant source. This is the citrate flow rate mathematical relation which is stored in the memory 16 of the control unit 12 and used to determine the citrate infusion flow rate $Q_{cit}$ given the citrate dose $D_{cit}$ and citrate concentration in the anticoagulant source 10 as input. Blood flow can be either an input value (prescription parameter) or a calculated value (operating parameter) that the control unit determines based on the system configuration and prescription parameters (see the following detailed description).

Citrate infusion is delivered with a dosage aimed to maintain ionized calcium level around 0.25-0.35 mmol/l in blood circuit downstream the dialyzer. Typically, citrate dose is included in the range 1.5 to 6.0 mmol/l-of-blood. The most common range is 2 to 4 mmol/L-of-blood. Citrate dose guideline of 3.0 mmol/L-of-blood is globally followed. Ionized calcium and citrate complexes are rather small molecules which are easily transferred through the filtration unit 2. Loss rates are basically dependent on flow rates, filter efficiency with respect to small molecules and solute concentrations. While about half of the total calcium is not available to mass transfer during standard anticoagulation (since it is protein-bound), about 90% of total calcium becomes available during citrate anticoagulation. Therefore, citrate regional anticoagulation combined with the use of calcium free dialysis and/or replacement fluids implies significant calcium losses to dialysate. In extracorporeal blood treatments with RCA, calcium infusion is required to balance calcium losses to dialysate. During RCA, calcium infusion is adjusted to keep patient systemic ionized calcium in the normal range (e.g., 1.0 - 1.2 mmol/l).

Therefore, the regional anticoagulation system of the apparatus 1 includes a source of ion balancing solution 11, which is reinfused in the blood, either in the return line 7, in particular close to the venous vascular access, or directly into the patient P (infusion into central catheter, which is recommended). The ion balancing solution 11 is contained in a vessel e.g., a syringe, a container or a bag; the regional anticoagulation system of the apparatus 1 comprises a ion replacement infusion line 74 and a corresponding ion replacement pump 75 to drive delivery of a proper ion replacement infusion rate $Q_{ca}$. Figure 1 shows a line 74 directly infuse in the blood return line 7, possibly close to the venous access. Of course, line 74 may alternatively directly infusing into the patient P. In the example of figure 1, the syringe pump 9 usually used to deliver heparin may be alternatively used to deliver the ion balancing solution either directly into the patient or alternatively in the blood return line. The ion balancing solution includes ionized (concentrated) calcium and its infusion is performed to restore patient systemic ionized calcium at normal level. Notably, ion balancing solution may include also ionized magnesium and its infusion is performed to restore patient systemic ionized magnesium at normal level since also magnesium removal in dialysate is increased during RCA. The ion replacement infusion rate $Q_{ca}$ may be adjusted based on the revealed patient ionized calcium concentration in blood or an automatic control may be implemented, such as the one described in patent publication US8668825B2 (which is here incorporated by reference).

In an implementation, the ion balancing solution flow rate is kept proportional to the estimated calcium loss rate in dialysate. For example it is computed by the apparatus control unit through the equation (calcium flow rate mathematical relation):

$$Q_{ca} = \frac{CaComp \cdot J_{Ca}}{C_{ca}} - \frac{Q_{rep.post} \cdot C_{ca_{rep.post}}}{C_{ca}} - \frac{Q_{HCO3} \cdot C_{ca_{HCO3}}}{C_{ca}}$$

(Eq. 2)

Where CaComp is a calcium compensation parameter, $Q_{ca}$ is the ion balancing solution flow rate (ml/h), $J_{ca}$ is the estimated calcium loss rate in the dialysate (mmol/h), $C_{ca}$ is calcium concentration of the ion balancing solution (mmol/l), $Q_{rep.post}$ is the post-dilution replacement flow rate (ml/h), $C_{ca\_rep.post}$ is calcium concentration of the replacement solution in post-dilution (mmol/l), $Q_{HCO3}$ is the fluid flow rate (ml/h) through the post-dilution bicarbonate infusion line 23, and $C_{ca\_HCO3}$ is the calcium concentration in the bicarbonate container. Calcium compensation is the user-controllable setting, which might be set by the operator generally in a range between 5% and 200%. Notably the above equation takes into account a post replacement solution including calcium, as well as bicarbonate infusion also including calcium. In case no calcium is in the post replacement solution (or no replacement solution is used) the second term of the equation should be disregarded (equal to zero). In case no calcium is in the bicarbonate replacement solution (or no bicarbonate solution is used) the third term of the equation should be disregarded (equal to zero).

The calcium flow rate mathematical relation is also used by the control unit 12 to determine the ion re-establishing flow rate $Q_{ca}$ based on known (input) calcium concentration in the various post infused solutions. Further, it is assumed that no calcium is infused upstream the filtration unit 2 to avoid undesired coagulation effects (in this respect the anticoagulant solution does not contain any calcium and the pre-dilution infusion line 67 is absent or the replacement solution pre-infused is calcium free). Further, the ion compensating parameter is part of the prescription as well. The post infusion flow rates, namely $Q_{rep.post}$ and $Q_{HCO3}$ are operating parameter and the control unit 12 determines them based on the prescription parameters and apparatus configuration. Also $J_{ca}$ is estimated, for example as disclosed in the mentioned US8668825B2 application.

Alternatively, the medical staff may provide a 'calcium dose' $D_{ca}$ in terms of calcium concentration in the effluent (mmol/l). The ion balancing solution flow rate $Q_{ca}$ is calculated based on the set calcium dose taking into account calcium concentration of the ion balancing solution, calcium concentration of the replacement solution and estimated calcium concentration in blood at the outlet of the filtration unit 2.

Indeed, as to dialysis fluid (and replacement solution), they are generally calcium free to prevent transferring ionized calcium to blood. Moreover, the dialysis and/or replacement fluids may have adapted buffer content due to citrate metabolism and adapted sodium if concentrated citrate solution (hypertonic) is used.

As to the buffer agent, since RCA has complex impact on acid-base balance equilibrium due to a significant fraction of citrate returned to patient (citrate is metabolized into bicarbonate), when assisting prescription, the control unit 12 would require input of a steady state acid-base balance target to be achieved. Indeed, blood returned to the patient contains a significant concentration of citrate-calcium complexes. These complexes are (quickly) metabolized in liver, skeletal muscles, kidney releasing calcium in the blood stream, thus preventing systemic anticoagulation to develop; the citrate metabolism produces bicarbonate (3 moles $HCO_3^-$ for 1 mole citrate).

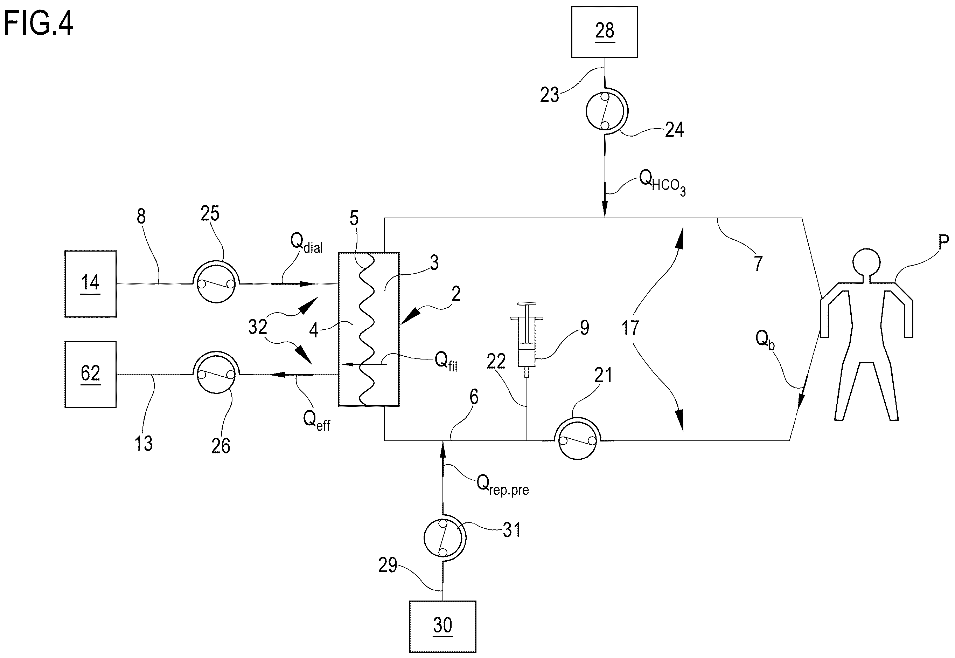

In this respect, the dialysis fluid may contain no buffer agent, e.g., no bicarbonate. A buffer agent from a source/container/bag 64 may be infused into the blood return line 7 via a suitable buffer agent supply line 63, 69, 66 and the corresponding buffer agent pump 65. Alternatively or in combination, to allow for acid-balance additional control, the apparatus 1 might also be designed to vary buffer balance of the extracorporeal blood circuit in an easy and controlled way via the possibility to set dialysis fluid (low) buffer concentration and/or to use source bags 14, 64 with different buffer concentration, e.g., in the range 15 to 25 mmol/l (and up to 40 mmol/l and/or down to 0 mmol/l) for bicarbonate. As described, a specifically designed bicarbonate solution may be post-infused to allow for acid-base balance additional control (see e.g., figures 4 to 6).

As mentioned, citrate accumulation in the patient may correlate with hypocalcemia, metabolic acidosis (low bicarbonate production due to poor metabolism) or metabolic alkalosis (excessive bicarbonate production subsequent to high citrate load). As citrate measurement is not commonly available at the hospital, ratio of total calcium to ionized calcium is used as indicator, namely values below 2.5 are considered as normal (normal value below 2.0) and values above 2.5 indicate low ionized calcium concentration with respect to total calcium, possibly due to the presence of a significant systemic concentration of citrate. However, this monitoring is considered an insufficient measure, particularly in treatment involving relevant risks of acid/base unbalance, such as in RCA with 'large' flow rates, such as certain SCUF treatments.

### Specific embodiments

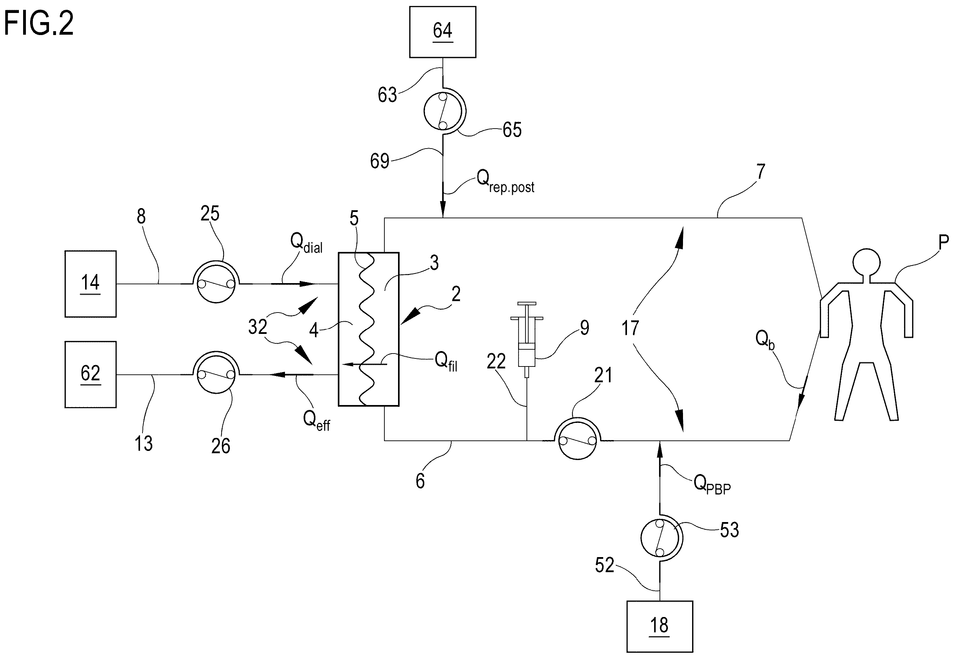

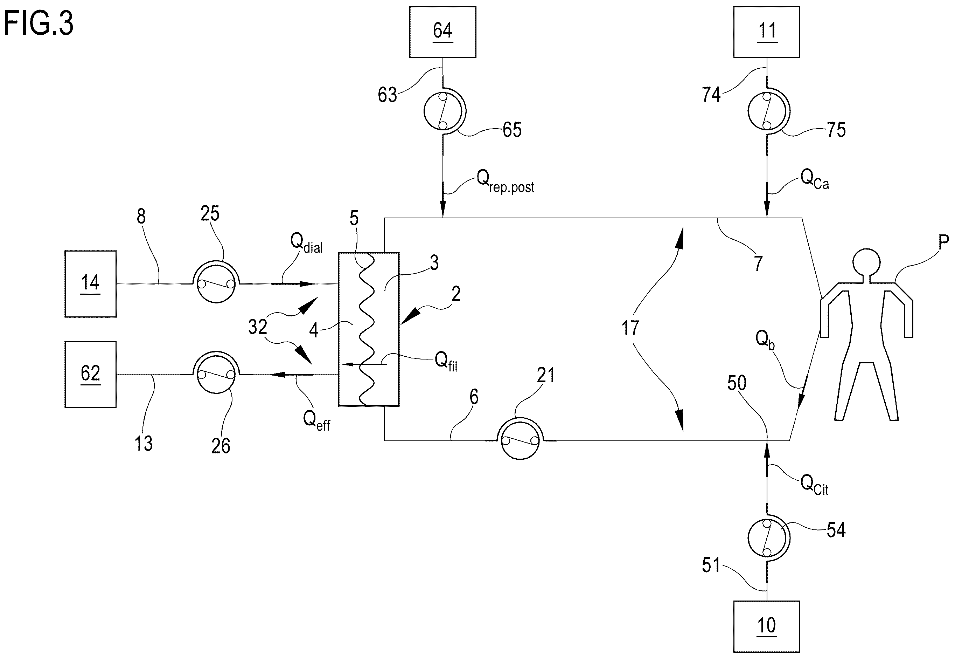

The embodiment of figures 2 and 3 show different configurations of the apparatus 1 shown in figure 1, where the same components described for the embodiment of figure 1 are also present and are identified by same reference numerals and thus not described again in detail.

The embodiment of figure 2 refers to a CRRT configuration with no or systemic anticoagulation. In particular, in case systemic anticoagulation is used, a heparin container (e.g., a syringe 9) is used to inject boluses of anticoagulant into the withdrawal line 6 via a heparin line 22 usually infusing downstream the blood pump 21. The embodiment includes a pre-blood-pump (PBP) line 52 for infusing a replacement solution (no citrate) contained in a PBP container 18 with a PBP pump 53 generating a pre-blood-pump infusion flow rate $Q_{PBP}$. Further, a post-infusion line is present for post-infusing a replacement solution contained in auxiliary container 64. A dialysis liquid source 14 is also included to provide a dialysis fluid flow rate $Q_{dial}$ to the second chamber 4 of the filtration unit 2; an effluent fluid flow rate $Q_{eff}$ is directed to the collection container 62 by means of the dialysate pump 26. If we compare the embodiment of figures 1 and 2, we note that the embodiment of figure 2 lacks any ion balancing infusion line 74 (no regional anticoagulation is provided), that the fluid from liquid source 14 is directed to the dialyzer (selector 59 is configured to prevent any fluid to route to post-infusion) and that the replacement liquid from auxiliary container 64 is exclusively routed in post dilution (additional selector 70 is configured to prevent any fluid to route to pre-infusion).

Embodiment of figure 3 differs from the embodiment of figure 2 since it refers to a CRRT configuration with regional anticoagulation. No heparin syringe 9 is used. The container 10 is a source of regional anticoagulant (citrate) and the anticoagulant pump 54 generates a citrate infusion flow rate $Q_{cit}$. A corresponding ion balancing infusion line 74 and ion balancing pump 75 are included to infuse a calcium concentrated solution from the source of ion balancing solution 11 at a calcium infusion flow rate $Q_{ca}$.

The embodiment of figure 4 includes the same dialysis fluid circuit of embodiments 2 and 3. As already shown in embodiment 2, the embodiment of figure 4 refers to a CRRT configuration with no or systemic anticoagulation. Differently, a pre-infusion line, downstream the blood pump 21, for infusing a replacement solution in pre-dilution is included. The pre-infusion line may be the infusion line 63 of figure 1, wherein the other selector 70 is configured to direct all the replacement fluid coming from auxiliary container 63 in pre-dilution through pre-infusion branch 67. Alternatively, a pre-dilution infusion line 29 delivering the replacement fluid contained in replacement source 30 is provided. The infusion pump 31 generates a pre-infusion replacement flow rate $Q_{rep.pre}$. Additionally, also a post-dilution bicarbonate infusion line 23 for concentrate bicarbonate solution is included. A bicarbonate pump 24 injects a bicarbonate concentrate solution from a bicarbonate container 28 at a bicarbonate concentrate post-infusion flow rate $Q_{HCO3}$.

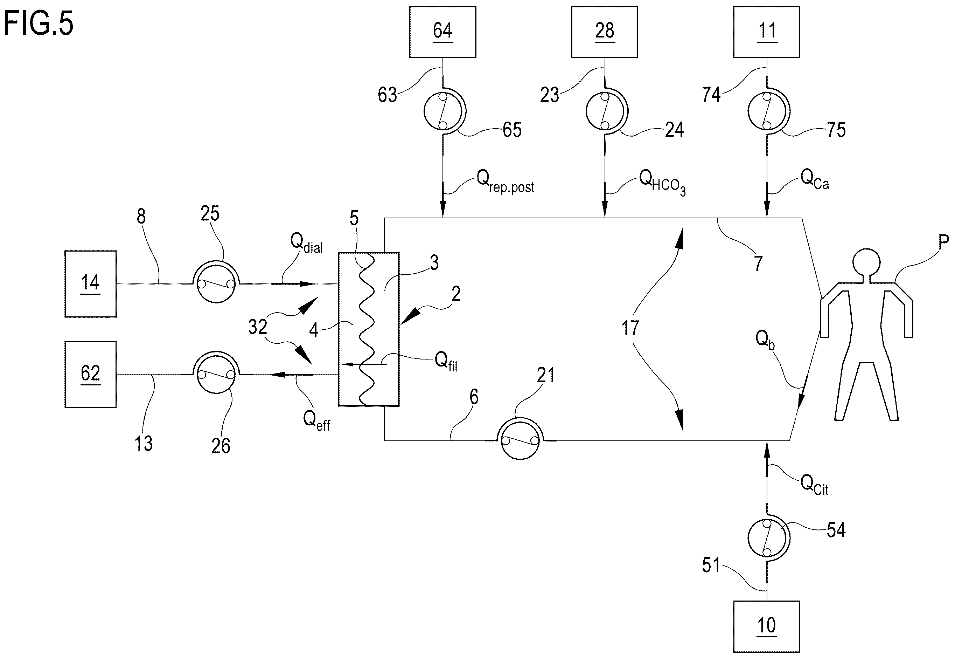

The embodiment of figure 5 refers to a CRRT configuration with regional anticoagulation. No heparin syringe 9 is used. The container 10 is a source of regional anticoagulant (citrate) and the anticoagulant pump 54 generates a citrate infusion flow rate $Q_{cit}$. A corresponding ion balancing infusion line 74 and ion balancing pump 75 are included to infuse a calcium concentrated solution from the source of ion balancing solution 11 at a calcium infusion flow rate $Q_{ca}$. Further, a post-infusion line 63 is present for post-infusing a replacement solution contained in auxiliary container 64. Additionally, also a post-infusion line 23 for concentrate bicarbonate solution is included. A bicarbonate pump 24 injects a bicarbonate concentrate solution from a bicarbonate container 28 at a bicarbonate concentrate post-infusion flow rate $Q_{HCO3}$.

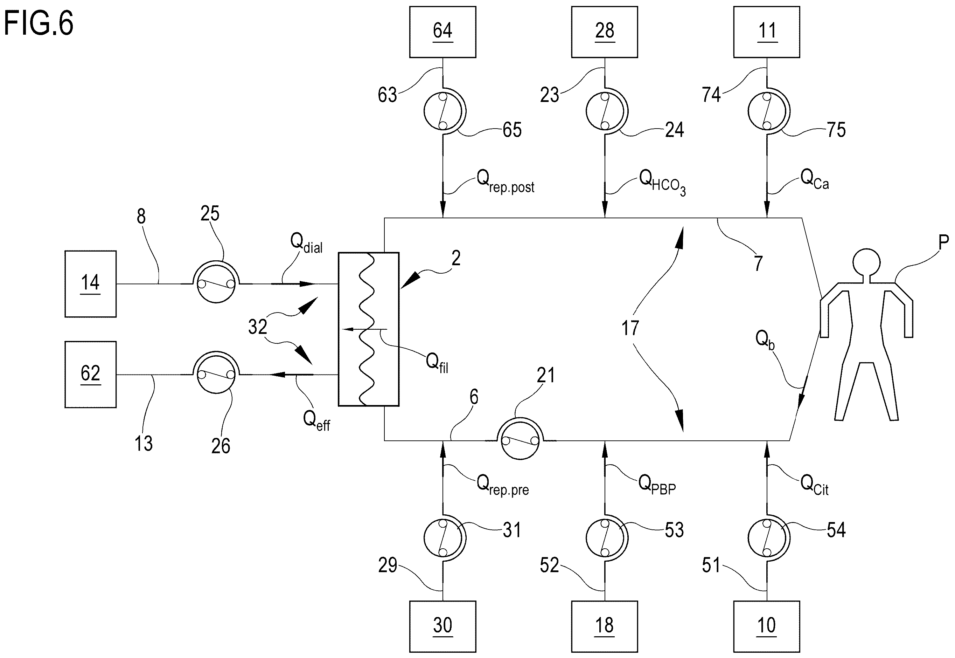

Figure 6 is another embodiment, wherein the apparatus includes all possible infusion lines, namely two separate pre-blood-pump infusion lines: one anticoagulant line 51 for citrate infusion (flow rate: $Q_{cit}$), and one PBP infusion line 52 for pre-bood-pump replacement fluid infusion (flow rate: $Q_{PBP}$), one pre-dilution infusion line 29 infusing replacement fluid from a replacement source 30 (e.g., a pre-packaged container) by means of a respective pre-infusion pump 31 (flow rate: $Q_{rep.pre}$), one post-dilution infusion line 63 infusing from an auxiliary container 64 including a replacement solution (flow rate: $Q_{rep.post}$), one post-dilution bicarbonate infusion line 23 (flow rate: $Q_{HCO3}$), one ion balancing infusion line 74 for infusing a calcium concentrate solution (flow rate: $Q_{ca}$), one dialysis supply line 8 for directing fresh dialysis fluid to the filtration unit (flow rate: $Q_{dial}$) and one effluent line 13 for sending spent dialysate to a collection container 62 (flow rate: $Q_{eff}$). In the embodiment of figure 6 the pre-infusion post-blood-pump line 29 and the post-infusion line 63 are shown as separate and independent lines; alternatively, the two lines 29, 63 may be merged together as in the embodiment of figure 1, wherein one single bag 64 provides the same replacement fluid to two branches one directing the fluid in pre-dilution, the other in post-dilution. The respective infusion fluid flow rates $Q_{rep.post}$ and $Q_{rep.pre}$ are obtained by properly driving the infusion pump 65 and the selector 70: the flow from the single pump 65 is alternatively directed either to pre-infusion or to post-infusion via selector 70 over time; the control of the time spent in each of the two states allows to control the split of the flows between pre and post infusion. The desired flow values are obtained as average values over a certain period of time, which must naturally be long enough, i.e. at least several minutes.

Further, the two infusion lines 51 and 52 infusing fluids pre-blood-pump, may be substituted by a single line, used for citrate in case of regional anticoagulation, or for PBP replacement fluid in case of no or system anticoagulation. Finally, it is noted that in case any line is missing in a specific circuit configuration, the corresponding flow rate may be omitted in any of the following formula/mathematical relations (or its value set to zero).

### Equations for flow rates

The following equations for flow rates express the relations between the flow rates which are used in the detailed description. These equations are stored in the memory 16 of the apparatus. When necessary, the control unit 12 makes use of the following equations to determine the operative parameters (as explained in the following sections). The plasma flow rate is function of blood flow rate as follows:

$$Qp = Q_b \cdot (1 - Hct)$$

(Eq. 3)

The plasma water flow rate is function of blood flow rate as follows:

$$Qpw = Qp \cdot F_p = Q_b \cdot (1 - Hct) \cdot F_p$$

(Eq. 4)

The plasma water flow rate at the filter inlet is:

$$Qpw_{inlet} = Qpw + Q_{cit} + Q_{syr} + Q_{PBP} + Q_{rep.pre} = Q_b \cdot (1 - Hct) \cdot Fp + Q_{cit} + Q_{PBP} + Q_{rep.pre}$$

(Eq. 5)

The blood water flow rate is function of blood flow rate as follows:

$$Qbw = Q_b \cdot [(1 - Hct) \cdot F_p + Hct \cdot Frbc]$$

(Eq. 6)

The blood water flow rate at the filter inlet is:

$$Qbw_{inlet} = Q_{bw} + Q_{cit} + Q_{syr} + Q_{PBP} + Q_{rep.pre}$$

$$= Q_b \cdot [(1 - Hct) \cdot Fp + Hct \cdot Frbc] + Q_{cit} + Q_{PBP} + Q_{rep.pre}$$

(Eq. 7)

The ultrafiltration rate in the filtration unit is:

$$Q_{fil} = Q_{PBP} + Q_{cit} + Q_{syr} + Q_{rep.pre} + Q_{rep.post} + Q_{HCO3} + Q_{PFR} + Q_{ca}$$

(Eq. 8)

In case one single infusion line 63 (as in figure 1) is used, the pre-infusion rate of replacement fluid may be calculated based on the (total) replacement flow rate ($Q_{rep}$) delivered by infusion pump 65 times a pre-replacement fluid flow factor (PRE) is:

$$Q_{rep.pre} = PRE \cdot Q_{rep}$$

$$Q_{rep} = (Q_{rep.pre} + Q_{rep.post})$$

(Eq. 9)

The effluent flow rate is:

$$Q_{eff} = Q_{PBP} + Q_{cit} + Q_{syr} + Q_{dial} + Q_{rep.pre} + Q_{rep.post} + Q_{HCO3} + Q_{PFR} + Q_{ca} = Q_{dial} + Q_{fil}$$

(Eq. 10)

## Clinical prescription parameters

Clinical prescription parameters are the parameters that the CRRT apparatus requires from the medical staff when initiating or updating treatment prescription on a specific patient. The apparatus may either request the physician to input the prescription parameter or provide a suggested value to be confirmed or updated. Prescription parameters to be confirmed/updated may be read from patient cards or similar supports.

Purpose of the 'assisted prescription' process is to operate from prescription parameters being fully meaningful from the clinical perspective; selected prescription parameters of interest are listed in next table.

| Symbol | Parameter name | Clinical aspect |
|---|---|---|
| $D_{CRRT}$ | CRRT dose (ml/kg/h) | Defines intensity of the RRT treatment |
| $D_{cit}$ | Citrate dose (mmol/L) | Intensity of citrate anticoagulation (RCA) |
| CaComp | Calcium compensation (%) | Intensity of calcium balancing (RCA) |
| $D_{ca}$ | Calcium dose (mmol/l) | |
| $Cp_{HCO3\_pat}$ | Steady patient bicarbonate (mM) | Steady state acid-base balance target (either $Cp_{HCO3\_pat}$ or nNBL) |
| nNBL | Normalized Net Buffer Load (mmol/h/kg) | |
| $Q_{PFR}$ | Patient Fluid Removal rate (ml/h) | Patient fluid balance |
| $Q_b$ | Blood flow rate (ml/min) | Optional; see below |

Not all the above mentioned clinical prescription parameters are necessary/required, but their relevance depends on selected treatment options and medical staff preferences.

For example, blood flow rate listed in the table may be used as prescription parameter (set by medical staff) or as operating parameters (set by the system algorithm) according to the customer preference as hereinafter described. Citrate dose and calcium compensation are relevant in case of regional anticoagulation treatments and not necessary in case of no or systemic anticoagulation.

Patient fluid removal rate may be entered as a prescription or, alternatively, may be calculated by the control unit. In general, CRRT dose is the 'running' dose which has to be derived from the clinical target (20 to 25 ml/kg/h according to KDIGO guidelines) corrected for system down times; this leads to a recommended running dose in the 25-30 ml/kg/h range. The CRRT dose is further explained in the following paragraph.

As previously indicated, citrate dose controls the intensity of anticoagulation; it should be adjusted as to decrease ionized calcium concentration in the extracorporeal blood circuit down to the 0.2-0.35 mM range. In parallel, calcium compensation controls the balancing level of calcium losses relatively to a computed default loss rate; alternatively to a percentage, calcium balancing may be prescribed as a concentration of calcium in effluent (calcium dose $D_{ca}$). Steady state acid-base balance target is either defined as a steady state patient bicarbonate concentration (requiring an assumption on nNBL), or as normalized Net Buffer Load (requiring an assumption on bicarbonate concentration). Second option is normally preferred. At steady state, nNBL is expected to balance the protons (H+) generation rate from patient metabolism. The steady state acid-base balance target is further discussed in the following paragraphs.

Patient fluid removal prescription directly derives from the analysis of the patient fluid balance, considering all patient's fluid inputs and outputs, as well as the desired correction of patient's fluid status. It is clear that setting the patient fluid removal rate may be preferred; however, the setting of another fluid flow rate may allow to determine patient fluid removal rate as a consequence (e.g., setting effluent flow rate and using Eq. 10 having knowledge of the infusion flow rates and dialysis flow rate).

Blood flow rate is not expected to have any strong clinical background aside some technical considerations on the patient's vascular access. If chosen as prescription parameter, the medical prescription may either provide a desired set value or simply define lower and/or upper limit(s) of the blood flow rate setting range.

**CRRT Dose Definitions**

In the present specification, CRRT dose refers primarily to a flow rate or to a combination of flow rates (expressed then in ml/h). It is however noted that an additional definition of treatment dose in CRRT is expressed in ml/kg/h, meaning the normalisation of a flow rate (or combination of flow rates) to patient body weight (BW). Particularly for infants, literature CRRT dose also refers to a treatment dose normalised to patient Body Surface Area (BSA).

Dose (aside from its 'normalisation' to patient size) can be defined as a combination of the flow rates in the most general way, with following examples:

- effluent dose ($D_{eff}$): $Q_{eff}$ as perfect typical (and simple) example of field practice,
- convective dose ($D_{conv}$): sum of all infusion flow rates to blood circuit or patient plus patient fluid removal rate,
- diffusive dose ($D_{dial}$): dialysis flow rate $Q_{dial}$,
- all above doses corrected for blood (or plasma) pre-dilution upstream the filter
- urea dose ($D_{urea}$): estimated urea clearance,
- clearance dose ($D_{sol}$): any dose computed on the basis of an estimated clearance of a given solute (=> expression function of all flow settings and of dialyzer/filter related parameters).

**Formulae for CRRT dialysis Dose**

All below expressions of Dose are not normalised to patient size (body weight BW or patient surface area PA); expressions of normalised dose (NDose) can be directly expressed as:

$$NDose = Dose/BW;$$

or NDose = Dose/BSA x 1.73 (when normalised to a 1.73 $m^2$ surface area patient - Body Surface Area; see paediatric literature)

For example, one of the following magnitudes may be used as dose:

- effluent dose ($D_{eff}$): the flow rate across the effluent line, namely $D_{eff}=Q_{eff}$;
- convective dose ($D_{conv}$): the sum of the flow rates through the infusion line or lines connected directly to the patient or connected to the blood circuit and of the patient fluid removal rate, namely

$$D_{conv}=Q_{PBP}+Q_{cit}+Q_{syr}+Q_{rep.pre}+Q_{rep.post}+Q_{PFR}+Q_{ca}+Q_{HCO3},$$

or

$$D_{conv} = Q_{fil}$$

of course, in case one or more lines are absent, the corresponding flow rate term/s may be absent from the definition or the value/s set to 0;

- diffusive dose ($D_{dial}$): the flow rate of fluid supplied to the filtration unit secondary chamber, namely $D_{dial}=Q_{dial}$.
- urea dose ($D_{urea}$): estimated urea clearance ($K_{urea}$); note that in CRRT conditions, a first approximated expression assumes that filter urea clearance is more or less identical to effluent flow rate $Q_{eff}$; in a further alternative, an estimate of urea clearance more accurate than $Q_{eff}$; especially when operating with large flow rates or small filters (paediatric conditions), may be provided by the following equations:

a) For purely diffusive mode (where there is no infusion of replacement fluid and where the patient fluid removal rate is zero or substantially zero) and counter-courant flow configuration (fluids in the chambers of the filtration unit 2 are counter-current):

$$Z = \frac{Q_{bw_{inlet}}}{Q_{dial}} \qquad NT = \frac{(\frac{S}{RT})_{urea}}{Q_{bw_{inlet}}}$$

$$D_{urea}(Q_{bw_{inlet}}; Q_{dial}) = K_{urea} = Q_{bw_{inlet}} \cdot \frac{e^{[NT\cdot(1-Z)]}-1}{e^{[NT\cdot(1-Z)]}-Z} \qquad \text{if } Z{\neq}1$$

$$D_{urea}(Q_{bw_{inlet}}; Q_{dial}) = K_{urea} = Q_{bw_{inlet}} \cdot \frac{NT}{NT+1} \qquad \text{if } Z{=}1$$

(Eq. 11)

where: S (effective filter surface area) is dependent on the hemodialyzer (i.e. the filtration unit 2) in use; RT is total mass transfer resistance dependent of the hemodialyzer in use (membrane properties, filter design) and the solute of interest, in this case urea; note that S/RT is also the mass transfer property of the filtration unit, namely KO.A (ml/min) and $Qbw_{inlet}$ is the blood water flow rate at the inlet of the filtration unit 2.

b) In case of presence of both dialysis fluid flow rate, $Q_{dial}$, and of one or more infusions of fluid, then:

$$\gamma = e^{\frac{SC_{urea}\cdot Q_{fil}}{(S/RT)_{urea}}} - 1$$

$$f_{urea} = \left(\frac{Q_{bw_{inlet}} - SC_{urea} \cdot Q_{fil}}{Q_{bw_{inlet}}} \cdot \frac{Q_{dial} + SC_{urea} \cdot Q_{fil}}{Q_{dial}}\right)^{\frac{1}{\gamma}}$$

$$D_{urea}(Q_{bw_{inlet}}; Q_{dial}; Q_{fil}) = K_{urea}$$
$$= \frac{Q_{bw_{inlet}} \cdot Q_{dial} - f_{urea} \cdot (Q_{bw_{inlet}} - SC_{urea} \cdot Q_{fil}) \cdot (Q_{dial} + SC_{urea} \cdot Q_{fil})}{Q_{dial} - f_{urea} \cdot (Q_{bw_{inlet}} - SC_{urea} \cdot Q_{fil})}$$

(Eq. 12)

where: S (effective filter surface area) is dependent on the hemodialyzer in use; RT is total mass transfer resistance dependent of the hemodialyzer in use (membrane properties, filter design) and the solute of interest, in this case urea; $Q_{fil}$ is the ultrafiltration rate in CRRT filter; and $Qbw_{inlet}$ is the blood water flow rate at the inlet of the filtration unit 2. $SC_{urea}$ is the sieving coefficient for urea (dependent of selected filtration unit).

- clearance dose: an estimated clearance for a given solute; for certain solutes a first approximated expression assumes that filter solute clearance is more or less identical to effluent flow rate $Q_{eff}$; alternatively solute clearance may be estimated as function of all flow settings and of dialyzer/filter related parameters; alternatively appropriate sensors could be placed to measure conductivity or concentration and thereby allow calculation of an actual clearance for a given solute (e.g. sodium), for instance using one of the methods described in EP patent n. 0547025 or EP patent n. 0658352 or EP patent n. 0920887 (which are here in incorporated by reference). In a further alternative the equations of above paragraphs a) and b) as described for the urea clearance could be used with RT (KO.A) and $SC_{xxx}$ adapted to take into account the specific solute 'xxx'.

In the course of the following description reference will be made to the above dose definitions which are relating to doses not normalized to patient body weight (BW) or patient surface area (BSA). Of course the same principles and formulas below described could be normalized to body weight or patient surface area by dividing the dose value by either body weight BW or surface area BSA. The examples at the end of the description comprise normalized CRRT doses and the patient weight.

Furthermore, the above defined doses could be corrected to take into account the predilution effect, when a fluid replacement line is present upstream the treatment unit, such as lines 51 and 67 in the enclosed drawings. Each of the above defined doses could be corrected multiplying the dose value times a dilution factor $F_{dilution}$:

$$Dose_{corr\_xxx}=F_{dilution} \cdot D_{xxx}$$

(with xxx = eff, conv, dial, etc);

The dilution factor $F_{dilution}$ may be defined according to one of the following:

Blood dilution factor:

$$F_{dilution_{blood}} = \frac{Q_b}{Q_b+Q_{rep.pre}+Q_{PBP}+Q_{cit}+Q_{syr}}$$

(Eq. 13)

Plasma dilution factor:

$$F_{dilution_{plasma}} = \frac{Q_p}{Q_p+Q_{rep.pre}+Q_{PBP}+Q_{cit}+Q_{syr}}$$

(Eq. 14)

Plasma water dilution factor:

$$F_{dilution_{pw}} = \frac{Q_{pw}}{Q_{pw}+Q_{rep.pre}+Q_{PBP}+Q_{cit}+Q_{syr}}$$

(Eq. 15)

Blood water dilution factor:

$$F_{dilution_{bw}} = \frac{Q_{bw}}{Q_{bw}+Q_{rep.pre}+Q_{PBP}+Q_{cit}+Q_{syr}}$$

(Eq. 15')

Where $Q_{cit}$ and/or $Q_{PBP}$ and/or $Q_{rep.pre}$ and/or $Q_{syr}$ in above equations is absent or negligible, the corresponding term may be cancelled (or set to 0). In the entire application, whenever a term in a formula is negligible (usually this applies to syringe flow rate, but not exclusively to it, the term may be neglected, i.e., its value considered 0 or the term removed.

In practice, the effluent dose corrected for the predilution effect would be: $Dose_{corr\_eff} = F_{dilution} \times D_{eff}$.

$F_{dilution}$ factor shall be selected according to the solute distribution and ability to move through red blood cells membrane (example: creatinine with slow diffusion through red blood cells => plasma dilution factor).

For example, in case the urea dose ($D_{urea}$) is assumed to be more or less identical to effluent flow rate and as urea is distributed in whole blood and can transfer quickly through the red blood cells membrane, the correction factor to consider for predilution shall refer to whole blood. Accordingly:

$$D_{urea\_corr} = F_{dilution_{blood}} \cdot K_{urea} = \frac{Q_b}{Q_b + Q_{rep.pre} + Q_{PBP} + Q_{cit} + Q_{syr}} \cdot Q_{eff}$$

(Eq. 16)

More sophisticated equations can provide for a more accurate estimate of urea clearance ($K_{urea}$) than $Q_{eff}$ (as previously indicated in Eq. 11 and 12), especially when operating with large flow rates or small filters (paediatric conditions).

**Buffer load definition**

Net buffer load during extracorporeal therapies ($J_{buffer\_load}$) is defined as the combination of (one or more of):

- Bicarbonate generated from the metabolism of citrate infused to the patient ($J_{met\_cit}$) and/or of lactate infused to the patient ($J_{met\_lact}$) - more in general generated from metabolism of all bicarbonate precursors,
- Bicarbonate balance from the extracorporeal blood therapy ($J_{HCO3\_bal}$) which may match with net loss or net gain for the patient,
- Acid infusion from e.g., citric acid content of the anticoagulant solution ($J_{H+}$), when relevant.

From the mathematical point of view, the general definition of net buffer load is:

$$J_{buffer\_load} = J_{met\_cit} + J_{HCO3_{bal}}(+J_{met\_lact}) - J_{H+}$$

(Eq. 17)

By convention, net buffer load is positive in the case extracorporeal blood therapy provides for a net gain in buffer/bicarbonate to the patient, and negative in the case of loss in buffer.

From a physiologic perspective, the extracorporeal blood therapy is expected to provide for a net buffer gain to the patient, as to balance the metabolic production of protons (proteins metabolism). However, a net buffer loss may be desirable in the scenario where the patient initiates the therapy in the situation of (severe) metabolic alkalosis. Buffer balance parameter is derived from a modelling of one or more of:

- citrate infusion rate to patient (citrate load),
- balance of bicarbonate and other buffers (e.g. lactate),
- assumption on citrate metabolism (1 mole citrate metabolized in 3 moles of bicarbonate),
- assumptions on patient systemic concentration for citrate, bicarbonate and other buffers (can be fixed values or computed from other sub-models).

Set buffer balance target does not match with the current buffer balance of the CRRT running therapy (which would require specific knowledge of current patient levels for citrate and bicarbonate), but with the (normalized) Net Buffer Load expected at a steady state where patient bicarbonate would stabilize at e.g., 25 mM.

Acid-base steady state is established slowly and measurable changes are commonly present after 24 hours; two days appear as a reasonable minimum to consider that acid-base status is reaching steady state in the context of CRRT.

In the framework of the buffer balance analysis here introduced, acid-base balance steady state is reached when:

- Patient systemic citrate concentration has stabilized across all the body compartments, thus leading to constant citrate load and bicarbonate generation,
- Patient bicarbonate concentration has stabilized across all the body compartments as a result of Net Buffer Load balancing the metabolic proton generation rate $G_{H+}$.

**Citrate load**

Citrate load is defined as the net infusion rate of citrate to the patient and it matches with the difference between the citrate infusion rate from the pre-blood-pump anticoagulant circuit ($J_{cit\_PBP}$) and the citrate removal rate into dialysate ($J_{cit\_dial}$), namely with the difference between citrate infusion in the blood circuit and citrate losses through dialyzer/dialysate.

From the mathematical point of view, below equations are directed to the computation of the amount of citrate returned to the patient (citrate load) which is relevant to the acid-base balance prescription during RCA.

The definition of patient citrate load is:

$$J_{citrate\_load} = J_{cit\_PBP} - J_{cit\_dial}$$

(Eq. 18)

Computation of citrate infusion can be expressed in two ways, according to the definition of citrate dose ($D_{cit}$). From the mathematical point of view, the definition of citrate infusion rate is:

$$J_{cit\_PBP} = Q_{cit} \cdot C_{cit\_PBP} = D_{cit} \cdot Q_b$$

(Eq. 19)

Citric acid and citrate forms are considered in the same way in this approach.

Removal rate of citrate into the dialysate is expressed from the definition of filter clearance for the citrate-calcium complexes ($K_{cit}$) and the citrate concentration (in plasma water) at the filter inlet.

From the mathematical point of view, the definition of citrate removal to effluent is:

$$J_{cit\_dial} = K_{cit} \cdot Cpw_{cit\_inlet}$$

(Eq. 20)

**Citrate Load (Main Variant)**

The hypotheses for modelling the citrate mass transfer in the extracorporeal blood circuit includes the assumptions that citrate is distributed in plasma (and not in red blood cells), that CRRT filter citrate clearance is computed also based on citrate concentration in plasma water for mass transfer computations, that consideration is taken of patient citrate metabolism and non-zero steady state citrate concentration at blood access and that patient citrate clearance is proportional to body weight.

The definition of plasma water flow rate at filter inlet is the following:

$$Q_{pw_{inlet}} = Q_{pw} + Q_{cit}(+Q_{syr} + Q_{PBP}) + Q_{rep.pre}$$

$$= Q_b \cdot (1 - Hct) \cdot Fp + Q_{cit}(+Q_{syr} + Q_{PBP}) + Q_{PBP} + Q_{rep.pre}$$

(Eq. 5)

Here (and in the following) it is assumed that no pre-blood-pump infusion line other than (citrate) anticoagulant line 51 is present and that no heparin is used/injected since RCA is provided (i.e., no PBP replacement fluid and no syringe flow rate are considered - the terms are included between brackets for the explained reason).

The equations (equations 21) for computation of citrate clearance in CRRT with non-zero dialysis fluid and filtration flow rates are as follows:

$$K_{cit} = \frac{Qpw_{inlet} \cdot Q_{dial} - f_{cit} \cdot (Qpw_{inlet} - SC_{cit} \cdot Q_{fil}) \cdot (Q_{dial} + SC_{cit} \cdot Q_{fil})}{Q_{dial} - f_{cit} \cdot (Qpw_{inlet} - SC_{cit} \cdot Q_{fil})}$$

$$f_{cit} = \left( \frac{Qpw_{inlet} - SC_{cit} \cdot Q_{fil}}{Qpw_{inlet}} \cdot \frac{Q_{dial} + SC_{cit} \cdot Q_{fil}}{Q_{dial}} \right)^{\frac{1}{\gamma_{cit}}}$$

$$\gamma_{cit} = e^{\left( \frac{SC_{cit} \cdot Q_{fil}}{\frac{S}{RT_{cit}}} \right)} - 1$$

(Eq. 21)

It is noted that citrate mass transfer parameters used for computation of above removal rate are known and constant values depending on the selected dialyzer.

For example, the following table reports the values for some used Baxter Prismaflex sets:

| Prismaflex set | S/RT$_{cit}$ ml/h | SC$_{cit}$ |
|---|---|---|
| M100 | 7500 | 1.0 |

Simpler equations might be used with lower accuracy. For example, K$_{cit}$ may be assumed equivalent to effluent flow rate Q$_{eff}$.

Plasma water citrate concentration at filter inlet ($Cpw_{cit\_inlet}$) can be defined considering or not an increase of patient citrate concentration.

Simple and rough estimate will consider that patient citrate concentration remains negligible all along the therapy (see next paragraph), however, more accurate approach needs to estimate the increase of patient systemic citrate concentration ($Cp_{cit\_pat}$) via an estimate of the patient citrate metabolic clearance (K$_{cit\_met}$) - see below. Indeed, during an RCA treatment, citrate concentration at the blood access is never zero as some citrate accumulates in the patient. This accumulation should be taken into consideration to avoid bias of about 10% (in case of neglecting). It requires the knowledge of the citrate metabolism rate (K$_{cit\_met}$) in liver and muscles of the patient that can vary in a wide range and significantly bias the final estimation. However it could be relevant to consider a 'minimum' accumulation that occurs for a patient having a 'normal' citrate metabolism. In this respect, patient citrate concentration is computed at steady state, assuming a typical metabolic clearance value of (about) 700 ml/min (from literature). Although not described in literature, patient citrate clearance is assumed as proportional to body weight.

The expression of patient systemic citrate concentration at steady state is the following:

$$Cp_{cit\_pat} = \frac{J_{citrate\_load}}{K_{cit\_met}}$$

(Eq. 22)

According to the above, estimation of patient citrate metabolic clearance (ml/min) is:

$$K_{cit\_met} = 700 \cdot \frac{BW}{72}$$

(Eq. 23)

The expression of citrate plasma water concentration at filter inlet is as follows:

$$Qp \cdot Cp_{cit_{pat}} + J_{cit_{PBP}} = Qpw_{inlet} \cdot Cpw_{cit\_inlet}$$

(Eq. 24)

Combination of above Equation 18, Equation 20, Equation 22 and Equation 24 allows eliminating citrate concentration parameters and expressing patient citrate load as a function of flow rates and clearances.

$$J_{citrate\_load} = D_{cit} \cdot Q_b \cdot \left(1 - \frac{K_{cit}}{Qpw_{inlet}}\right) \cdot \left(1 - \frac{1}{1 + \frac{K_{cit\_met}}{K_{cit}} \cdot \frac{Qpw_{inlet}}{Qp}}\right)$$

(Eq. 25)

The above citrate load mathematical relation is used in the context of the present application to relate the buffer load parameter (which depends on citrate load) with the citrate dose, the blood flow, the metabolic clearance and citrate clearance (depending on respective flow rates), as well as plasma flow rate and plasma water flow rate at filtration unit inlet.

## Citrate Load (Simplified Variant)

According to previously described main variant, the increase of patient systemic citrate concentration ($Cp_{cit\_pat}$) subsequent to citrate anticoagulation is considered and estimated through the equations 22 and 23. This choice leads to the above reported equation 25 for citrate load.

A simpler alternative to this formulation, is to neglect changes in the patient systemic citrate concentration and to take it as a constant, for example zero. Equation 22 and Equation 23 are consequently not used according to this alternative. In the case where patient citrate systemic concentration is assumed as zero ($Cp_{cit\_pat}$ = 0), Equation 24 and Equation 25 turn into following equations Equation 24' and Equation 25':

$$J_{cit\_PBP} = Qpw_{inlet} \times Cpw_{cit\_inlet}$$

(Eq. 24')

$$J_{citrate\_load} = D_{cit} \times Qb \times \left(1 - \frac{K_{cit}}{Qpw_{inlet}}\right)$$

(Eq. 25')

## Bicarbonate balance in extracorporeal blood circuit

Bicarbonate balance is defined as the net infusion or loss rate of bicarbonate in the extracorporeal blood treatment; it matches with the difference between the infusion rate from the dialysis and/or all replacement fluids ($J_{HCO3\_inf}$) and the bicarbonate removal rate into effluent ($J_{HCO3\_effl}$).

The definition of bicarbonate balance rate is the following:

$$J_{HCO3\_bal} = J_{HCO3\_inf} - J_{HCO3\_eff}$$

(Eq. 26)

The hypotheses for modelling the bicarbonate mass transfer in the extracorporeal blood circuit includes the assumptions that bicarbonate is distributed in both plasma and red blood cells, that bicarbonate concentration at blood access $Cp_{HCO3\_pat}0$ is fixed (e.g., equal to 25 mM); of course, a different (fixed) value for bicarbonate concentration at blood access may be used.

Other assumptions include that the citrate solution is bicarbonate free and no other pre-blood-pump infusion with bicarbonate is present (in the reverse case, the bicarbonate content/concentration is to be taken into consideration in the bicarbonate balance, namely $Q_{cit} \cdot C_{HCO3cit}$ and/or $Q_{PBP} \cdot C_{HCO3PBP}$), that CRRT filtration unit bicarbonate clearance is identical to urea clearance, and that bicarbonate removal in dialysate is computed according to similar equations as for citrate and consideration of bicarbonate concentration in plasma water for mass transfer computations.

Computation of bicarbonate infusion rate is based on knowledge of the fluids composition (i.e. known bicarbonate concentration).

$$J_{HCO3\_inf} = Q_{dial} \cdot C_{HCO3_{dial}} + Q_{cit} \cdot C_{HCO3_{cit}} + Q_{PBP} \cdot C_{HCO3_{PBP}} + Q_{rep.pre} \cdot C_{HCO3_{rep.pre}}$$
$$+ Q_{rep.post} \cdot C_{HCO3_{rep.post}} + Q_{HCO3} \cdot C_{HCO3_{HCO3post}}$$

(Eq. 27)

In case bicarbonate is in the calcium replacement infusion line 74 (unlikely due to potential risks of precipitation), then a term $Q_{ca} \cdot C_{HCO3ca}$ is to be added to equation 27. Fluid composition (i.e., bicarbonate concentration and/or replacement fluid prescription) may be entered by the physician (upon request from the dialysis apparatus) or read through e.g., a reader of the dialysis apparatus, for example by associating a product name with its bicarbonate content/concentration.

Equations of bicarbonate removal to dialysate are very similar to those of citrate; however, they differ for the fact that bicarbonate is present in the dialysis fluid, that the value of mass transfer parameter ($K_0A$) is different and that a fixed value is considered for patient systemic bicarbonate. Clearly, in case citrate was present in the dialysis fluid, the corresponding citrate load/balance may take into consideration such dialysis fluid citrate concentration in the corresponding equations for citrate and in the same way as below indicated for bicarbonate.

The definition of bicarbonate removal to dialysate is the following:

$$J_{HCO3\_eff} = Q_{dial} \cdot C_{HCO3_{dial}} + K_{HCO3} \cdot \left( Cpw_{HCO3\_inlet} - C_{HCO3\_dial} \right) + Q_{fil} \cdot C_{HCO3\_dial}$$

(Eq. 28)

Opposite to citrate, bicarbonate is easily transferred between red blood cells and plasma; whole blood water is thus considered for the computation of mass transfer to dialysate. Moreover, CRRT filter diffusive mass transfer coefficient of bicarbonate is taken identical to urea on the basis of their respective molecular weight (61 vs 60 g/mole). Sieving coefficient is taken as 1.

A constant physiological value of bicarbonate at blood access is considered.

The definition of blood water flow rate at filter inlet is as follows:

$$Qbw_{inlet} = Q_{bw} + Q_{cit} + Q_{syr} + Q_{PBP} + Q_{rep.pre}$$

$$= Q_b \cdot [(1 - Hct) \cdot Fp + Hct \cdot Frbc] + Q_{cit} + Q_{syr} + Q_{PBP} + Q_{rep.pre}$$

(Eq. 7)

The equations for computation of bicarbonate clearance in CRRT with non-zero dialysis fluid and filtration rates are similar to those for citrate; however, for the reasons above stated, mass transfer coefficients SC and $K_0A$ are different and the flow rate considered on the blood circuit is whole blood water flow (Qbw), instead of plasma water flow (Qpw). Indeed, bicarbonate is distributed in both plasma and red blood cells with the assumption of non-hindered transfer leading to the consideration of total blood water flow rate for the clearance estimate. The equations (equations 29) for computation of citrate clearance in CRRT with non-zero dialysis fluid and filtration rates are as follows:

$$K_{HCO3} = \frac{Qbw_{inlet} \cdot Q_{dial} - f_{HCO3} \cdot \left( Qbw_{inlet} - SC_{HCO3} \cdot Q_{fil} \right) \cdot \left( Q_{dial} + SC_{HCO3} \cdot Q_{fil} \right)}{Q_{dial} - f_{HCO3} \cdot \left( Qbw_{inlet} - SC_{HCO3} \cdot Q_{fil} \right)}$$

$$f_{HCO3} = \left( \frac{Qbw_{inlet} - SC_{HCO3} \cdot Q_{fil}}{Qbw_{inlet}} \cdot \frac{Q_{dial} + SC_{HCO3} \cdot Q_{fil}}{Q_{dial}} \right)^{\frac{1}{\gamma_{HCO3}}}$$

$$\gamma_{HCO3} = e^{\left( \frac{SC_{HCO3} \cdot Q_{fil}}{\left( \frac{S}{RT} \right)_{HCO3}} \right)} - 1$$

(Eq. 29)

It is noted that bicarbonate mass transfer parameters used for computation of above removal rate are known and constant values depending on the selected dialyzer.

For example, the following table reports the values for some used Baxter Prismaflex sets:

| Prismaflex set | S/RT$_{bic}$ ml/h | SC$_{bic}$ |
|---|---|---|
| M100 | 17000 | 1.0 |

Alternatively to the complexity of the above equations (Eq. 29), a reasonable approximation of bicarbonate clearance in most circumstances is given by below equation for simplified estimate of filter bicarbonate clearance:

$$K_{HCO3} = Q_{eff}$$

(Eq. 30)

The plasma water concentration at filter inlet is derived from the set of below equations 31, namely:

$$Cpw_{HCO3\_pat} = \frac{Cp_{HCO3\_pat}}{Fp}$$

$$Q_{bw} \cdot Cpw_{HCO3_{pat}} + Q_{cit} \cdot C_{HCO3_{cit}} + Q_{PBP} \cdot C_{HCO3_{PBP}} + Q_{rep.pre} \cdot C_{HCO3_{rep.pre}}$$

$$= Qbw_{inlet} \cdot Cpw_{HCO3\_inlet}$$

(Eq. 31)

From above equations, the expression of bicarbonate plasma water concentration at filter inlet is:

$$Cpw_{HCO3\_inlet} = \frac{Q_{bw} \cdot \dfrac{Cp_{HCO3_{pat}}}{Fp} + Q_{cit} \cdot C_{HCO3_{cit}} + Q_{PBP} \cdot C_{HCO3_{PBP}} + Q_{rep.pre} \cdot C_{HCO3_{rep.pre}}}{Qbw_{inlet}}$$

(Eq. 32)

**Lactate balance in extracorporeal blood circuit (optional)**

Lactate balance is defined as the net infusion or loss rate of lactate in the extracorporeal blood treatment; it matches with the difference between the infusion rate from the dialysate and/or replacement fluids ($J_{lact\_inf}$) and the lactate removal rate into dialysate ($J_{lact\_dial}$).

Lactate can be used as an alternative buffer to bicarbonate with the benefit of getting more stable solutions. Lactate based dialysis fluid is well known in dialysis; for example, it is used in the home dialysis version of the System One device from NxStage. Furthermore, lactate is also present in a certain number of bicarbonate solutions in the form of lactic acid as to control pH and solution stability. This is the case for the Baxter Hemosol/PrismaSol CRRT solutions range having 3 mM lactic acid. Similarly to citrate, lactate is quickly metabolized into bicarbonate when infused to the patient, with a mole per mole conversion rate. Lactate may be modelled in the very same way as bicarbonate, assuming the patient steady state plasma lactate concentration is about 1.5 mM.

Lactate clearance may be assumed identical to urea clearance, even though lactate molecular weight is about double of urea (112 vs 60 g/mole). However, clearance estimate error is minimal in the CRRT context where flow rates are the primary limiting factor. Of course, a more accurate estimate might be used, e.g. using the power dependence of $K_0A$ on solute molecular weight (meaning possible to derive $K_0A\_lactate$ from known $K_0A$ on Urea, creatinine, vitamin B12, inulin).

The hypotheses for modelling of lactate mass transfer in the extracorporeal blood circuit include the assumptions that lactate is distributed in plasma and red blood cells and that CRRT filtration unit lactate clearance is identical to urea clearance. Further, patient steady state plasma lactate concentration at blood access is assumed fixed at 1.5 mM; obviously a different fixed value may be assumed and used. Lactate mass balance in the extracorporeal blood circuit is computed with similar equations to bicarbonate considering metabolism of lactate load leading to 1 mole of bicarbonate per mole of lactate.

Mass transfer equations for lactate are as follows. The definition of lactate balance rate is the following:

$$J_{lact\_bal} = J_{lact\_inf} - J_{lact\_eff}$$

(Eq. 33)

Computation of lactate infusion rate is based on knowledge of the fluid composition (i.e. known lactate concentration); here it is assumed that no bicarbonate concentrate solution is infused/no lactate is present in the bicarbonate concentrate solution (otherwise, the terms $Q_{HCO3} \cdot C_{lact.HCO3}$ is to be considered); the same occurs for pre-blood-pump infusions.

$$J_{lact\_inf} = Q_{dial} \cdot C_{lact\_dial} + Q_{rep.pre} \cdot C_{lact_{rep.pre}} + Q_{rep.post} \cdot C_{lact_{rep.post}}$$

(Eq. 34)

Fluid composition (i.e., lactate concentration and/or replacement fluid prescription) may be entered by the physician or read through e.g., a reader of the dialysis apparatus.

The definition of lactate removal to dialysate is the following:

$$J_{lact\_eff} = Q_{dial} \cdot C_{lact\_dial} + K_{lact} \cdot \left( Cpw_{lact\_inlet} - C_{lact\_dial} \right) + Q_{fil} \cdot C_{lact\_dial}$$

(Eq. 35)

Lactate is easily transferred between red blood cells and plasma; whole blood water is thus considered for the computation of mass transfer to dialysate. Moreover, CRRT filter diffusive mass transfer coefficient of bicarbonate is taken identical to urea. Sieving coefficient is taken as 1.

Lactate clearance ($K_{lact}$) is considered equal to bicarbonate clearance ($K_{HCO3}$) and therefore the control unit calculates it in the same way with same equations previously presented. The expression of lactate plasma water concentration at filter inlet is:

$$Cpw_{lact\_inlet} = \frac{Q_{bw} \cdot \dfrac{Cp_{lact\_pat}}{Fp} + Q_{rep.pre} \cdot C_{lact_{rep.pre}}}{Qbw_{inlet}}$$

(Eq. 36)

**Net patient buffer load**

Net patient buffer load is defined in relation to citrate infusion rate to patient (namely, citrate load) and bicarbonate generation. To achieve this target, the hypotheses on citrate metabolism include the following assumptions: metabolism of citrate load leads to 3 moles of bicarbonate per mole of citrate and Net Buffer Load (NBL) may be reduced by the rate of acid infusion, such as citric acid. The expression of bicarbonate generation rate from citrate metabolism (at steady state) is the following:

$$J_{met\_cit} = 3 \cdot J_{cit\_load}$$

(Eq. 37)

The expression referring to acid infusion rate is as follows:

$$J_{H+} = 3 \cdot J_{citric\_acid} = 3 \cdot Q_{cit} \cdot C_{cit\_PBP}$$

(Eq. 38)

Combination of Equation 17, Equation 37 and Equation 38 leads to the expression for the net buffer load as a function of citrate load and bicarbonate balance:

$$J_{buffer\_load} = 3 \cdot J_{cit\_load} + J_{HCO3\_bal} - 3 \cdot J_{citric\_acid}$$

(Eq. 39)

It is remarked that the expression of $J_{citrate\_load}$ is given in Equation 25 (or simplified Eq. 25'), while full expression of $J_{HCO3\_bal}$ is to be derived from Equation 26, Equation 27, Equation 28, Equation 29 (or simplified Eq. 30) and Equation 32. $J_{citric\_acid}$ is to be derived from Equation 38.

From a therapy perspective, net buffer load should be positive as to neutralize the proton ($H^+$) generation rate $G_{H+}$ from metabolism. Literature report typical $G_{H+}$ values of about 1 mmol/day/kg, or 0.04 mmol/h/kg. Production of protons from metabolism is however strongly dependent on protein catabolism.

In case lactate is taken into consideration (optional), the expression for the net buffer load as a function of citrate load, lactate balance and bicarbonate balance becomes:

$$J_{buffer\_load} = 3 \cdot J_{cit\_load} + J_{HCO3\_bal} + J_{lact\_bal} - 3 \cdot J_{citric\_acid}$$

(Eq. 40)

In this case, in addition to mentioned equations for $J_{citrate\_load}$, $J_{HCO3\_bal}$ and $J_{citric\_acid}$, the equations for $J_{lact\_bal}$ are required, namely Equation 33, Equation 34, Equation 35, Equation 29 - if lactate clearance $K_{lact}$ is considered equal to bicarbonate clearance $K_{HCO3}$- (or corresponding equation for lactate clearance, or simplification $K_{lact}=Q_{eff}$) and Equation 36.

**Steady state acid-base balance prescription**

The apparatus control unit is configured to receive a steady state acid-base balance target, namely a steady state acid-base balance prescription set value before the start of the treatment. This additional prescription value influences various flow rates to be determined by the control unit 12. Indeed, the definition for the steady state acid-base balance target depends on almost all operating parameters and other prescription parameters (as well as on system configuration).

As previously indicated, the steady state acid-base balance prescription may be specified either as:

A) a steady state patient bicarbonate concentration (requiring an assumption on nNBL, namely nNBL0), or as

B) normalized Net Buffer Load or nNBL (requiring an assumption on bicarbonate concentration, namely $(Cp_{HCO3\_pat}0)$).

At steady state, nNBL is expected to balance the protons (H+) generation rate from patient metabolism.

**nNBL**

The control unit 12 of the apparatus for extracorporeal blood treatment allow the operator to enter a parameter ($J_{buffer\_load}/BW$) indicative of a steady state acid-base (or buffer) balance in the blood of the patient who has to undergo the CRRT blood treatment. This parameter defines a quantitative information on the intensity of the therapy with respect to the net patient buffer (bicarbonate) gain or loss. This parameter is of high and peculiar interest in the complex cases of citrate anticoagulation, but it remains also relevant to any extracorporeal dialysis therapy (run with systemic or no anticoagulation). In more detail, the control unit 12 receives the net buffer load at the apparatus setting (i.e., before the CRRT treatment is started).

The definition of normalized net buffer load (nNBL) is the following:

$$nNBL = \frac{J_{buffer\_load}}{BW}$$

(Eq. 41)

nNBL is chosen as the indicative parameter of acid-base balance level at steady state, and is expressed as amount of buffer infused per unit of time and per patient kg (mmol/h/kg).

In this respect, it is noted that $J_{buffer\_load}$ is linked to several other flow rates through the equations previously indicated. Indeed, $J_{buffer\_load}$ and consequently NBL or nNBL, is defined based on equations 17, 26, 27, 28 (or 26' combination of Eq. 26, 27 and 28), 29 (or 30), 32, 38; in case lactate is present definition is additionally based on equations 29 (or 30), 33, 34, 35 and 36. All these equations are function of one or more other flow rates of the CRRT apparatus configuration. Therefore, imposing a set value for the steady state acid base balance prescription generate a constraint between the various fluid flow rates and consequently influences the operating flow rates of the apparatus. By solving the mentioned equations, the apparatus control unit 12 may derive corresponding flow rates of operating parameters matching with the prescription (as apparent from the following detailed description). Notably, review of published clinical data on CRRT with RCA at steady state has shown good correlation of this nNBL parameter with both steady state patient bicarbonate and base excess. Therefore, instead of using the (normalized) net buffer load as above defined, the buffer balance parameter could be expressed as the steady state bicarbonate concentration, once assuming a 'default' value for the normalized net buffer load (nNBL); see further paragraph in this respect.

In the previously described embodiment, nNBL matches with the value of buffer balance when the patient reaches the assumed bicarbonate level (e.g; 25 mM) => $nNBL_{25}$). If $nNBL_{25}$ matches with the protons generation rate (G), then steady state is reached and patient will stabilize at the assumed $HCO_3$ level (25 mM). Alternatively, if $nNBL_{25}$ is larger than the protons generation rate, patient bicarbonate will increase up to Ceq such as $nNBL_{Ceq}$ matches with the (current) protons generation rate. In case $nNBL_{25}$ is lower than $G_{H+}$, then patient bicarbonate will stabilize at a lower value than the assumed level.

Though the parameter indicative of a steady state acid-base (or buffer) balance in the blood of the patient has been described as normalized over patient weigh, it is also possible to normalize the parameter over patient surface or over another patient-related variable. Of course, and even if it is not considered the best approach, the control unit 12 may also receive the Net Buffer Load (mmol/h) without any normalization.

**Variant with steady state HCO$_3$ indicator - $Cp_{HCO3\_pat}$**

Patient bicarbonate concentration ($Cp_{HCO3\_pat}$) could be alternatively taken as the indicative parameter of steady state acid-base equilibrium, providing that the (desirable/targeted) nNBL level has been chosen. In this scenario, previous equations may be re-arranged as to express the patient steady state bicarbonate concentration as a function of a predefined nNBL level, for example nNBL0 = 0.1 mmol/h/kg. Citrate equations, namely equations 18-25, 24' and 25', remain unchanged.

Differently, bicarbonate equations require some rearrangement. In more detail, expression of steady state patient bicarbonate (rearrangement of Equation 31) is the following:

$$Cp_{HCO3\_pat} = \frac{Fp}{Qbw}$$
$$\cdot \left( Cpw_{HCO3_{inlet}} \cdot Qbw_{inlet} - Q_{rep.pre} \cdot C_{HCO3rep.pre} - Q_{cit} \cdot C_{HCO3_{cit}} - Q_{PBP} \right.$$
$$\left. \cdot C_{HCO3_{PBP}} \right)$$

$$(\text{Eq. 42})$$

The expression of plasma water bicarbonate concentration at filter inlet (rearrangement of Equation 28) becomes:

$$Cpw_{HCO3\_inlet} = C_{HCO3\_dial} + \frac{J_{HCO3\_eff} - Q_{dial} \times C_{HCO3_{dial}} - Q_{fil} \times C_{HCO3\_dial}}{K_{HCO3}}$$

$$(\text{Eq. 43})$$

Merging equations 42 and 43 provides the following expression for steady state patient bicarbonate:

$$Cp_{HCO3\_pat} = \frac{Fp}{Qbw}$$
$$\cdot \left( C_{HCO3\_dial} + \frac{J_{HCO3\_eff} - Q_{dial} \times C_{HCO3_{dial}} - Q_{fil} \times C_{HCO3\_dial}}{K_{HCO3}} \cdot Qbw_{inlet} \right.$$
$$\left. - Q_{rep.pre} \cdot C_{HCO3rep.pre} - Q_{cit} \cdot C_{HCO3_{cit}} - Q_{PBP} \cdot C_{HCO3_{PBP}} \right)$$

$$(\text{Eq. 42'})$$

The expression of bicarbonate losses to effluent (from equations 17 and 26):

$$J_{HCO3\_eff} = J_{HCO3\_inf} - J_{HCO3_{bal}} = J_{HCO3\_inf} + J_{met\_cit} - J_{H+} - J_{buffer\_load}$$

$$(\text{Eq. 44})$$

The relation between chosen/set nNBL0 and $J_{buffer\_load}$ is as follows:

$$J_{buffer\_load} = nNBL0 \times BW$$

$$(\text{Eq. 45})$$

Therefore, equation 44 may be expressed as follows:

$$J_{HCO3\_eff} = J_{HCO3\_inf} + J_{met\_cit} - J_{H+} - nNBL0 \times BW$$

(Eq. 44')

$J_{buffer\_load}$ from equation 45, $J_{H+}$ from equation 38, $J_{met\_cit}$ from equation 37 and equation 25 or 25' and $J_{HCO3\_inf}$ from equation 27 are input to equation 44; the latter is then combined with equation 43 so that all terms of the this combined expression are known. $Cp_{wHCO3\_inlet}$ is finally introduced in equation 42 to derive an expression of variables defining the value of steady state patient bicarbonate which is the parameter to be set by the medical staff, which influences almost all fluid flow rates to be set.

As apparent, this solution provides an alternative parameter, namely the steady state patient bicarbonate concentration (which is again a parameter indicative of the steady state acid-base balance in the blood of the patient who has to undergo to a CRRT blood treatment), with respect to the normalized buffer load parameter, allowing medical staff to set a proper value and the control unit 12 to maintain a proper acid-base balance during the CRRT treatment.

**Therapy configuration parameters**

Therapy configuration parameters are user settings that can be set in the system as to configure the CRRT modality according to local protocols and/or some customer preferences. Though it is possible to set the therapy configuration parameters any time it is needed, these user settings are generally set once for all at a certain customer place and then used for all treated patients.

| Parameter | Configuration purpose |
|---|---|
| CRRT Dose | Selection of the formula to be used for CRRT dose / see detailed paragraph |
| Steady state acid-base balance target | Selection of the prescription parameter: $Cp_{HCO3\_pat}$ or nNBL |
| Blood flow rate | Selection as prescription parameter or not |
| Convection/diffusion split | Parameters used to configure the CRRT therapy with respect to the relative proportion of diffusion (dialysate flow) and convection (all infusion fluids), as well as distribution of infusion fluids pre or post filter / see next paragraphs for a more complete list and a detailed description of possible CRRT parameters |
| Predilution ratio | |
| Pre/post infusion ratio | |

Other default therapy configuration parameters may come from the system technical constraints and/or manufacturer choices. These constraints are embedded in the system and not user selectable. Examples of such parameters are flow operating ranges of the fluid circuits (e.g. calcium infusion in RCA), or mandatory relations between specific flow rates related to safety or technical considerations (e.g. maximum PBP or citrate flow rate versus blood flow rate.

**Treatment configuration parameters**

The treatment configuration parameters are variables specific of the patient to be treated and of the composition of prescribed fluids (which are also part of the medical prescription).

| Symbol | Parameter name | Comment |
|---|---|---|
| BW | Patient body weight (kg) | Generally required for prescription from $D_{CRRT}$ |
| Hct | Hematocrit (%) | Not required in some simple variants of assisted prescription |
| Filtration unit | CRRT filter type | Not required in the simplest approaches |
| $C_{cit}$ $C_{citr\_acid}$ | Composition of citrate solution in sodium citrate and citric acid with RCA (concentrations) | Generally required for prescription from $D_{cit}$ |

(continued)

| Symbol | Parameter name | Comment |
|---|---|---|
| $C_{HCO3.xxx}$ | Bicarbonate concentration of used solution 'xxx' (dialysate, replacement, PBP...) | Generally required for prescription of steady state acid-base balance target |
| $C_{lact.xxx}$ | Lactate concentration of used solution 'xxx' (dialysate, replacement, PBP...) | Generally required for prescription of steady state acid-base balance target in case lactate is considered |
| $C_{ca.xxx}$ | Calcium concentration of used solution 'xxx' (dialysate, replacement, PBP...) | Required with RCA only |
| $C_{ca.bal}$ | Calcium concentration in the solution used to balance Ca losses | |

Notably, hematocrit may alternatively be directly measured by the apparatus and on-line monitored.

Multiple default treatment configuration parameters may come together from the filter type, such as flow operating ranges and/or mass transfer coefficient(s). These are not user selectable and originate from the manufacturer. Of course, instead of providing the filter type to the apparatus (either by reading with a reader a filtration unit code or by inputting/selecting onto the user interface the used filtration unit), the relevant parameter values may be manually entered. As apparent from the following description, in the simplest approaches where clearance of small solutes is estimated identical to effluent flow rate, identification of the filter is not required. This type of approach becomes inaccurate in the scenario where dialysate and blood flow become similar, like in pediatrics application or high volume CRRT.

**Operating parameters**

Operating parameters are the CRRT monitor treatment parameters computed by the assisted prescription algorithm (here below illustrated) as a function of:

- Prescription parameters
- Therapy configuration parameters
- Treatment configuration parameters.

All the operating parameters are flow rates. The list of operating parameters is dependent on the anticoagulation method, as well as on the CRRT system capabilities and configuration.

In certain embodiments, calcium infusion may not be directly controlled by the CRRT system during RCA and infusion may be performed by a separate infusion pump. In this situation, the calculated prescribed value should be input in the external ion balancing infusion pump 75 and the control unit 12 may not verify or insure assisted prescription compliance. The same applies to anticoagulant infusion during systemic anticoagulation (usually a syringe boluses infusion).

The CRRT system may have a variable number of pumps available for fluid infusion at different locations along the extracorporeal blood circuit. See the examples of figures 2 to 6 having a different number of infusion lines and/or pumps.

The following table identifies operating parameters (associated to respective pumps) which will be considered in the examples. While this list refers to a maximum of 7 pumps (including the blood pump), systems including more pumps and infusion lines could be considered as well.

PBP and citrate flow rates are both indicated in the table, although they are typically delivered by the same pump in alternative configurations. PBP flow rate is specific of therapies not using citrate anticoagulation. Note that embodiment of figure 6 provides for two different pre-blood-pump infusion lines, namely an anticoagulant (citrate) infusion line 51 and a separate PBP infusion line 52.

| Symbol | Parameter name | Comment |
|---|---|---|
| $Q_{cit}$ | Citrate solution infusion flow rate in RCA | Parameters specific of citrate anticoagulation |
| $Q_{Ca}$ | Calcium solution infusion flow rate in RCA | |
| $Q_{dial}$ | Dialysis flow rate | If applicable |
| $Q_{PBP}$ | Pre blood pump infusion flow rate | If applicable |
| $Q_{rep.pre}$ | Pre filter replacement solution infusion flow rate | If applicable |

(continued)

| Symbol | Parameter name | Comment |
|---|---|---|
| $Q_{rep.post}$ | Post filter replacement solution infusion flow rate | If applicable |
| $Q_{HCO3}$ | Post-filter bicarbonate infusion flow rate | Specific to 'Bicarbonate Post Infusion' system configuration |
| $Q_{eff}$ | Effluent flow rate | Control CRRT fluid balance process |
| $Q_b$ | Blood flow rate | In case blood flow is not a prescription parameter |

**Assisted prescription with no/systemic anticoagulation**

In the context of systemic anticoagulation, a concentrated anticoagulant solution is infused in the extracorporeal blood circuit. While this prescription is clinically important, heparin infusion is not particularly relevant with respect to the CRRT prescription and calculation of flow rates since (heparin) infusion flow rate is very low, typically less than 5 ml/h matching with about 0.1% of all the fluid infusion rates, thus not impacting CRRT dosage measurably, and used anticoagulant does not interfere with acid-base equilibrium. Due to these characteristics, anticoagulation prescription in systemic anticoagulation during RRT can be seen as fully independent from the RRT prescription in itself and will not be treated in the next section which can thus address simultaneously all prescriptions using no or systemic anticoagulation.

When no or systemic anticoagulation is used, the CRRT prescription can be built from the following:

1. CRRT dose,
2. Steady-state acid-base equilibrium target,
3. Patient fluid removal rate (PFR),
4. Optionally, blood flow rate.

Anticoagulant prescription could be listed as well in the case of systemic anticoagulation; it however does not interfere measurably with any of the other prescription parameters and can be addressed independently.

In a system with 3 fluid infusion pumps and one effluent pump, 4 fluid flow rates are to be defined from the above 3 first prescription parameters. There is thus one degree of freedom, which may allow to consider one additional setting from the therapy configuration in the definition of operating flow rate parameters. If one additional infusion pump is added, then two degrees of freedom are available and two further settings might be selected to allow the control unit to propose corresponding flow rates matching prescription and settings. With one infusion pump less, the 3 first prescription parameter are sufficient to define the fluid flow rates for the therapy configuration.

**Qualitative dependence of operating parameters**

Next table provides an indication for the interdependence between operating and prescription parameters in no or systemic anticoagulation; notably, this dependence is conditional to the definition given to/selection of the CRRT dose.

| Operating parameter | Prescription parameter | | | |
|---|---|---|---|---|
| | CRRT dose | Acid-base equilibrium | PFR | Blood flow |
| $Q_{dial}$ | X1 | X3 | - | - |
| $Q_{PBP}$ | X1 | X3 | - | - |
| $Q_{rep.pre}$ | X1 | X3 | - | - |
| $Q_{rep.post}$ | X1 | X3 | - | - |
| $Q_{HCO3}$ | X1 | X3 | - | - |
| $Q_{eff}$ | X1 | X3 | X4 | - |
| $Q_b$ | X2 | X3 | - | X5 |

X1: contribution of flow rates to the CRRT dose is dependent on the definition given to it (see previous paragraph). Most relevant definitions make use of all flow rates (whatever simply referring to $Q_{eff}$ or to an estimated clearance). X2:

contribution of blood rate to CRRT dose is also dependent on the chosen dose definition. Blood flow is relevant as soon as predilution effects are considered (which is not the case when simply referring the effluent flow rate as CRRT dose).

X3: all infusion fluids or dialysis fluid contribute to the acid-base balance according to the fluid composition associated to each infused solution. Blood flow rate impacts predilution and concentration of buffer solutes at the CRRT filter inlet.

X4: patient fluid removal rate prescription is directly controlled by the definition of effluent flow rate versus all the other infusion flow rates of the dialysis system, including the anticoagulant flow rate if relevant.

X5: when blood flow is defined as one prescription parameter, there is a loss of one degree of freedom in the definition of operating flow rates, meaning that less constraints from the therapy configuration parameters can be used. Number of degrees of freedom is defined as the difference between the numbers of operating flow rates to be computed minus the number of prescription parameters.

**The control unit and the assisted prescription algorithm**

The control unit 12 is connected to the various sensors, to the actuators for regulating the flow rate through the various lines (in the above examples these actuators comprise the pumps active on the lines and the switch valves) and to the user interface. The control unit 12 may comprise a digital processor (CPU) and necessary memory (or memories) such as memory 16, an analogical type circuit, or a combination thereof. In the course of the present description it is indicated that the control unit is "configured" or "programmed" to execute certain steps: this may be achieved in practice by any means which allow configuring or programming the control unit. For instance, in case of a control unit comprising one or more CPUs, a program may be stored in an appropriate memory containing instructions which, when executed by the control unit, cause the control unit to execute the steps herein described. Alternatively, if the control unit is of an analogical type, then the circuitry of the control unit may be designed to include circuitry configured in use to execute the steps herein disclosed. The steps in the method aspects are generally performed by the control unit unless differently indicated by the circumstances.

In the examples of figures 1 to 6, the control unit 12 is configured to execute a flow-rate setup procedure as described here below. Starting from the embodiment of figure 2, there are five pumps to be set, that define (or generate) five flow rates in total; the flow rates should be either received by the medical staff or calculated by the control unit 12.

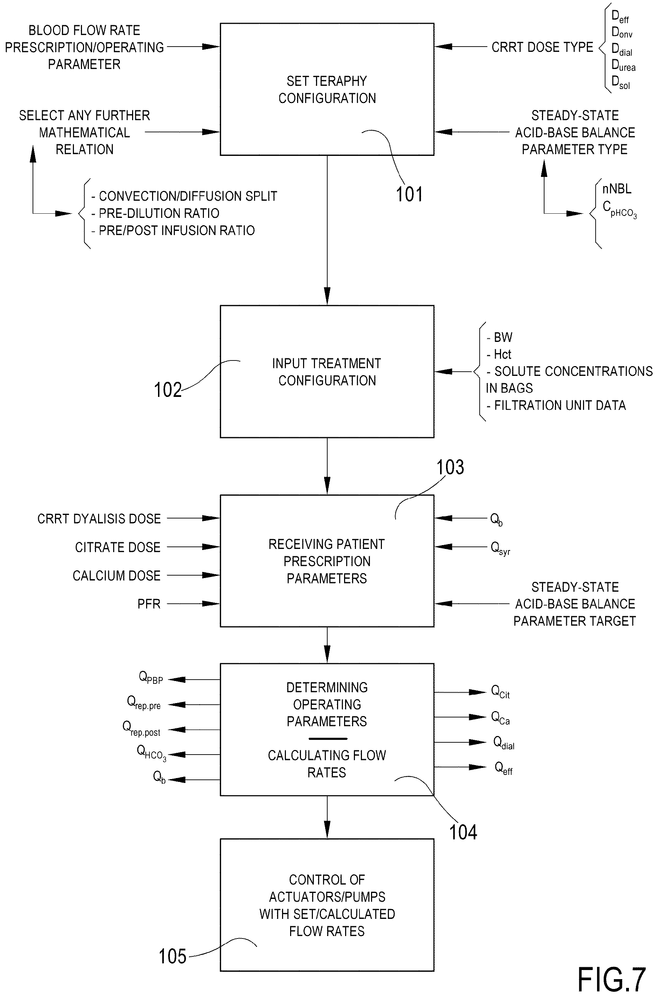

This flow-rate setup procedure comprises to initially set (or accept or maintain) the therapy configuration. Indeed, the control unit 12 is configured to allow the user to select one or more therapy configurations. Therapy configurations available are exemplificatively disclosed in the previous paragraph of the present description. The CRRT dialysis dose is to be selected among the described (or additional) possibilities. For example, CRRT dose as effluent dose $D_{eff}$ may be selected; alternatively, urea dose $D_{urea}$ or any different dose type may be selected. The acid base balance parameter is selected to, e.g., between (normalized) net buffer load (n)NBL or patient systemic bicarbonate plasma concentration. Blood flow rate may be decided as prescription parameter or as operating parameter. Finally, depending on the number of degrees of freedom (namely on the number of flow rates to be set by the control unit 12 minus the prescription parameters), none, one, two or more of a convection-diffusion relation, blood pre-dilution relation and pre-post relation may be selected as additional constraints (see step 100 in figure 7).

After setting/accepting the therapy configuration, the necessary treatment configuration is to be provided. See step 101 in figure 7. In particular, patient specific values and apparatus or disposable or solutions specific values have to be provided. Typically, patient body weight BW (or equivalent, for CRRT dose - body surface BSA depending on parameter normalization) needs to be provided. This is requested if any one of the patient prescription is normalized over patient weight as per the examples at the end of the present disclosure. Depending on apparatus configuration haematocrit Hct is required. If the specific apparatus configuration and mathematical relations to calculate the operating parameters so require, concentration of certain solutes contained in the relevant bags is to be provided as well. In particular, all solutes contributing to acid-base balance requires their concentration and specific location to be provided. For example, all bicarbonate solutions are generally requested, as well as citrate and calcium concentrations in the various bags; lactate and other bicarbonate precursors, such as acetate may be necessary, too. Finally, filtration unit data, such as KO.A of one or more solutes, might be requested. As already indicated, data may be entered manually or automatically acquired e.g., via a reader. For example, the apparatus memory may include data associated to a product code (a solution bag code or a filtration unit code, for example). Of course, the apparatus input system (graphic user interface) may prompt entering only necessary values/parameters or, alternatively, request all data and thereafter the control unit 12 exclusively makes use of the necessary ones. After the above steps, the apparatus is ready for receiving the prescription parameters, whose values are entered by the operator (see step 102 in figure 7). Once acquired, the control unit 12 uses one or more of the previously mentioned mathematical relations together with the entered treatment configuration data and the prescription parameters to determine the operating parameters consisting of the various requested flow rates. In this respect, the control unit 12 uses the mathematical relations that links the treatment configuration data, the prescription parameters and the operating parameters as a system of equations that, once solved, provides all the relevant flow rates to be proposed or set. At the end of step 103, the control unit 12 stores the calculated operating parameters to the memory 16 and provides the calculated values to the user interface for displaying to the user. In general, the control unit 12 waits for acceptance of the proposed values before driving the corresponding actuators (pumps) and achieving the calculated flow rates in the respective lines. In another embodiment, the control unit 12 automatically set the pumps to achieve the flow rates in the respective lines once the flow rates are available.

In case the imposed conditions are conflicting, namely there is no solution to the flow rate system given the therapy and treatment configurations, the imposed and existing constraints and the prescription parameters, an alert to the user is provided. Possibly, one or more solutions in terms of proposed operating flow rates close to the imposed conditions are provided to the user via the user interface. For example, the further mathematical relations (convection-diffusion relation, blood pre-dilution relation and pre-post relation) are given lower priority with respect to other main mathematical relations linking the input prescription parameters. A rank may also be imposed to said convection-diffusion relation, blood pre-dilution relation and pre-post relation. Alternatively, in case blood flow rate is selected as prescription parameter, the blood flow is moved to operating parameter and its value is calculated, rather than received. In other terms, the control unit 12 may be configured to manage conflicting situations with different protocols of intervention. Optionally, more than one set of solutions for the operating flow rates is provided for the user selection.

Moving to the example of figure 2, only PBP infusion line and post dilution infusion line are used for replacement fluid. There are five flow rates to be set, namely blood flow rate $Q_b$, dialysis fluid flow rate $Q_{dial}$, PBP flow rate $Q_{PBP}$, post-dilution flow rate $Q_{rep.post}$, and effluent flow rate $Q_{eff}$. As previously indicated, once the therapy configuration is received, the treatment configuration has to be provided. If effluent dose is selected among available dose types (meaning that blood predilution does not influence the CRRT dialysis dose) and assumption is accepted that filtration unit clearance for small solutes can be taken sufficiently equal to effluent flow rate, no filter data are necessary. Body weight, haematocrit and bicarbonate concentration have to be entered into the memory via user interface. The control unit waits then to receive a prescribed dose value $D_{set}$, a prescribed value for the fluid removal rate $Q_{pfr}$ from the patient, a steady state acid-base balance parameter target and a setting for the blood flow rate $Q_b$ (see step 102 in figure 7).

The memory 16 associated with or connected to the control unit 12 stores the plurality of mathematical relations correlating the fluid flow rates $Q_{PBP}$, $Q_{rep.post}$, $Q_{eff}$, $Q_b$, $Q_{PFR}$, and $Q_{dial}$. Since we have four prescription parameters and five flow rates to be set, an additional constraint is selectable. In this respect, there are additional mathematical relations stored in said memory, which may be the following:

- a convection-diffusion relation, relating the total fluid flow rate through said infusion lines + patient fluid removal rate $Q_{PBP} + Q_{rep.post} + Q_{PFR}$ with the fluid flow rate through said dialysis fluid line $Q_{dial}$, the convection-diffusion relation may define in practice a first ratio $R_1 = (Q_{PBP} + Q_{rep.post} + Q_{PFR})/(Q_{dial})$,
- a blood pre-dilution relation, relating the flow rate of blood or of plasma $Q_b$ or $Q_p$ and the fluid flow rate infused in the blood withdrawal line $Q_{PBP}$ through said pre-dilution infusion line 52; the blood pre-dilution relation may define a second ratio $R_2 = Q_b /(Q_{PBP})$ or $R_2 = Q_p /(Q_{PBP})$;
- a pre-post relation, relating the fluid flow rates $Q_{PSP}$ through pre-dilution infusion line with the fluid flow rate through the post-dilution infusion line $Q_{rep.post}$; the pre-post relation may define in practice a third ratio $R_3 = (Q_{PBP})/(Q_{rep.post})$. The control unit 12 allows the user, e.g. through user interface 12, to select one of said relations and then may calculate the set values of all flow rates $Q_{PBP}$, $Q_{rep.post}$, $Q_{eff}$; and $Q_{dial}$ (step 103 in figure 7) by applying the set value of the dose $D_{set}$, the target value for the steady state acid-base balance, the set blood flow rate $Q_b$ and the set value of fluid removal rate $Q_{pfr}$ entered by the operator to the mathematical relations previously discussed that links the steady state acid-base balance parameters to the various flow rates and the further mathematical relation above identified and selected by the user (and the fluid balance equation which needs to be satisfied in order to maintain the fluid balance in line with the prescription: $Q_{PBP} + Q_{rep.post} + Q_{dial} + Q_{pfr} = Q_{eff}$; FBE - fluid balance equation).

In case a syringe pump is also present/considered for injecting an auxiliary fluid, e.g. heparin, in the blood withdrawal line, then the above equation may be modified accordingly to account for the syringe flow rate.

Note that preset values for each one of said first, second and third ratios $R_1$, $R_2$, $R_3$ may be pre-stored in the memory or that the control unit may allow entry by an operator of a set value or a set range for each one of said first, second and third ratios $R_1$, $R_2$, $R_3$, e.g. via the user interface 12.

In one alternative, the memory 16 of the apparatus of figure 1 may (further) store a plurality of optimization criteria, which the control unit 12 may use to calculate the set values for the relevant operating flow rates in alternative or in combination with the above ratios $R_1$, $R_2$, $R_3$.

For instance, optimization criteria stored in memory 16 may comprise a first optimization criterion imposing that an emptying time of at least one among the containers of fresh fluid 14, 64, 10, 11 and/or a filling time of the waste container 62 is substantially same as, or multiple of, or proportional to the emptying time of one or more of the other containers of fresh fluid. A second optimization criterion stored in the memory 16 may impose that fluid consumption through the fluid lines is minimized. A third optimization criterion stored in memory 16 may impose that a life time of filtration unit 2 is maximized. A fourth optimization criterion stored in the memory 16 may impose that urea clearance or dialysance of a given solute is maximized.

In practice, if optimization criteria are stored in memory 16, the control unit 12 may be configured to allow the user to select (step 100), e.g. via the user interface 12, the criteria he wants to have satisfied and may be further configured to calculate the set values for the relevant operating flow rates based on the mathematical relations previously discussed that links the steady state acid-base balance parameters to the various flow rates and the selected optimization criteria and on the above mentioned fluid balance equation (FBE).

In case the selected mathematical relations (and eventually the selected criteria) are compatible, then the set flow rates are calculated based on the selected mathematical relations (and eventually optimization criteria). On the other hand, in case selected mathematical relations (and/or the selected criteria) are conflicting, the control unit 12 may be configured to execute one or more of the following sub-steps:

- inform the user; the user has then the power to re-enter compatible selections;
- assign a priority ranking to the mathematical relations (and/or selected criteria); the priority ranking is either predetermined or user adjustable: in any case the control unit is configured to ignore criteria or mathematical relations as soon as flow rates have been calculated from the prioritized criteria/mathematical relations;
- define a compromise between conflicting mathematical relations and/or criteria using preset rules.

In accordance with a variant, the control unit may use the flow-rate setup procedure to initially calculate the flow rates set values through the various lines and during a first interval in the treatment control the actuators for regulating using said calculated set values. Then, after a certain time interval or upon detection of a user input, the control unit may recalculate the set values for the flow rates through the various lines exclusively based on one or more different prescription value and/or differently selected (or modified in value) further mathematical relation and, apply the newly calculated set values during a second time period subsequent to the first time period. For instance the flow-rate setup procedure may allow setting of the flow rates such that a certain delivered dose is attained. On the other hand, if at a certain point the user wants to privilege bag-emptying synchronization he may select to impose the first optimization criteria so that the control unit may recalculate the set values of the flow-rates allowing to synchronize as possible the emptying of the fluid bags.

Figure 3 shows a different configuration of the CRRT apparatus, wherein regional anticoagulation protocol is used. In this second example, there are six flow rates to be set, namely $Q_{cit}$, $Q_{rep.post}$, $Q_{eff}$, $Q_b$, $Q_{ca}$, and $Q_{dial}$. However, regional anticoagulation requires a citrate dose and a calcium compensation parameter to be provided as patient prescription parameters. Therefore, the CRRT apparatus control unit 12 requires the medical staff to enter, CRRT dialysis dose, acid-base balance parameter, patient fluid removal rate, citrate dose and calcium compensation parameter. If blood flow rate is selected as prescription parameters, there are no more degrees of freedom. Therefore, once the physician has entered the requested info, no further mathematical equation may be selected. The control unit 12 calculates the operating parameter through the equation system of the relevant mathematical relations. Of course, based on the different system configuration (namely the additional post-infusion of calcium) and the different content of the used bags (e.g., citrate in the bag 10), the relevant mathematical relations that the control unit 12 uses are different with respect to the example of figure 2 (as apparent form the detailed examples here-below reported). The equation system defines some constraints, mainly due to citrate infusion and the need to keep the imposed acid-base parameter target that may leads to a system with no solutions for the flow rates. Again, one or more of the approaches above defined may be taken is such a situation. Again, the detailed examples clarify this situation.

The configuration of figure 4 provides for an HDF configuration, wherein a pre-dilution line and a bicarbonate post-dilution line are used. In particular, the post-dilution line infuses a replacement solution with high bicarbonate concentration. This configuration allows some more freedom to regulate acid-base balance. Also in this configuration, the control unit 12 makes use of the relevant mathematical relations based on CRRT apparatus design, on decided prescription parameters type/nature, the treatment configuration parameters, the imposed values for the prescription parameters and the corresponding relevant mathematical relations.

Figure 5 is directed to another embodiment of the CRRT apparatus: HDF configuration with regional anticoagulation including a post-dilution replacement solution and a post-dilution bicarbonate solution in addition to the calcium reinfusion line. There are seven flow rates to be set and usually six prescription parameters, namely blood flow rate, acid-base balance target, CRRT dialysis dose, citrate dose and calcium reinfusion parameter value. Therefore, once again it is possible for the user to additionally select one among the convection/diffusion split, the pre-dilution ratio and the pre-post infusion ratio. The configuration of figure 5 requires the control unit 12 to use many of the mathematical relations to properly take into account the interactions of the various fluids, particularly in terms of acid-base balance.

### Examples

In the present section, some examples are provided to better explain working of the CRRT apparatus.

In the context of CRRT with no or systemic anticoagulation, the possibility to adjust the steady state acid-base balance equilibrium when using fluids having all similar bicarbonate content appears limited. Some possibilities exist when using simultaneously one fluid with low bicarbonate content (e.g. below 25 mM) and one with a higher content (e.g. higher than 40 mM).

Reference is made to the apparatus as shown in figure 2, provided with 4 fluid pumps (dialysis fluid pump 25, effluent pump 26, PBP infusion pump 53 and post-dilution infusion pump 65) and thus capable of running an HDF therapy.

| Fluid circuit / pump | Symbol | Present |
|---|---|---|
| dialysis fluid pump/supply line | dial | X |
| PBP infusion pump/line | PBP | X |
| pre-dilution infusion pump/line | rep.pre | absent or no use |
| post-dilution infusion pump/line | rep.post | absent or no use |
| post-dilution bicarbonate infusion line/pump | $HCO_3$ | absent or no use |
| effluent pump/line | eff | X |
| blood pump | b | X |

Next examples S1-S5 (S stands for systemic anticoagulation or no anticoagulation) are based on the above circuit configuration and on figure 2.

### Example S1

Effluent dose is selected as CRRT dose, therefore blood predilution does not influence dose and equations are simpler to solve. In this context of simple approach, filter clearance of small solutes (like bicarbonate) is taken equal to effluent flow rate as well; consequently no information is required on the filter type/performances.

Further, steady state bicarbonate is selected as the acid-base balance prescription parameter (when selecting steady state bicarbonate, the apparatus control unit assumes that nNBL0 is equal to 0.1 mmol/h/kg); blood flow rate is also selected among prescription parameters (reducing the degrees of freedom of the configuration). Five pumps are available and four prescription values are entered. Therefore, the system has one degree of freedom and one therapy configuration constrain may be further selected to obtain a calculation of all operating flow rate parameters.

Among therapy configurations, any of convection/diffusion split, predilution ratio and pre/post infusion ratio may be selected. Predefined (modifiable) values for these relations are also included in the memory and presented to the user.

Further, necessary treatment configuration values are also entered by the operator or acquired by the control unit 12.

| Therapy configuration | |
|---|---|
| CRRT Dose | Effluent |
| Acid-base balance target | $Cp_{HCO3\_pat}$ (nNBL0=0.1 mmol/h/kg) |
| Blood flow rate | Prescription |
| **Convection/diffusion split** | **50-50 or R1=1** |

| Treatment configuration | |
|---|---|
| BW (kg) | 75 |
| Hct (%) | 32 |
| Filter | No data |
| $C_{HCO3dial}$ (mM) | 32 |
| $C_{HCO3PBP}$ (mM) | 30 |

(continued)

| Treatment configuration | |
|---|---|
| $C_{HCO3rep.post}$ (mM) | 32 |

It is assumed that the following prescription is entered by the user via user interface 12:

- Patient: BW (body weight) = 75kg
- Hct (%) = 32
- CRRT dose $D_{eff}$ = 28 ml/kg/h, where the dose is an 'effluent dose' per kg
- steady state bicarbonate target value: $Cp_{HCO3\_pat}$= 25 mmol/l
- patient fluid removal rate: $Q_{PFR}$ = 120 ml/h
- blood flow rate: $Q_b$ = 200 ml/min

| Prescription | | unit |
|---|---|---|
| $D_{CRRT}$ | 28 | ml/kg/h |
| $Cp_{HCO3\_pat}$ | 25 | mmol/L |
| $Q_{PFR}$ | 120 | ml/h |
| $Q_b$ | 200 | ml/min |
| *Degrees of freedom* | *5-4 = 1* | - |

There are a number of additional constrains stored in the in memory 16, particularly in terms of min/max flow rates; for example: dialysate flow rate ($Q_{dial}$): 0 to 6000 ml/h and replacement flow rate ($Q_{rep.post}$): 0 to 4000 ml/h.
The operator selects (or has previously configured):

- diffusion/convection split: 50-50, namely a ratio: $R_1$ = 1.0

The control unit 12 then computes the operating parameters, namely the various flow rates as follows:

| Operating flow rate parameters (ml/h) | |
|---|---|
| $Q_{dial}$ (ml/h) | 990 |
| $Q_{PBP}$ (ml/h) | 220 |
| $Q_{rep.post}$ (ml/h) | 770 |
| $Q_{eff}$ (ml/h) | 2100 |
| *$Q_b$ (ml/min)* | *200* |

In particular:
Effluent flow rate is determined from the CRRT dialysis dose and body weight as follows: $Q_{eff}$ = 28 x 75 = 2100 ml/h,

- $Q_{dial}$, $Q_{PBP}$, $Q_{rep.post}$, and $Q_{eff}$, are defined through below equation set, explicitly indicated:

$$R_1 = Q_{dial}/(Q_{rep.post} + Q_{PBP}) = 1.0$$

$$Q_{dial} + Q_{PBP} + Q_{rep.post} + Q_{PFR} = Q_{eff} = 2100$$

With the above two equations, $Q_{dial}$ can be directly calculated and set to 990 ml/h; further $Q_{PBP}$ + $Q_{rep.post}$ is equal to 990 ml/h.
$Q_{PBP}$ is then - arbitrarily - chosen as the remaining unknown parameter to compute in the next steps.
$Q_{fil}$ is calculated based on Eq. 8 that relates it to PBP flow rate, post-dilution flow rate and patient fluid removal rate:

$$Q_{fil} = Q_{PBP} + Q_{rep.post} + Q_{PFR} = 990 + 120 = 1110 \; ml/h$$

Then, patient steady state bicarbonate concentration ($Cpw_{HCO3pat}$) is defined through the following mathematical relations, namely Eq. 42, Eq. 43 (with the assumption that $K_{HCO3}$ is equalt to $Q_{eff}$ - see Eq. 30), which relates the various flow rates generating a further fluid flow rate constrain (in below equations, the terms referred to missing fluid lines have been removed from the general formulation provided in Eq. 42 and 43):

$$Cp_{HCO3\_pat} = \frac{Fp}{Qbw} \cdot \left( Cpw_{HCO3_{inlet}} \cdot Qbw_{inlet} - Q_{PBP} \cdot C_{HCO3_{PBP}} \right)$$

$$Cpw_{HCO3\_inlet} = C_{HCO3\_dial} + \frac{J_{HCO3\_eff} - Q_{dial} \times C_{HCO3_{dial}} - Q_{fil} \times C_{HCO3\_dial}}{Q_{eff}}$$

All the terms of above two equations can be either computed or expressed as a function of $Q_{PBP}$, as shown below.
$Q_{bw}$ and $Q_{bw\_inlet}$ are determined using Eq. 6 and Eq. 7:

$$Qbw = Q_b \cdot [(1 - Hct) \cdot F_p + Hct \cdot Frbc] = 200 \cdot [(1 - 0.32) \cdot 0.95 + 0.32 \cdot 0.85] \cdot 60$$
$$= 11016 \; ml/h$$

$$Qbw_{inlet} = Q_{bw} + Q_{PBP} = 11016 + Q_{PBP}$$

$J_{HCO3\_eff}$ is determined based on Eq. 44:

$$J_{HCO3\_eff} = J_{HCO3\_inf} - J_{HCO3_{bal}} = J_{HCO3\_inf} - J_{buffer\_load}$$

$J_{buffer\_load}$ is determined based on Eq. 45 (with assumed nNBL0=0.1):

$$J_{buffer\_load} = nNBL0 \times BW = 7,5 \; mmol/h$$

$J_{HCO3\_inf}$ is determined based on Eq. 27, referencing $J_{HCO3\_inf}$ in mmol/h and $Q_{PBP}$ in ml/h:

$$J_{HCO3\_inf} = Q_{dial} \cdot C_{HCO3_{dial}} + Q_{rep.post} \cdot C_{HCO3_{rep.post}} + Q_{PBP} \cdot C_{HCO3_{PBP}}$$

$$J_{HCO3\_inf} = \frac{990}{1000} \cdot 32 + \left( \frac{990 - Q_{PBP}}{1000} \right) \cdot 32 + \frac{Q_{PBP}}{1000} \cdot 30 = 63.36 - \frac{Q_{PBP}}{1000} \cdot 2$$

With the above expressions of bicarbonate rates, following expression of plasma water concentration of bicarbonate at filter inlet can be derived:

$$Cpw_{HCO3\_inlet} = 32 + \frac{63.36 - 7.5 - 0.002 \cdot Q_{PBP} - 31.68 - 35.52}{\frac{2100}{1000}} = 26.6 - 0.000952 \cdot Q_{PBP}$$

Expression of steady state patient plasma bicarbonate thus becomes:

$$Cp_{HCO3\_pat} = 25 = \frac{0.95}{11016} \cdot \left( (26.6 - 0.000952 \cdot Q_{PBP}) \cdot (11016 + Q_{PBP}) - Q_{PBP} \cdot 30 \right)$$

$$25 \cdot \frac{11016}{0.95} = 289894 = 293026 + (26.6 - 10.49 - 30) \cdot Q_{PBP} - 0.000952 \cdot Q_{PBP}^2$$

$$3132 - 13.89 \cdot Q_{PBP} - 0.000952 \cdot Q_{PBP}^2 = 0$$

One single solution to above equation is positive and matches with the expected PBP flow rate:

$$Q_{PBP} = \frac{13.89 - \sqrt{13.89^2 - 4 \cdot (-0.000952) \cdot 3123}}{2 \cdot (-0.000952)} = \frac{-13.89 + 14.31}{2 \cdot 0.000952} = 222 \, ml/h$$

Post-replacement flow rate is then easily derived as:

$$Q_{rep.post} = 990 - Q_{PBP} = 768 \, ml/h$$

The flow rate values are finally rounded.

Example S2

Similarly to previous example S1, effluent dose is selected as CRRT dose (blood predilution does not influence dose), filter clearance of small solutes (like bicarbonate) is considered equal to effluent flow rate, too.

Vice versa, normalized net buffer load nNBL is selected as acid-base balance prescription (when nNBL, the apparatus control unit assumes that $Cp_{HCO3\_pat}0$ is equal to 25mM); blood flow rate is also selected among prescription parameters (reducing the degrees of freedom of the configuration). As in the previous example, five pumps are available and four prescription values are entered. Therefore, the system has one degree of freedom and one therapy configuration constrain may be further selected to obtain a calculation of all operating flow rate parameters.

Among therapy configurations, any of convection/diffusion split, predilution ratio and pre/post infusion ratio may be selected. Predefined (modifiable) values for these relations are also included in the memory and presented to the user.

Further, necessary treatment configuration values are also entered by the operator or acquired by the control unit 12. Changed values are identified with different background shading.

| Therapy configuration | |
|---|---|
| CRRT Dose | Effluent |
| Acid-base balance target | nNBL ($Cp_{HCO3\_pat}$ =25mM) |
| Blood flow rate | Prescription |
| **Pre/post infusion ratio** | **33-67** |
| Note that acid-base balance prescription is switched to nNBL and solutions with low and high bicarbonate concentrations are used to provide for some possibilities of adjustment of acid-base balance. | |

| Treatment configuration | |
|---|---|
| BW (kg) | 75 |
| Hct (%) | 32 |
| Filter | No data |
| $C_{HCO3dial}$ (mM) | 32 |
| $C_{HCO3PBP}$ (mM) | 22 |
| $C_{HCO3rep.post}$ (mM) | 40 |

It is assumed that the following prescription is entered by the user via user interface 12:

- Patient: BW (body weight) = 75kg
- Hct (%) = 32
- CRRT dose $D_{eff}$ = 28 ml/kg/h, where the dose is an 'effluent dose' per kg
- normalized net buffer load nNBL target value: nNBL = 0.15 mmol/h/kg

- patient fluid removal rate: $Q_{PFR}$ = 120 ml/h
- blood flow rate: $Q_b$ = 200 ml/min

| Prescription | | unit |
|---|---|---|
| $D_{CRRT}$ | 28 | ml/kg/h |
| nNBL | 0.15 | mmol/h/kg |
| $Q_{PFR}$ | 120 | ml/h |
| $Q_b$ | 200 | ml/min |
| *Degrees of freedom* | *5-4 = 1* | - |

This time, the operator selects:

- Pre/post infusion ratio 33%-67%, namely a ratio: $R_2$ = 0.5

The control unit 12 then computes the operating parameters, namely the various flow rates as follows:

| Operating flow rate parameters (ml/h) | |
|---|---|
| $Q_{dial}$ (ml/h) | 600 |
| $Q_{PBP}$ (ml/h) | 460 |
| $Q_{rep.post}$ (ml/h) | 920 |
| $Q_{eff}$ (ml/h) | 2100 |
| *$Q_b$ (ml/min)* | *200* |

In particular:
Effluent flow rate is determined as follows: $Q_{eff}$ = 28 x 75 = 2100 ml/h,

- $Q_{dial}$, $Q_{PBP}$, $Q_{rep.post}$, and $Q_{eff}$; are defined through below equation set explicitly indicated:

$$R_2 = Q_{PBP}/Q_{rep.post} = 0.5$$

$$Q_{dial} + Q_{PBP} + Q_{rep.post} + Q_{PFR} = Q_{eff} = 2100$$

Then, normalized net buffer balance (nNBL) is defined on the following mathematical relations, namely Eq. 41, Eq. 26, Eq. 27, Eq. 28, which relates the various flow rates generating a further fluid flow rate constrain (the terms relating to missing fluid lines have been removed from the relevant equations):

$$nNBL = \frac{J_{buffer\_load}}{BW} = \frac{J_{HCO3\_bal}}{BW}$$

$$J_{HCO3\_bal} = J_{HCO3\_inf} - J_{HCO3_{eff}}$$

$$= Q_{dial} \cdot C_{HCO3_{dial}} + Q_{rep.post} \cdot C_{HCO3_{rep.post}} + Q_{PBP} \cdot C_{HCO3_{PBP}} - [Q_{dial} \cdot C_{HCO3_{dial}}$$

$$+ K_{HCO3} \cdot \left(Cpw_{HCO3_{inlet}} - C_{HCO3_{dial}}\right) + Q_{fil} \cdot C_{HCO3_{dial}}]$$

According to assumption $K_{HCO3}$ is approximated with the effluent flow rate (see Eq. 30):

$$J_{HCO3\_bal} = Q_{rep.post} \cdot C_{HCO3_{rep.post}} + Q_{PBP} \cdot C_{HCO3_{PBP}} - [Q_{eff} \cdot (Cpw_{HCO3_{inlet}} - C_{HCO3_{dial}}) + Q_{fil} \cdot C_{HCO3_{dial}}]$$

$Q_{fil}$ is calculated based on Eq. 8:

$$Q_{fil} = Q_{PBP} + Q_{rep.post} + Q_{PFR}$$

$Cpw_{HCO3_{inlet}}$ is determined based on Eq. 32, with the assumption that patient plasma bicarbonate concentration in the patient is imposed at 25 mM:

$$Cpw_{HCO3\_inlet} = \frac{Q_{bw} \cdot \dfrac{Cp_{HCO3_{pat}}0}{Fp} + Q_{PBP} \cdot C_{HCO3_{PBP}}}{Qbw_{inlet}}$$

$Q_{bw}$ **and** $Q_{bw\_inlet}$ are determined using Eq. 6 and Eq. 7:

$$Qbw = [Q_b \cdot (1 - Hct) \cdot F_p + Hct \cdot Frbc]$$

$$Qbw_{inlet} = Q_{bw} + Q_{PBP} = Q_b \cdot [(1 - Hct) \cdot Fp + Hct \cdot Frbc] + Q_{PBP}$$

Leading to $Q_{dial}$ = 600 ml/h, $Q_{rep.post}$ = 920 ml/h, $Q_{PBP}$ = 460 ml/h

**Example S3**

Similarly to previous example S2, effluent dose is selected as CRRT dose, filter clearance of small solutes (like bicarbonate) is considered equal to effluent flow rate, normalized net buffer load nNBL is selected as acid-base balance prescription (when nNBL, the apparatus control unit assumes that $Cp_{HCO3\_pat}$ is equal to 25mM), too. Vice versa, blood flow rate is removed from prescription parameters (increasing the degrees of freedom of the configuration). Now, five pumps are available and three prescription values are entered. Therefore, the system has two degrees of freedom and two therapy configuration constrains may be further selected to obtain a calculation of all operating flow rate parameters.

Among therapy configurations, any of convection/diffusion split, predilution ratio and pre/post infusion ratio may be selected. Predefined (modifiable) values for these relations are also included in the memory and presented to the user.

Further, necessary treatment configuration values are also entered by the operator or acquired by the control unit 12. Changed values are identified with different background shading.

| Therapy configuration | |
|---|---|
| CRRT Dose | Effluent |
| Acid-base balance target | nNBL *($Cp_{HCO3-pat}$ =25mM)* |
| Blood flow rate | Operating par. |
| **Convection/diffusion split** | **50-50** |
| **Pre/post infusion ratio** | **33-67** |

| Treatment configuration | |
|---|---|
| BW (kg) | 75 |
| Hct (%) | 32 |
| Filter | No data |
| $C_{HCO3dial}$ (mM) | 32 |
| $C_{HCO3PBP}$ (mM) | 22 |

(continued)

| Treatment configuration | |
|---|---|
| $C_{HCO3\text{rep.post}}$ (mM) | 40 |

Note that acid-base balance prescription is switched to nNBL and solutions with low and high bicarbonate concentrations are used to provide for some possibilities of adjustment of acid-base balance.

It is assumed that the following prescription is entered by the user via user interface 12:

- Patient: BW (body weight) = 75kg
- Hct (%) = 32
- CRRT dose $D_{eff}$ = 28 ml/kg/h, where the dose is an 'effluent dose' per kg
- normalized net buffer load nNBL target value: nNBL = 0.15 mmol/h/kg
- patient fluid removal rate: $Q_{PFR}$ = 120 ml/h

| Prescription | | unit |
|---|---|---|
| $D_{CRRT}$ | 28 | ml/kg/h |
| nNBL | 0.15 | mmol/h/kg |
| $Q_{PFR}$ | 120 | ml/h |
| | | |
| *Degrees of freedom* | *5-3 = 2* | - |

The operator selects:

- Diffusion/convection split: 50-50, namely a ratio: $R_1$ = 1.0
- Pre/post infusion ratio 33-67, namely a ratio: $R_2$ = 0.5

Since $Q_b$ is an operating parameter to be calculated, the convection diffusion further mathematical relation is added to the previous equations/mathematical relations used for example S2.

The control unit 12 then tries to compute the operating parameters, namely the various flow rates. The additional degree of freedom provided by the blood flow setting left to the control unit calculation allows to select the additional equation imposing the pre/post infusion ratio. The other used equations are the same used for the previous example S2; however, in the present case, the equation system has no solution with the imposed settings.

| Operating parameters | With the imposed flow configuration constraints | With pre/post ratio 33-67 | |
|---|---|---|---|
| $Q_{dial}$ (ml/h) | | 720 | 600 |
| $Q_{PBP}$ (ml/h) | | 420 | 460 |
| $Q_{rep.post}$ (ml/h) | No solution | 840 | 920 |
| $Q_{eff}$ (ml/h) | | 2100 | 2100 |
| $Q_b$* (ml/min) | | 100 | 200 |
| *Convection/diffusion split* | 50-50 | 66-34 | 71-29 |
| * $Q_b$: 100 ml/min is assumed as minimum acceptable value | | | |

In examples S1 and S2, it is possible to find operating parameters exactly matching with all prescription parameters, as well as to the CRRT flow configuration constraint. In the example S3, with one additional degree of freedom (blood flow), it is not possible to meet all the prescription parameters simultaneously to the two CRRT flow configuration constraints; in this case, the system should choose one out of the two flow configuration constraints (possibly from a predefined prioritization ranking) as to offer for operating flow rates meeting all prescription parameters, but the second constraint.

In the same example S3, once considering only one of the two therapy flow configuration constraints, multiple solutions exist. The system can either report the solution which is the closest to the 'dropped' flow constraint (in example S3, the set of parameters with $Q_b$ = 100 ml/min, wherein the convection diffusion split is 66%-34% instead of 67%-33%), or to offer several options to the operator. In the example, operating parameters with one larger blood flow rate ($Q_b$ = 200 ml/min) is suggested in addition to the closest solution.

Next examples S4 and S5 include the same system configuration as in previous series of examples is considered; moreover, examples S4 and S5 are identical to examples S1 and S2 except for the chosen definition of CRRT dose; in below examples S4 and S5, CRRT dose refers to urea clearance, and thus considers the effect of predilution.

In this context, more complex equations are used for the estimation of the clearances of urea (CRRT dose - see Eq. 12) and bicarbonate (acid-base balance analysis); mass transfer properties (KO.A) of the filter must be known.

### Example S4

This example is identical to example S1, but for the chosen definition of CRRT dose.

| Therapy configuration | |
|---|---|
| CRRT Dose | Urea clearance |
| Acid-base balance target | $Cp_{HCO3_{pat}}$ (nNBL=0.1 mmol/h/kg) |
| Blood flow rate | Prescription |
| **Convection/diffusion split** | **50-50** |

| Treatment configuration | |
|---|---|
| BW (kg) | 75 |
| Hct (%) | 32 |
| K0.A (ml/min) | 240 |
| $C_{HCO3_{dial}}$ (mM) | 32 |
| $C_{HCO3_{PBP}}$ (mM) | 30 |
| $C_{HCO3_{rep.post}}$ (mM) | 32 |

| Prescription | | unit |
|---|---|---|
| $D_{CRRT}$ | 28 | ml/kg/h |
| $Cp_{HCO3\_pat}$ | 25 | mmol/L |
| $Q_{PFR}$ | 120 | ml/h |
| $Q_b$ | 200 | ml/min |
| *Degrees of freedom* | *5-4 = 1* | - |

With the above prescription data and selected constrain, the control unit may propose to the user the following operating flow rate parameters:

| Operating flow rate parameters (ml/h) | |
|---|---|
| Qdial (ml/h) | 1030 |
| $Q_{PBP}$ (ml/h) | 400 |
| Qrep.post (ml/h) | 630 |
| Qeff (ml/h) | 2180 |
| *Qb (ml/min)* | *200* |

*Example S5*

This example is identical to example S2, but for the chosen definition of CRRT dose.

| Therapy configuration | |
|---|---|
| CRRT Dose | Urea |
| Acid-base balance target | nNBL ($Cp_{HCO3\_pat}$ 0=25 mM) |
| Blood flow rate | Prescription |
| **Pre/post infusion ratio** | **33-67** |

| Treatment configuration | |
|---|---|
| BW (kg) | 75 |
| Hct (%) | 32 |
| K0.A (ml/min) | 240 |
| $C_{HCO3dial}$ (mM) | 32 |
| $C_{HCO3PBP}$ (mM) | 22 |
| $C_{HCO3rep.post}$ (mM) | 40 |

| Prescription | | unit |
|---|---|---|
| $D_{CRRT}$ | 28 | ml/kg/h |
| nNBL | 0.15 | mmol/h/kg |
| $Q_{PFR}$ | 120 | ml/h |
| $Q_b$ | 200 | ml/min |
| *Degrees of freedom* | *5-4 = 1* | - |

With the above prescription data and selected constrain, the control unit may propose to the user the following operating flow rate parameters:

| Operating flow rate parameters (ml/h) | |
|---|---|
| $Q_{dial}$ (ml/h) | 870 |
| $Q_{PBP}$ (ml/h) | 395 |
| $Q_{rep.post}$ (ml/h) | 870 |
| $Q_{eff}$ (ml/h) | 2175 |
| *$Q_b$ (ml/min)* | *200* |

KO.A parameter relates to the type of CRRT filter in use and specifies the diffusive mass transfer performance for bicarbonate or urea, which are assumed to be identical in these examples; this assumption relies of the similar molecular weight to the 2 species (60 g/mole for urea, 61 g/mole for $HCO_3^-$).

As expected with the use of a CRRT dose integrating the effect of predilution, higher effluent flow rates are computed in examples S4 and S5 when compared to examples S1 and S2.

**Assisted prescription with citrate anticoagulation**

In the context of citrate anticoagulation and as previously described, a citrate solution is infused in the access line of the extracorporeal circuit, particularly next to the patient vascular access, upstream from the blood pump. Simultaneously, a calcium solution for re-establishing the ion content in the blood is infused directly into the patient or into the return line of the circuit as to balance calcium (and possibly other ions) losses to effluent.

Flow rates of the two infusions relate to the anticoagulation prescription (amount of citrate titrated to reach a certain level of ionised calcium in the extracorporeal blood circuit) and CRRT dose prescription (for the calcium losses), as well as to the concentrations of the respective citrate and calcium solutions.

Even in the context of 'concentrated' solutions (e.g. 110-140 mM of citrate, >500 mM calcium), the combined flow rate of citrate and calcium infusions has a measurable contribution to the CRRT dose (in the 5 to 10% range).

In addition, a fraction of infused citrate returns to the patient and is metabolized, leading to the release of calcium and production of bicarbonate impacting on the acid-base balance equilibrium.

Accordingly, at the opposite of systemic anticoagulation, citrate anticoagulation prescription has complex interactions with both CRRT dose and acid-base balance management. These interactions introduce significant level of complexity in the prescription of CRRT with citrate anticoagulation.

**Prescription**

When citrate anticoagulation is used, the CRRT patient prescription may be built from following clinical prescription parameters, all of which have a clear meaning for the medical staff dealing with CRRT treatments):

1. CRRT dose,
2. Steady-state acid-base equilibrium target,
3. Anticoagulant (Citrate) dose,
4. Ion (Calcium) balancing,
5. Patient fluid removal rate (PFR),
6. Optionally, blood flow rate.

In a system with 4 fluid pumps (dialysis + infusion pumps) and one effluent pump, 5 fluid flow rates are to be defined from the above 5 first prescription parameters. There is thus no degree of freedom left, and no option to consider additional setting from the therapy configuration (unless defining blood flow as operating parameter).

**Qualitative dependence of operating parameters**

Next table provides for the interdependence between operating and prescription parameters; importantly this dependence can be conditional to the definition given to the CRRT dose.

| Operating parameter | Prescription parameter | | | | | |
|---|---|---|---|---|---|---|
| | CRRT dose | Acid-base equilibrium | Citrate dose | Calcium balancing | PFR | Blood flow |
| $Q_{dial}$ | X1 | X3 | | - | - | - |
| $Q_{cit}$ | X1 | X3 | X6 | - | - | - |
| $Q_{Ca}$ | X1 | X3 | | X7 | | |
| $Q_{rep.pre}$ | X1 | X3 | | - | - | - |
| $Q_{rep.post}$ | X1 | X3 | | - | - | - |
| $Q_{HCO3}$ | X1 | X3 | | - | - | - |
| $Q_{eff}$ | X1 | X3 | | X7 | X4 | - |
| $Q_b$ | X2 | X3 | X6 | - | - | X5rca |

X1: contribution of flow rates to the CRRT dose is dependent on the definition given to it (see previous paragraph). Most relevant definitions make use of all flow rates (whatever simply referring to $Q_{eff}$ or to an estimated clearance). X2: contribution of blood rate to CRRT dose is dependent on the chosen dose definition. In the context of RCA, where citrate infusion is proportional to blood flow, blood flow rate will interfere with virtually all expressions of CRRT dose practically used.

X3: all fluid infusions or dialysate contribute to the acid-base balance according to the fluid composition associated to it. Impact of blood flow rate is more significant than in no or systemic anticoagulation as it defines the amount of citrate returned to the patient - and related bicarbonate generation - on top of the predilution effect. Contribution of calcium infusion is marginal when associated to very low infusion flow rates (< 20 ml/h).

X4: patient fluid removal rate prescription is directly controlled by the definition of effluent flow rate versus all the other infusion flow rates of the dialysis system, including the anticoagulant flow rate if relevant.

X5rca: when blood flow defined as prescription parameter, there is a loss of one degree of freedom in the definition of operating flow rates, meaning that less constraints from the therapy configuration parameters can be used. In most of current CRRT systems with RCA, no degree of freedom is left when selecting blood flow as prescription parameter and intending to prescribe acid-base balance equilibrium.

X6: citrate infusion flow rate in RCA is typically prescribed to be proportional to the blood flow, via a prescribed citrate dose coefficient. The resulting flow rate is typically infused pre-blood pump and matches with the PBP flow rate of systemic anticoagulation.

X7: calcium infusion is defined in relation to estimate losses to effluent. These losses are dependent on the clearance of citrate-calcium complexes, and thus on all operating flow rates, as well as on filter mass transfer characteristics. Basic systems may assume calcium losses proportional to effluent flow rate.

**Examples**

Although simplified expressions may be considered, the complexity of interactions between the mass transfer of citrate complexes and bicarbonate in the CRRT filter justifies the use of more accurate estimates of filter clearance, requiring access to the mass transfer properties of the filter in use. In this context, all examples will include these parameters.

Examples will illustrate use of 'concentrated' or 'diluted' citrate solutions, as commonly used in the clinical practice. In all the following examples, calcium concentration of the citrate and dialysate solutions is assumed to be zero. Presence of calcium in the post-infusion fluid will be considered in some examples.

All flow rates are rounded to the next ten, except when less than 100 ml/h.

The system configuration used throughout the next examples is comparable to the system configuration considered in examples S1 to S5, but for the addition of one pump for calcium infusion. Of course the pre-blood-pump infusion line infuses citrate, rather than a replacement solution as in previous examples S1 to S5. Though the pre-blood-pump infusion line can be physically the same used in the previous examples (see figure 1), a notation 'cit' is used to identify the respective flow rate and container concentration of citrate and different reference numbers are used (see figure 3 vs figure 2). The system for RCA includes 5 fluid pumps and the blood pump.

CRRT system definition for the examples C1 to C6 is indicated in the following table.

| Fluid circuit / pump | Symbol | Present |
|---|---|---|
| dialysis fluid pump/supply line | Dial | X |
| anticoagulant pump/infusion line (citrate) | Cit | X |
| ion balancing pump/infusion line (calcium) | Cal | X |
| pre-dilution infusion pump/fluid line | rep.pre | absent or no use |
| post-dilution infusion pump/fluid line | rep.post | X |
| post-dilution bicarbonate infusion pump/fluid line | $HCO_3$ | absent or no use |
| Effluent pump/line | Eff | X |
| Blood pump | B | X |

Next examples C1 to C5 are based on the above circuit configuration and on figure 3.

**Examples with effluent dose as CRRT dose**

In this first series of examples, CRRT dose is referred to effluent rate without correction for predilution. Parameters changed in the consecutive examples of this section are identified in coloured cells.

**Example C1**

The first reference example is built using normalized Net Buffer Load as acid-base balance prescription, use of diluted citrate solution and blood flow rate as clinical prescription parameters.

Therapy configuration is set as per the following table. Treatment configuration data are requested and entered according to the second table below reported.

| Therapy configuration | |
|---|---|
| CRRT Dose | Effluent |
| Acid-base balance target | nNBL ($Cp_{HCO3\_pat}$ 0=25 mM) |
| Blood flow rate | Prescription |

| Treatment configuration | |
|---|---|
| BW (kg) | 85 |
| Hct (%) | 27 |
| $K0.A_{HCO3}$ (ml/min) | 240 |
| $K0.A_{cit}$ (ml/min) | 109 |
| $C_{cit\_PBP}$ (mM) | 18 |
| $C_{ca}$ (mM) | 350 |
| $C_{HCO3dial}$ (mM) | 22 |
| $C_{HCO3rep.post}$ (mM) | 30 |
| $C_{ca\,rep.post}$ (mM) | 0 |

As indicated the following clinical prescription parameters are input by the medical staff:

| Prescription | | unit |
|---|---|---|
| $D_{CRRT}$ | 30 | ml/kg/h |
| nNBL | 0.15 | mmol/h/kg |
| $D_{cit}$ | 3.1 | mmol/L |
| CaComp | 110 | % |
| $Q_{PFR}$ | 90 | ml/h |
| $Q_b$ | 130 | ml/min |
| *Degrees of freedom* | *6-6 = 0* | - |

Since the prescription parameter provides for 6 constraints, there is no degree of freedom available to further set additional therapy configurations.

With the above apparatus set, the control unit determines the following operating flow rates ($Q_b$ is indicated, but it is a prescription parameter already set to 130 ml/min).

| Operating flow rate parameters (ml/h) | |
|---|---|
| $Q_{dial}$ (ml/h) | 970 |
| $Q_{cit}$ (ml/h) | 1343 |
| $Q_{ca}$ (ml/h) | 13.3 |
| $Q_{rep.post}$ (ml/h) | 130 |
| $Q_{eff}$ (ml/h) | 2550 |

In particular, effluent flow rate $Q_{eff}$ is determined based on CRRT dose and patient weight:

$$Q_{eff} = 30 \cdot 85 = 2550 \text{ ml/h}$$

Anticoagulant flow rate $Q_{cit}$ is related to blood flow rate by the citrate flow rate mathematical relation (Eq. 1):

$$Q_{cit} = \frac{D_{cit}}{C_{cit\_PBP}} \cdot Q_b = 1343 \text{ ml/h}$$

Since blood flow rate $Q_b$ and citrate dose $D_{cit}$ are imposed and citrate concentration is known, $Q_{cit}$ is immediately derived from above equation.

Calcium flow rate derives from calcium flow rate mathematical relation (Eq. 2); missing flow rates and terms equal to zero, or negligible have been removed from the general formulation of the description:

$$Q_{ca} = \frac{CaComp \cdot J_{Ca}}{C_{ca}}$$

As indicated in the previous examples, the effluent flow rate should respect the fluid balance (see equation 10):

$$Q_{eff} = Q_{cit} + Q_{dial} + Q_{rep.post} + Q_{PFR} + Q_{ca}$$

The last but more complex constraint is based on the normalized net buffer load parameter target nNBL due to its interaction with all flow rates and effects of buffer balance from bicarbonate and bicarbonate precursors, i.e., citrate. Net buffer load mathematical relations links the parameter with the citrate load and bicarbonate balance (Eq. 40 and Eq. 41):

$$J_{buffer\_load} = 3 \cdot J_{cit\_load} + J_{HCO3\_bal}$$

$$nNBL = \frac{J_{buffer\_load}}{BW}$$

The citrate load is determined based on citrate load mathematical relation (Eq. 25) that takes into account for citrate metabolic clearance:

$$J_{citrate\_load} = D_{cit} \cdot Q_b \cdot \left(1 - \frac{K_{cit}}{Qpw_{inlet}}\right) \cdot \left(1 - \frac{1}{1 + \frac{K_{cit\_met}}{K_{cit}} \cdot \frac{Qpw_{inlet}}{Qp}}\right)$$

Citrate clearance $K_{cit}$ derives from Eq. 21 (with $SC_{cit} = 1$) :

$$K_{cit} = \frac{Qpw_{inlet} \cdot Q_{dial} - f_{cit} \cdot (Qpw_{inlet} - Q_{fil}) \cdot (Q_{dial} + Q_{fil})}{Q_{dial} - f_{cit} \cdot (Qpw_{inlet} - Q_{fil})}$$

$$f_{cit} = \left(\frac{Qpw_{inlet} - Q_{fil}}{Qpw_{inlet}} \cdot \frac{Q_{dial} + Q_{fil}}{Q_{dial}}\right)^{\frac{1}{\gamma_{cit}}}$$

$$\gamma_{cit} = e^{\left(\frac{\frac{Q_{fil}}{S}}{RT_{cit}}\right)} - 1$$

Wherein the plasma water flow rate $Qpw_{inlet}$ at the filtration unit inlet comes from Eq. 5:

$$Qpw_{inlet} = Q_b \cdot (1 - Hct) \cdot Fp + Q_{cit}$$

And plasma water flow rate $Q_{pw}$ is calculated from Eq. 4:

$$Qpw = Q_b \cdot (1 - Hct) \cdot F_p$$

Ultrafiltration flow rate through the filtration unit 2 is (Eq. 8):

$$Q_{fil} = Q_{cit} + Q_{rep.post} + Q_{PFR} + Q_{ca}$$

Metabolic clearance for citrate is estimated with Eq. 22:

$$K_{cit\_met} = 700 \cdot \frac{BW}{72}$$

Vice versa, second term of the net buffer load, namely the bicarbonate balance $J_{HCO3\_bal}$, comes from e.g., Eq. 26':

$$J_{HCO3\_bal} = Q_{rep.post} \cdot C_{HCO3_{rep.post}} - K_{HCO3} \cdot \left( Cpw_{HCO3_{inlet}} - C_{HCO3_{dial}} \right) - Q_{fil}$$
$$\cdot C_{HCO3\_dial}$$

Bicarbonate clearance is more precisely estimated through bicarbonate clearance mathematical relations (Eq. 29); $SC_{HCO3} = 1$:

$$K_{HCO3} = \frac{Qbw_{inlet} \cdot Q_{dial} - f_{HCO3} \cdot \left( Qbw_{inlet} - Q_{fil} \right) \cdot \left( Q_{dial} + Q_{fil} \right)}{Q_{dial} - f_{HCO3} \cdot \left( Qbw_{inlet} - Q_{fil} \right)}$$

$$f_{HCO3} = \left( \frac{Qbw_{inlet} - Q_{fil}}{Qbw_{inlet}} \cdot \frac{Q_{dial} + Q_{fil}}{Q_{dial}} \right)^{\frac{1}{\gamma_{HCO3}}}$$

$$\gamma_{HCO3} = e^{\left( \frac{Q_{fil}}{\left( \frac{S}{RT} \right)_{HCO3}} \right)} - 1$$

Blood water flow rate $Qbw_{inlet}$ at filtration unit inlet comes from Eq. 7:

$$Qbw_{inlet} = Q_b \cdot [(1 - Hct) \cdot Fp + Hct \cdot Frbc] + Q_{cit}$$

Blood water flow rate is derived from Eq. 6:

$$Qbw = Q_b \cdot [(1 - Hct) \cdot F_p + Hct \cdot Frbc]$$

Finally, required plasma water bicarbonate concentration $Cpw_{HCO3\_inlet}$ at filter inlet from Eq. 32 is:

$$Cpw_{HCO3\_inlet} = \frac{Q_{bw} \cdot \frac{Cp_{HCO3_{pat}}0}{Fp}}{Qbw_{inlet}}$$

Wherein bicarbonate concentration in the patient plasma at steady state is fixed and assumed constant (e.g., 25 mM).

Using all the above mathematical relations, the control unit 12 determines all the operative prescription parameters.

### Example C2

Second example is using previous example C1 as reference and illustrates the changes in flow rates required further to a change of citrate dose prescription, which could be required to keep blood circuit ionized calcium within recommended levels (e.g. in the 0.25-0.35 mM range).

Therapy configuration is set as per the following table. Treatment configuration data are requested and entered according to the second table below reported.

| Therapy configuration | |
|---|---|
| CRRT Dose | Effluent |
| Acid-base balance target | nNBL ($Cp_{HCO3\_pat}$ 0=25 mM) |
| Blood flow rate | Prescription |

| Treatment configuration | |
|---|---|
| BW (kg) | 85 |
| Hct (%) | 27 |
| $K0.A_{HCO3}$ (ml/min) | 240 |
| $K0.A_{cit}$ (ml/min) | 109 |
| $C_{cit\_PBP}$ (mM) | 18 |
| $C_{ca}$ (mM) | 350 |
| $C_{CHO3_{dial}}$ (mM) | 22 |
| $C_{HCO3_{rep.post}}$ (mM) | 30 |
| $C_{ca_{rep.post}}$ (mM) | 0 |

| Prescription | | unit |
|---|---|---|
| $D_{CRRT}$ | 30 | ml/kg/h |
| nNBL | 0.15 | mmol/h/kg |
| $D_{cit}$ | 2.8 | mmol/L |
| CaComp | 110 | % |
| $Q_{PFR}$ | 90 | ml/h |
| Qb | 130 | ml/min |
| *Degrees of freedom* | *6-6 = 0* | - |

Since the prescription parameter provides for 6 constraints, there is no degree of freedom available to further set additional therapy configurations.

With the above apparatus set, the control unit determines the following operating flow rates ($Q_b$ is indicated, but it is a prescription parameter already set to 130 ml/min). As can be seen, all operating flow rates are significantly affected by the citrate dose change, but the effluent flow rate.

| Operating flow rate parameters (ml/h) | |
|---|---|
| $Q_{dial}$ (ml/h) | 720 |
| $Q_{cit}$ (ml/h) | 1213 |
| $Q_{cal}$ (ml/h) | 13.7 |
| $Q_{rep.post}$ (ml/h) | 510 |

(continued)

| Operating flow rate parameters (ml/h) | |
|---|---|
| $Q_{eff}$ (ml/h) | 2550 |

## Example C3

In the continuation of the two previous examples, example C3 illustrates changes required further to an increase of citrate dose.

Therapy configuration is set as per the following table. Treatment configuration data are requested and entered according to the second table below reported.

| Therapy configuration | |
|---|---|
| CRRT Dose | Effluent |
| Acid-base balance target | nNBL<br>($Cp_{HCO3\_pat}0$=25 mM) |

| Treatment configuration | |
|---|---|
| BW (kg) | 85 |
| Hct (%) | 27 |

| Blood flow rate | Prescription |
|---|---|
| ~~Convection/diffusion split~~ | ~~50-50~~ |
| ~~Predilution ratio~~ | ~~< 0.2~~ |
| ~~Pre/post infusion ratio~~ | ~~33-67~~ |
| | |
| | |
| | |

| | |
|---|---|
| K0.A$_{HCO3}$ (ml/min) | 240 |
| K0.A$_{cit}$ (ml/min) | 109 |
| $C_{cit\_PBP}$ (mM) | 18 |
| $C_{ca}$ (mM) | 350 |
| $C_{HCO3_{dial}}$ (mM) | 22 |
| $C_{HCO3_{rep.post}}$ (mM) | 30 |
| $C_{ca_{rep.post}}$ (mM) | 0 |

| Prescription | | unit |
|---|---|---|
| D$_{CRRT}$ | 30 | ml/kg/h |
| nNBL | 0.15 | mmol/h/kg |
| D$_{cit}$ | 3.5 | mmol/L |
| CaComp | 110 | % |
| Q$_{PFR}$ | 90 | ml/h |
| Q$_b$ | 130 | ml/min |
| *Degrees of freedom* | *6-6 = 0* | - |

Since the prescription parameter provides for 6 constraints, there is no degree of freedom available to further set additional therapy configurations.

With the above apparatus set, the control unit tries to determine the operating flow rates (in the second column, $Q_b$ is indicated, but it is a prescription parameter already set to 130 ml/min). As can be seen, there is no solution to the equation system compatible with the prescription.

All operating flow rates are significantly affected by the citrate dose change, but the effluent flow rate and it is not possible to achieve the desired targets. Third column provides a proposed solution considering $Q_b$ as an operating parameter (that may vary within prescribes limit values).

| Operating flow rate parameters (ml/h) | | With Qb as operating parameter |
|---|---|---|
| $Q_{dial}$ (ml/h) | | 940 |
| $Q_{cit}$ (ml/h) | No solution | 1400 |
| $Q_{cal}$ (ml/h) | | 13.0 |
| $Q_{rep.post}$ (ml/h) | | 110 |
| $Q_{eff}$ (ml/h) | 2550 | 2550 |
| *$Q_b$ (ml/min)* | | 120 |

## Example C4

The example C4 is based on C1 reference with the replacement of the diluted citrate solution with a 'concentrated' one; calcium solution is changed as well with a less concentrated one.

Highlighted changes are those in reference to C1 example.

Therapy configuration is set as per the following table. Treatment configuration data are requested and entered according to the second table below reported.

| Therapy configuration | |
|---|---|
| CRRT Dose | Effluent |
| Acid-base balance target | nNBL (*$Cp_{HCO3\_pat}$ 0=25 mM*) |
| Blood flow rate | Prescription |

| Treatment configuration | |
|---|---|
| BW (kg) | 85 |
| Hct (%) | 27 |
| $K0.A_{HCO3}$ (ml/min) | 240 |
| $K0.A_{cit}$ (ml/min) | 109 |
| *$C_{cit\_PBP}$* (mM) | 136 |
| *$C_{ca}$* (mM) | 100 |
| *$C_{HCO3dial}$* (mM) | 22 |
| *$C_{HCO3rep.post}$* (mM) | 30 |
| *$C_{carep.post}$* (mM) | 0 |

| Prescription | | unit |
|---|---|---|
| $D_{CRRT}$ | 30 | ml/kg/h |
| nNBL | 0.15 | mmol/h/kg |
| $D_{cit}$ | 3.1 | mmol/L |
| CaComp | 110 | % |
| $Q_{PFR}$ | 90 | ml/h |
| Qb | 130 | ml/min |
| *Degrees of freedom* | *6-6 = 0* | - |

Since the prescription parameter provides for 6 constraints, there is no degree of freedom available to further set additional therapy configurations.

With the above apparatus set, the control unit tries to determine the operating flow rates (in the second column,

$Q_b$ is indicated, but it is a prescription parameter already set to 130 ml/min). As can be seen, there is no solution to the equation system compatible with the prescription.

All operating flow rates are significantly affected by the citrate dose change, but the effluent flow rate and it is not possible to achieve the desired targets. Third column provides a proposed solution considering $Q_b$ as an operating parameter (that may vary within prescribes limit values).

| Operating flow rate parameters (ml/h) | | With Qb as operating parameter |
|---|---|---|
| $Q_{dial}$ (ml/h) | No solution | 1960 |
| $Q_{cit}$ (ml/h) | | 137 |
| $Q_{cal}$ (ml/h) | | 52.7 |
| $Q_{rep.post}$ (ml/h) | | 310 |
| $Q_{eff}$ (ml/h) | 2550 | 2550 |
| $Q_b$ (ml/min) | | 100 |

Above examples C1 to C4 illustrates the importance of blood flow rate during citrate anticoagulation for the definition of adequate acid-base balance when having fixed bicarbonate composition of the fluids.

## Examples with Urea Clearance dose as CRRT dose

Similarly to the series of example provided in no or systemic anticoagulation, next examples are provided in the context of RCA, where CRRT dose includes a factor for predilution, for example where referring to Urea clearance dose.

Same system configuration is the same used for examples C1 to C4 (see figure 3). The used mathematical relations are basically the same used for example C1, but the CRRT dialysis dose is determined using Eq. 12.

## Example C5

This example is built in reference to example C1, replacing effluent CRRT dose with urea clearance dose (see relevant paragraph). Changes are highlighted in reference to example C1.

Therapy configuration is set as per the following table. Treatment configuration data are requested and entered according to the second table below reported.

| Therapy configuration | |
|---|---|
| CRRT Dose | Urea clearance |
| Acid-base balance target | nNBL ($Cp_{HCO3\_pat}$ 0=25 mM) |
| Blood flow rate | Prescription |

| Treatment configuration | |
|---|---|
| BW (kg) | 85 |
| Hct (%) | 27 |
| $K0.A_{HCO3}$ (ml/min) | 240 |
| $K0.A_{cit}$ (ml/min) | 109 |
| $C_{cit\_PBP}$ (mM) | 18 |
| $C_{ca}$ (mM) | 350 |
| $C_{HCO3dial}$ (mM) | 22 |
| $C_{HCO3rep.post}$ (mM) | 30 |
| $C_{carep.post}$ (mM) | 0 |

| | Prescription | unit |
|---|---|---|
| | | |
| $D_{CRRT}$ | 30 | ml/kg/h |
| nNBL | 0.15 | mmol/h/kg |
| $D_{cit}$ | 3.1 | mmol/L |
| CaComp | 110 | % |
| $Q_{PFR}$ | 90 | ml/h |
| Qb | 130 | ml/min |
| *Degrees of freedom* | *6-6 = 0* | - |

Since the prescription parameter provides for 6 constraints, there is no degree of freedom available to further set additional therapy configurations.

With the above apparatus set, the control unit determines the following operating flow rates ($Q_b$ is indicated, but it is a prescription parameter already set to 130 ml/min).

| Operating flow rate parameters (ml/h) | |
|---|---|
| $Q_{dial}$ (ml/h) | 780 |
| $Q_{cit}$ (ml/h) | 1343 |
| $Q_{cal}$ (ml/h) | 15.9 |
| $Q_{rep.post}$ (ml/h) | 800 |
| $Q_{eff}$ (ml/h) | 3030 |

## Example C6

Similar simulation as done between examples C2 and C1 is repeated here between C6 and C5.

Changes between C6 and C5 are those highlighted in next table.

Therapy configuration is set as per the following table. Treatment configuration data are requested and entered according to the second table below reported.

| Therapy configuration | |
|---|---|
| CRRT Dose | Urea clearance |
| Acid-base balance target | nNBL ($Cp_{HCO3\_pat}$ 0=25 mM) |
| Blood flow rate | Prescription |

| Treatment configuration | |
|---|---|
| BW (kg) | 85 |
| Hct (%) | 27 |
| $K0.A_{HCO3}$ (ml/min) | 240 |
| $K0.A_{cit}$ (ml/min) | 109 |
| $C_{cit\_PBP}$ (mM) | 18 |
| $C_{ca}$ (mM) | 350 |
| $C_{HCO3dial}$ (mM) | 22 |
| $C_{HCO3rep.post}$ (mM) | 30 |
| $C_{ca rep.post}$ (mM) | 0 |

| Prescription | | unit |
|---|---|---|
| $D_{CRRT}$ | 30 | ml/kg/h |
| nNBL | 0.15 | mmol/h/kg |
| $D_{cit}$ | 2.8 | mmol/L |
| CaComp | 110 | % |
| $Q_{PFR}$ | 90 | ml/h |
| $Q_b$ | 130 | ml/min |
| *Degrees of freedom* | *6-6 = 0* | - |

Since the prescription parameter provides for 6 constraints, there is no degree of freedom available to further set additional therapy configurations.

With the above apparatus set, the control unit determines the following operating flow rates ($Q_b$ is indicated, but it is a prescription parameter already set to 130 ml/min).

| Operating flow rate parameters (ml/h) | |
|---|---|
| $Q_{dial}$ (ml/h) | 570 |
| $Q_{cit}$ (ml/h) | 1213 |
| $Q_{cal}$ (ml/h) | 16.1 |
| $Q_{rep.post}$ (ml/h) | 1090 |
| $Q_{eff}$ (ml/h) | 2980 |

Examples C5 and C6 shows the major impact of blood predilution when using 'diluted' citrate solutions and referring to a CRRT dose integrating the impact of predilution, like urea clearance dose. Effluent flow rate is significantly increased between examples C5-C6 and C1-C2 (about 400 to 500 ml/h).

In parallel, these examples show the major adjustments of dialysate and post-replacement flow rates required to maintain the desired acid-base balance while increasing the CRRT dose.

### Bicarbonate post-infusion system configuration

This section provides for assisted prescription examples in the context of a system where a concentrated bicarbonate solution is infused post filter and contributes to a major part of all bicarbonate infused through the CRRT therapy (via all fluids including dialysate). Ideally, this major part is more than 50% and may reached 100%; in the latter case, this means that all fluids are bicarbonate free, except the concentrated bicarbonate infusion.

Purpose of the system configuration is to decouple as much as possible acid-base balance prescription from CRRT dose and/or citrate dose prescriptions, providing thus for more freedom in varying these parameters independently. All examples provided in this section are built using a CRRT dose based on urea clearance, and thus factoring the impact of predilution.

*Examples in no or systemic anticoagulation*

The system configuration used throughout the next examples is comparable to the system configuration considered in examples S1 to S5 for the number of fluid pumps (n=4) and the presence of one infusion circuit in pre-dilution and one in post-dilution; it however includes several changes as reported in next table.

The CRRT system definition referred to examples BS1 to BS2 (BS stands for bicarbonate + systemic or no anticoagulation) is shown in the following table and also shown in figure 4.

| Fluid circuit / pump | Symbol | Present |
|---|---|---|
| dialysis fluid pump/supply line | Dial | X |
| PBP infusion pump/line | PBP | absent or no use |

(continued)

| Fluid circuit / pump | Symbol | Present |
|---|---|---|
| pre-dilution infusion pump/line | rep.pre | X |
| post-dilution infusion pump/line | rep.post | absent or no use |
| post-dilution bicarbonate infusion line/pump | HCO3 | X |
| effluent pump/line | eff | X |
| blood pump | b | X |

**Example BS1**

This example is comparable to example S5, but for the composition of fluids and the therapy configuration parameter.

Therapy configuration is set as per the following table. Treatment configuration data are requested and entered according to the second table below reported.

| Therapy configuration | |
|---|---|
| CRRT Dose | Urea clearance |
| Acid-base balance target | nNBL ($Cp_{HCO3\_pat}$ 0=25 mM) |
| Blood flow rate | Prescription |
| **Convection/diffusion split** | **50-50** |

| Treatment configuration | |
|---|---|
| BW (kg) | 75 |
| Hct (%) | 32 |
| KO.A (ml/min) | 240 |
| $C_{HCO3dial}$ (mM) | 0 |
| $C_{HCO3rep.pre}$ (mM) | 22 |
| $C_{HCO3HCO3\ post}$ (mM) | 140 |

| Prescription | | unit |
|---|---|---|
| $D_{CRRT}$ | 28 | ml/kg/h |
| nNBL | 0.15 | mmol/L |
| $Q_{PFR}$ | 120 | ml/h |
| $Q_b$ | 200 | ml/min |
| *Degrees of freedom* | *5-4 = 1* | - |

Since one degree of freedom is available, one additional constraint may be selected, in the present example, convetion diffusion split (or ratio) is selected and set to 50%-50% or R1=1.

Given the above clinical prescription parameters and the additional user selection of therapy configuration, the control unit 12 determines the following operating parameters ($Q_b$ being part of the prescription):

| Operating flow rate parameters (ml/h) | |
|---|---|
| $Q_{dial}$ (ml/h) | 1050 |
| $Q_{rep.pre}$ (ml/h) | 660 |

(continued)

| Operating flow rate parameters (ml/h) | |
|---|---|
| $Q_{HCO3}$ (ml/h) | 390 |
| $Q_{eff}$ (ml/h) | 2220 |

**Example BS2**

This example is comparable to previous and targets a much higher net buffer load. Changes from previous example BS1 are highlighted with backgrounded cells.

Therapy configuration is set as per the following table. Treatment configuration data are requested and entered according to the second table below reported.

| Therapy configuration | |
|---|---|
| CRRT Dose | Urea clearance |
| Acid-base balance target | nNBL ($Cp_{HCO3\_pat}$ 0=25 mM) |
| Blood flow rate | Prescription |
| **Convection/diffusion split** | **50-50** |

| Treatment configuration | |
|---|---|
| BW (kg) | 75 |
| Hct (%) | 32 |
| K0.A (ml/min) | 240 |
| $C_{HCO3dial}$ (mM) | 0 |
| $C_{HCO3rep.pre}$ (mM) | 22 |
| $C_{HCO3HCO3post}$ (mM) | 140 |

The clinical prescription parameters are the same as in the previous example BS1, but the nNBL is changed from 0.15 to a sensibly higher 0.25 value.

| Prescription | | unit |
|---|---|---|
| $D_{CRRT}$ | 28 | ml/kg/h |
| nNBL | 0.25 | mmol/L |
| $Q_{PFR}$ | 120 | ml/h |
| $Q_b$ | 200 | ml/min |
| *Degrees of freedom* | *5-4 = 1* | - |

The calculated flow rates for the remaining operating parameters are as follows ($Q_b$ being still part of the prescription):

| Operating flow rate parameters (ml/h) | |
|---|---|
| $Q_{dial}$ (ml/h) | 1050 |
| $Q_{rep.pre}$ (ml/h) | 595 |
| $Q_{HCO3}$ (ml/h) | 455 |
| $Q_{eff}$ (ml/h) | 2220 |

In the context of a system having only bicarbonate infusion as post-infusion, it is not relevant / possible to include therapy parameters such pre-post infusion ratio, as the post infusion rate is dictated by the acid-base balance prescription. In other terms, selection of pre-post infusion ratio would have caused the control unit to return no solution to the equation system. Therefore, selection of convection/diffusion ratio appears to be the exclusively reasonable selection among therapy additional constraints.

Comparison of examples BS1 and BS2 illustrates the strong and direct dependence of acid-base balance equilibrium on the bicarbonate post infusion flow rate, in a configuration where more than 80% of total bicarbonate infusion comes from a single source.

Notably, the 'high' nNBL level prescription of example BS2 cannot be provided in the conventional system and bicarbonate fluid configurations of examples S4-S5, unless using extremely high CRRT prescribed dialysis dose (e.g. about 50 ml/kg/h).

### Examples in citrate anticoagulation

The concept of bicarbonate infusion is further illustrated in the context of citrate anticoagulation (RCA).

Selected system configuration is comparable to the configuration of examples C1-C6 with the addition of a bicarbonate post-infusion pump. This system is thus including six fluid pumps and one blood pump (see the example of figure 5).

CRRT system definition for examples BC1 to BC3 (BC stands for bicarbonate + citrate anticoagulation) is indicated in the following table:

| Fluid circuit / pump | Symbol | Present |
|---|---|---|
| dialysis fluid pump/supply line | Dial | X |
| anticoagulant pump/infusion line (citrate) | Cit | X |
| ion balancing pump/infusion line (calcium) | Cal | X |
| pre-dilution infusion pump/fluid line | rep.pre | Absent - no use |
| post-dilution infusion pump/fluid line | rep.post | X |
| post-dilution bicarbonate infusion pump/fluid line | $HCO_3$ | X |
| Effluent pump/line | Eff | X |
| Blood pump | b | X |

See figure 5 for reference to the used circuit configuration.

### Example BC1

This example is similar to previous example C5, but for the addition of the bicarbonate post infusion and the use of buffer-free dialysate.

Therapy configuration is set as per the following table. Treatment configuration data are requested and entered according to the second table below reported.

| Therapy configuration | |
|---|---|
| CRRT Dose | Urea clearance |
| Acid-base balance target | nNBL ($Cp_{HCO3\_pat}$ 0=25 mM) |
| Blood flow rate | Prescription |
| **Convection/diffusion split** | **50-50** |

| Treatment configuration | |
|---|---|
| BW (kg) | 85 |
| Hct (%) | 27 |

(continued)

| Treatment configuration | |
|---|---|
| K0.A$_{HCO3}$ (ml/min) | 240 |
| K0.A$_{cit}$ (ml/min) | 109 |
| $C_{cit\_PBP}$ (mM) | 18 |
| $C_{ca}$ (mM) | 350 |
| $C_{HCO3dial}$ (mM) | 0 |
| $C_{HCO3rep.post}$ (mM) | 30 |
| $C_{carep.post}$ (mM) | 0 |
| $C_{HCO3HCO3post}$ (mM) | 140 |

As below indicated, $Q_b$ is still a prescription parameter in addition to the prescribed dialysis dose ($D_{set}$ - urea clearance) to be delivered, to the normalized net buffer load, to the patient fluid removal rate ($Q_{PFR}$) and the RCA prescriptions:

| Prescription | | unit |
|---|---|---|
| D$_{CRRT}$ | 30 | ml/kg/h |
| nNBL | 0.15 | mmol/h/kg |
| D$_{cit}$ | 3.1 | mmol/L |
| CaComp | 110 | % |
| Q$_{PFR}$ | 90 | ml/h |
| Q$_b$ | 130 | ml/min |
| *Degrees of freedom* | *7-6 = 1* | - |

There is one remaining degree of freedom based on the six prescription parameters and the seven flow rates to be determined. However, the selected convection-diffusion split cannot be reached given the system configuration and the clinical prescription parameters:

| Operating flow rate parameters (ml/h) | | Closest to convection/diffusion target | With Qb as operating parameter |
|---|---|---|---|
| Q$_{dial}$ (ml/h) | | 1300 | 1520 |
| Q$_{cit}$ (ml/h) | | 1343 | 1033 |
| Q$_{cal}$ (ml/h) | | 15.2 | 14.8 |
| Q$_{rep.post}$ (ml/h) | No solution | 0 | 0 |
| Q$_{HCO3}$ (ml/h) | | 287 | 394 |
| Q$_{eff}$ (ml/h) | | 3036 | 3052 |
| *Qb (ml/min)* | | *130* | 100 |
| *Convection-diffusion split* | *N/A* | *57-43* | *50-50* |

The control unit 12 provides for the solution relating to flow rates for the operating parameter which is closest to the convection diffusion ratio selected. In the present case the convection diffusion ratio is 57%-43% (R1=1,33). As an alternative, $Q_b$ is moved from prescription parameter to operating parameter and the convection diffusion ratio is kept at 50%-50% as originally set. Resulting blood flow rate is reduced to 100 ml/min.

**Example BC2**

BC2 example is similar to BC1 with a change of the citrate solution towards a higher concentration. Changes from BC1 are highlighted.

Therapy configuration is set as per the following table. Treatment configuration data are requested and entered according to the second table below reported.

| Therapy configuration | |
|---|---|
| CRRT Dose | Urea clearance |

| Acid-base balance target | nNBL $(Cp_{HCO3\_pat}0=25 \text{ mM})$ |
|---|---|
| Blood flow rate | Prescription |
| **Convection/diffusion split** | **50-50** |
| ~~Predilution ratio~~ | ~~< 0.2~~ |
| ~~Pre/post infusion ratio~~ | ~~33-67~~ |
| | |
| | |
| | |
| | |

| Treatment configuration | |
|---|---|
| BW (kg) | 85 |

| Hct (%) | 27 |
|---|---|
| K0.A$_{HCO3}$ (ml/min) | 240 |
| K0.A$_{cit}$ (ml/min) | 109 |
| $C_{cit\_PBP}$ (mM) | 50 |
| $C_{ca}$ (mM) | 350 |
| $C_{HCO3_{dial}}$ (mM) | 0 |
| $C_{HCO3_{rep.post}}$ (mM) | 30 |
| $C_{ca_{rep.post}}$ (mM) | 0 |
| $C_{HCO3_{HCO3post}}$ (mM) | 140 |

Clinical prescription parameters are unaltered with respect to example BC1:

| Prescription | | unit |
|---|---|---|
| D$_{CRRT}$ | 30 | ml/kg/h |
| nNBL | 0.15 | mmol/h/kg |
| D$_{cit}$ | 3.1 | mmol/L |
| CaComp | 110 | % |
| Q$_{PFR}$ | 90 | ml/h |
| Q$_b$ | 130 | ml/min |
| *Degrees of freedom* | *7-6 = 1* | - |

With the change (increase) in anticoagulant bag concentration for citrate, the control unit 12 finds a solution for the operating flow rates without the need to change the set blood flow rate and/or changing the convection/diffusion split:

| Operating flow rate parameters (ml/h) | |
|---|---|
| Q$_{dial}$ (ml/h) | 1365 |
| Q$_{Cit}$ (ml/h) | 484 |
| Q$_{cal}$ (ml/h) | 15.7 |
| Q$_{rep.post}$ (ml/h) | 615 |
| Q$_{HCO3}$ (ml/h) | 161 |

(continued)

| Operating flow rate parameters (ml/h) | |
|---|---|
| $Q_{eff}$ (ml/h) | 2730 |
| | |

## Example BC3

BC3 illustrates the changes in flow rates when changing citrate dose prescription given in example BC2. Changes from BC2 are highlighted.

Therapy configuration is set as per the following table. Treatment configuration data are requested and entered according to the second table below reported.

| Therapy configuration | |
|---|---|
| CRRT Dose | Urea clearance |
| Acid-base balance target | nNBL ($Cp_{HCO3\_pat}$ 0=25 mM) |
| Blood flow rate | Prescription |
| **Convection/diffusion split** | **50-50** |

| Treatment configuration | |
|---|---|
| BW (kg) | 85 |
| Hct (%) | 27 |
| $K0.A_{HCO3}$ (ml/min) | 240 |
| $K0.A_{cit}$ (ml/min) | 109 |
| $C_{cit\_PBP}$ (mM) | 50 |
| $C_{ca}$ (mM) | 350 |
| $C_{HCO3dial}$ (mM) | 0 |
| $C_{HCO3rep.post}$ (mM) | 30 |
| $C_{carep.post}$ (mM) | 0 |
| $C_{HCO3HCO3post}$ (mM) | 140 |

As below indicated, citrate dose is raised from 3.1 to 3.5 mmol/L:

| Prescription | | unit |
|---|---|---|
| $D_{CRRT}$ | 30 | ml/kg/h |
| nNBL | 0.15 | mmol/h/kg |
| $D_{cit}$ | 3.5 | mmol/L |
| CaComp | 110 | % |
| $Q_{PFR}$ | 90 | ml/h |
| Qb | 130 | ml/min |
| *Degrees of freedom* | *7-6 = 1* | - |

The calculated operating parameters are as follows (blood flow rate being a prescription parameter):

| Operating flow rate parameters (ml/h) | |
|---|---|
| $Q_{dial}$ (ml/h) | 1375 |
| $Q_{cit}$ (ml/h) | 546 |
| $Q_{cal}$ (ml/h) | 15.7 |
| $Q_{rep.post}$ (ml/h) | 595 |
| $Q_{HCO3}$ (ml/h) | 128 |
| $Q_{eff}$ (ml/h) | 2750 |

Addition of the bicarbonate post-infusion pump adds one degree of freedom and enables to consider one therapy configuration constraint in example BC1 (when compared to C5). However use of a 'diluted' citrate solution drives for significant convective flow and makes difficult to achieve an HDF prescription with equal convection and diffusion flow rates.

Example BC2 documents the benefit of more concentrated citrate solution when targeting a CRRT therapy configuration with significant dialysis/diffusion contribution, further to the decrease of citrate solution flow rate (which contributes to convective fluid exchange).

Example BC3 indicates that changes in citrate dose (for the purpose of anticoagulation intensity adjustment) can be easily balanced via adjustment of the bicarbonate post-infusion rate in order to keep the same acid-base equilibrium target.

**Compatibility with other system features**

High level interactions exist between the assisted prescription feature and optional features and an advanced CRRT system.

Blood flow rate management: in the configuration where the system is equipped with a module optimising automatically the blood pump speed as a function of vascular access performance, the system should operate with blood flow defined as operating parameter, rather than prescription parameter. A maximum blood flow rate value should however be offered as an operator settable constraint. In these conditions, the control unit shall re-compute automatically the various flow settings each time the blood flow rate is adjusted by the 'blood pump management' module.

TMP monitoring/convection: in the configuration where the system is equipped with a module optimising automatically the convective flow rate as a function of TMP (or other pressure parameters), the assisted prescription system, namely the control unit shall re-compute automatically the various flow settings each time the convective flow rates are adjusted.

Dose Control: Assisted prescription module provides for the computation of flow rate settings, while the Dose Control system monitors the 'instantaneous' CRRT dose requirements along the therapy in order to achieve an overall CRRT dose clinical target over several days of treatment. The assisted prescription enhancement brought with the inclusion of acid-base balance prescription parameter makes the Dose Control system applicable to citrate anticoagulation in a safe and practical way.

**Mathematical relations**

Here below the main mathematical relations that the control unit 12 uses to link patient prescription parameters, treatment configuration parameters and operating parameters are reported. The mathematical relations are already described in detail in the previous sections of the present application. When the necessary and/or selected patient prescription parameters and treatment configuration parameters are received, the control unit 12 is configured to use the relevant mathematical equations to calculate the set flow rates of fluid in the lines for the operating parameters.

The control unit 12 may then output the set and calculated flow rates for acceptance to a user and, after acceptance, drive the various actuators (pumps) to obtain the set and calculated flow rates in the relevant lines. Alternatively, the control unit 12 may automatically set the actuators to achieve the set and calculated flow rates.

### Eq. 1 - Citrate flow rate mathematical relation

$$Q_{cit} = \frac{D_{cit}}{C_{cit\_PBP}} \cdot Q_b$$

### Eq. 2 - Calcium flow rate mathematical relation

$$Q_{ca} = \frac{CaComp \cdot J_{Ca}}{C_{ca}} - \frac{Q_{rep.post} \cdot C_{ca_{rep.post}}}{C_{ca}} - \frac{Q_{HCO3} \cdot C_{ca_{HCO3}}}{C_{ca}}$$

### Eq. 3 - Plasma flow rate mathematical relation

$$Qp = Q_b \cdot (1 - Hct)$$

### Eq. 4 - Plasma water flow rate mathematical relation

$$Qpw = Qp \cdot F_p = Q_b \cdot (1 - Hct) \cdot F_p$$

### Eq. 5 - Plasma water flow rate at filtration unit inlet mathematical relation

$$Qpw_{inlet} = Qpw + Q_{cit} + Q_{syr} + Q_{PBP} + Q_{rep.pre}$$
$$= Q_b \cdot (1 - Hct) \cdot Fp + Q_{cit} + Q_{syr} + Q_{PBP} + Q_{rep.pre}$$

### Eq. 6 - Blood water flow rate mathematical relation

$$Qbw = Q_b \cdot [(1 - Hct) \cdot F_p + Hct \cdot Frbc]$$

### Eq. 7 - Blood water flow rate at filtration unit inlet mathematical relation

$$Qbw_{inlet} = Q_{bw} + Q_{cit} + Q_{syr} + Q_{PBP} + Q_{rep.pre}$$
$$= Q_b \cdot [(1 - Hct) \cdot Fp + Hct \cdot Frbc] + Q_{cit} + Q_{syr} + Q_{PBP} + Q_{rep.pre}$$

### Eq. 8 - Ultrafiltration flow rate mathematical relation

$$Q_{fil} = Q_{PBP} + Q_{cit} + Q_{syr} + Q_{rep.pre} + Q_{rep.post} + Q_{HCO3} + Q_{PFR} + Q_{ca}$$

### Eq. 9 - Pre-post split

$$Q_{rep.pre} = PRE \cdot Q_{rep}$$

$$Q_{rep} = (Q_{rep.pre} + Q_{rep.post})$$

### Eq. 10 - Effluent flow rate mathematical relation

$$Q_{eff} = Q_{PBP} + Q_{cit} + Q_{syr} + Q_{dial} + Q_{rep.pre} + Q_{rep.post} + Q_{HCO3} + Q_{PFR} + Q_{ca}$$

### Eq. 11 - Urea clearance (purely diffusive mode)

$$Z = \frac{Q_{pw_{inlet}}}{Q_{dial}} \qquad\qquad NT = \frac{S/RT}{Q_{pw_{inlet}}}$$

$$D_{urea}\left(Q_{pw_{inlet}}; Q_{dial}\right) = K_{urea} = Q_{pw_{inlet}} \cdot \frac{e^{[NT\cdot(1-Z)]}-1}{e^{[NT\cdot(1-Z)]}-Z} \qquad\qquad \text{if } Z{\neq}1$$

$$D_{urea}\left(Q_{pw_{inlet}}; Q_{dial}\right) = K_{urea} = Q_{pw_{inlet}} \cdot \frac{NT}{NT+1} \qquad\qquad \text{if } Z{=}1$$

### Eq. 12 - Urea clearance (diffusive and/or convective mode)

$$\gamma = e^{\frac{SC_{urea}\cdot Q_{fil}}{(S/RT)_{urea}}} - 1$$

$$f_{urea} = \left(\frac{Q_{pw_{inlet}} - SC_{urea}\cdot Q_{fil}}{Q_{pw_{inlet}}} \cdot \frac{Q_{dial} + SC_{urea}\cdot Q_{fil}}{Q_{dial}}\right)^{\frac{1}{\gamma}}$$

$$D_{urea}\left(Q_{pw_{inlet}}; Q_{dial}; Q_{fil}\right) = K_{urea}$$

$$= \frac{Q_{pw_{inlet}}\cdot Q_{dial} - f_{urea}\cdot (Q_{pw_{inlet}} - SC_{urea}\cdot Q_{fil})\cdot (Q_{dial} + SC_{urea}\cdot Q_{fil})}{Q_{dial} - f_{urea}\cdot (Q_{pw_{inlet}} - SC_{urea}\cdot Q_{fil})}$$

### Eq. 13/14/15/15' - Dilution factors

$$F_{dilution_{blood}} = \frac{Q_b}{Q_b + Q_{rep.pre} + Q_{PBP} + Q_{cit} + Q_{syr}}$$

$$F_{dilution_{plasma}} = \frac{Q_p}{Q_p + Q_{rep.pre} + Q_{PBP} + Q_{cit} + Q_{syr}}$$

$$F_{dilution_{pw}} = \frac{Q_{pw}}{Q_{pw} + Q_{rep.pre} + Q_{PBP} + Q_{cit} + Q_{syr}}$$

$$F_{dilution_{bw}} = \frac{Q_{bw}}{Q_{bw} + Q_{rep.pre} + Q_{PBP} + Q_{cit} + Q_{syr}}$$

### Eq. 16 - Urea dose (corrected for pre-dilution)

$$D_{urea\_corr} = F_{dilution_{blood}}\cdot K_{urea} = \frac{Q_b}{Q_b + Q_{rep.pre} + Q_{PBP} + Q_{cit} + Q_{syr}}\cdot Q_{eff}$$

### Eq. 17 - Net buffer load mathematical relation

$$J_{buffer\_load} = J_{met\_cit} + J_{HCO3_{bal}} + J_{met\_lact} - J_{H+}$$

**Eq. 18/19/20 - Citrate load mathematical relations**

$$J_{citrate\_load} = J_{cit\_PBP} - J_{cit\_dial}$$

$$J_{cit\_PBP} = Q_{cit} \cdot C_{cit\_PBP} = D_{cit} \cdot Q_b$$

$$J_{cit\_dial} = K_{cit} \cdot Cpw_{cit\_inlet}$$

**Eq. 21/22/23/24/24' - Citrate clearance and inlet concentration mathematical relations**

$$K_{cit} = \frac{Qpw_{inlet} \cdot Q_{dial} - f_{cit} \cdot (Qpw_{inlet} - SC_{cit} \cdot Q_{fil}) \cdot (Q_{dial} + SC_{cit} \cdot Q_{fil})}{Q_{dial} - f_{cit} \cdot (Qpw_{inlet} - SC_{cit} \cdot Q_{fil})}$$

$$f_{cit} = \left(\frac{Qpw_{inlet} - SC_{cit} \cdot Q_{fil}}{Qpw_{inlet}} \cdot \frac{Q_{dial} + SC_{cit} \cdot Q_{fil}}{Q_{dial}}\right)^{\frac{1}{\gamma_{cit}}}$$

$$\gamma_{cit} = e^{\left(\frac{\frac{SC_{cit} \cdot Q_{fil}}{S}}{RT_{cit}}\right)} - 1$$

$$Cp_{cit\_pat} = \frac{J_{citrate\_load}}{K_{cit\_met}}$$

$$K_{cit\_met} = 700 \cdot \frac{BW}{72}$$

$$Qp \cdot Cp_{cit_{pat}} + J_{cit_{PBP}} = Qpw_{inlet} \cdot Cpw_{cit\_inlet}$$

$$J_{cit\_PBP} = Qpw_{inlet} \times Cpw_{cit\_inlet}$$

**Eq. 25/25' - Citrate load mathematical relation/s**

$$J_{citrate\_load} = D_{cit} \cdot Q_b \cdot \left(1 - \frac{K_{cit}}{Qpw_{inlet}}\right) \cdot \left(1 - \frac{1}{1 + \frac{K_{cit\_met}}{K_{cit}} \cdot \frac{Qpw_{inlet}}{Qp}}\right)$$

$$J_{citrate\_load} = D_{cit} \times Qb \times \left(1 - \frac{K_{cit}}{Qpw_{inlet}}\right)$$

### Eq. 26/26' - Bicarbonate balance mathematical relations

$$J_{HCO3\_bal} = J_{HCO3\_inf} - J_{HCO3\_eff}$$

$$J_{HCO3\_bal} = Q_{rep.pre} \cdot C_{HCO3_{rep.pre}} + Q_{rep.post} \cdot C_{HCO3_{rep.post}} + Q_{HCO3}$$

$$\cdot C_{HCO3_{HCO3post}} + Q_{cit} \cdot C_{HCO3_{cit}} + Q_{PBP} \cdot C_{HCO3_{PBP}} - K_{HCO3}$$

$$\cdot \left( Cpw_{HCO3_{inlet}} - C_{HCO3\_dial} \right) - Q_{fil} \cdot C_{HCO3\_dial}$$

### Eq. 27 - Bicarbonate infusion mathematical relation

$$J_{HCO3\_inf} = Q_{dial} \cdot C_{HCO3_{dial}} + Q_{cit} \cdot C_{HCO3_{cit}} + Q_{PBP} \cdot C_{HCO3_{PBP}} + Q_{rep.pre} \cdot C_{HCO3_{rep.pre}} + Q_{rep.post}$$

$$\cdot C_{HCO3_{rep.post}} + Q_{HCO3} \cdot C_{HCO3_{HCO3post}}$$

### Eq. 28 - Bicarbonate removal mathematical relation

$$J_{HCO3\_eff} = Q_{dial} \cdot C_{HCO3_{dial}} + K_{HCO3} \cdot \left( Cpw_{HCO3\_inlet} - C_{HCO3\_dial} \right) + Q_{fil} \cdot C_{HCO3\_dial}$$

### Eq. 29/30 - Bicarbonate clearance mathematical relations

$$K_{HCO3} = \frac{Qbw_{inlet} \cdot Q_{dial} - f_{HCO3} \cdot \left( Qbw_{inlet} - SC_{HCO3} \cdot Q_{fil} \right) \cdot \left( Q_{dial} + SC_{HCO3} \cdot Q_{fil} \right)}{Q_{dial} - f_{HCO3} \cdot \left( Qbw_{inlet} - SC_{HCO3} \cdot Q_{fil} \right)}$$

$$f_{HCO3} = \left( \frac{Qbw_{inlet} - SC_{HCO3} \cdot Q_{fil}}{Qbw_{inlet}} \cdot \frac{Q_{dial} + SC_{HCO3} \cdot Q_{fil}}{Q_{dial}} \right)^{\frac{1}{\gamma_{HCO3}}}$$

$$\gamma_{HCO3} = e^{\left( \frac{SC_{HCO3} \cdot Q_{fil}}{\left( \frac{S}{RT} \right)_{HCO3}} \right)} - 1$$

$$K_{HCO3} = Qeff$$

### Eq. 31 - Plasma water bicarbonate concentration equations

$$Cpw_{HCO3\_pat} = \frac{Cp_{HCO3\_pat}}{Fp}$$

$$Q_{bw} \cdot Cpw_{HCO3_{pat}} + Q_{cit} \cdot C_{HCO3_{cit}} + Q_{PBP} \cdot C_{HCO3_{PBP}} + Q_{rep.pre} \cdot C_{HCO3_{rep.pre}}$$

$$= Qbw_{inlet} \cdot Cpw_{HCO3\_inlet}$$

## Eq. 32 - Plasma water bicarbonate concentration at filter inlet mathematical relation

$$Cpw_{HCO3\_inlet} = \frac{Q_{bw} \cdot \dfrac{Cp_{HCO3_{pat}}}{Fp} + Q_{cit} \cdot C_{HCO3_{cit}} + Q_{PBP} \cdot C_{HCO3_{PBP}} + Q_{rep.pre} \cdot C_{HCO3_{rep.pre}}}{Qbw_{inlet}}$$

## Eq. 33/34/35/36 - Mathematical relations for lactate

$$J_{lact\_bal} = J_{lact\_inf} - J_{lact\_eff}$$

$$J_{lact\_inf} = Q_{dial} \cdot C_{lact\_dial} + Q_{cit} \cdot C_{lact_{cit}} + Q_{PBP} \cdot C_{lact_{PBP}} + Q_{rep.pre} \cdot C_{lact_{rep.pre}} + Q_{rep.post} \cdot C_{lact_{rep.post}}$$

$$J_{lact\_eff} = Q_{dial} \cdot C_{lact\_dial} + K_{lact} \cdot \left(Cpw_{lact\_inlet} - C_{lact\_dial}\right) + Q_{fil} \cdot C_{lact\_dial}$$

$$Cpw_{lact\_inlet} = \frac{Q_{bw} \cdot \dfrac{Cp_{lact\_pat}}{Fp} + Q_{rep.pre} \cdot C_{lact_{rep.pre}} + Q_{cit} \cdot C_{lact_{cit}} + Q_{PBP} \cdot C_{lact_{PBP}}}{Qbw_{inlet}}$$

## Eq. 37 - Patient citrate metabolic clearance

$$J_{met\_cit} = 3 \cdot J_{cit\_load}$$

## Eq. 38 - Acid infusion equation

$$J_{H+} = 3 \cdot J_{citric\_acid} = 3 \cdot Q_{cit} \cdot C_{cit\_PBP}$$

## Eq. 39/40/41 - Net buffer load mathematical relations

$$J_{buffer\_load} = 3 \cdot J_{cit\_load} + J_{HCO3\_bal} - 3 \cdot J_{citric\_acid}$$

$$J_{buffer\_load} = 3 \cdot J_{cit\_load} + J_{HCO3\_bal} + J_{lact\_bal} - 3 \cdot J_{citric\_acid}$$

$$nNBL = \frac{J_{buffer\_load}}{BW}$$

**Eq. 42/43/44/45/42'/44' - Steady state acid base balance indicator mathematical relations**

$$Cp_{HCO3\_pat} = \frac{Fp}{Qbw}$$

$$\cdot \left( Cpw_{HCO3_{inlet}} \cdot Qbw_{inlet} - Q_{rep.pre} \cdot C_{HCO3rep.pre} - Q_{cit} \cdot C_{HCO3_{cit}} - Q_{PBP} \right.$$

$$\left. \cdot C_{HCO3_{PBP}} \right)$$

$$Cpw_{HCO3\_inlet} = C_{HCO3\_dial} + \frac{J_{HCO3\_eff} - Q_{dial} \times C_{HCO3_{dial}} - Q_{fil} \times C_{HCO3\_dial}}{K_{HCO3}}$$

$$J_{HCO3\_eff} = J_{HCO3\_inf} - J_{HCO3_{bal}} = J_{HCO3\_inf} + J_{met\_cit} - J_{H+} - J_{buffer\_load}$$

$$J_{buffer\_load} = nNBL \times BW$$

$$Cp_{HCO3\_pat} = \frac{Fp}{Qbw}$$

$$\cdot \left( C_{HCO3\_dial} + \frac{J_{HCO3\_eff} - Q_{dial} \times C_{HCO3_{dial}} - Q_{fil} \times C_{HCO3\_dial}}{K_{HCO3}} \cdot Qbw_{inlet} \right.$$

$$\left. - Q_{rep.pre} \cdot C_{HCO3rep.pre} - Q_{cit} \cdot C_{HCO3_{cit}} - Q_{PBP} \cdot C_{HCO3_{PBP}} \right)$$

(Eq. 42")

$$J_{HCO3\_eff} = J_{HCO3\_inf} + J_{met\_cit} - J_{H+} - nNBL0 \times BW$$

(Eq. 44')

## Claims

1. A Continuous Renal Replacement Therapy (CRRT) apparatus comprising:

    a filtration unit (2) having a primary chamber (3) and a secondary chamber (4) separated by a semipermeable membrane (5);
    an extracorporeal blood circuit (17) having a blood withdrawal line (6) connected to an inlet of the primary chamber (3), and a blood return line (7) connected to an outlet of the primary chamber (3), said extracorporeal blood circuit (17) being configured for connection to a patient cardiovascular system;
    a blood pump (21) configured to control the flow of blood through the extracorporeal blood circuit (17);
    an effluent fluid line (13) connected to an outlet of the secondary chamber (4);
    one or more of further fluid lines selected in the group comprising:

        a pre-dilution infusion line (29) connected at one end thereof to the blood withdrawal line (6),
        a post-dilution infusion line (63) connected at one end thereof to the blood return line (7),
        a post-dilution bicarbonate infusion line (23) connected at one end thereof to the blood return line (7),
        a ion balancing infusion line (74) connected at one end thereof either to the blood return line (7) or to a patient catheter,
        a dialysis liquid supply line (8) connected at one end thereof to the inlet of the secondary chamber (4),

a pre-blood pump - PBP - infusion line (52) connected at one end thereof to the blood withdrawal line in a region of the blood withdrawal line which is positioned in use upstream from the blood pump (21), an anticoagulant infusion line (51) connected at one end thereof to the blood withdrawal line in a region of the blood withdrawal line which is positioned in use upstream from the blood pump (21), and a syringe fluid line (22) connected at one end thereof to the blood withdrawal line,

actuators for regulating the flow of fluid (24, 25, 26, 31, 53, 54, 65, 75) through said fluid lines (23, 8, 13, 29, 52, 51, 63, 74);
a memory (16) storing one or more mathematical relations; and
a control unit (12) connected to the memory (16) and to the actuators, the control unit being configured to execute a flow-rate setup procedure comprising:

- receiving a patient prescription comprising clinical prescription parameters, the receiving step including:

  ○ allowing entry of a target value for a parameter (nNBL; $Cp_{HCO3\_pat}$) indicative of a steady state acid-base balance in the blood of the patient who has to undergo a CRRT blood treatment,

- determining one or more operating parameters using said one or more mathematical relations, the determining of the operating parameters comprises calculating a set value of one or more fluid flow rates selected in the group including:

  • a fluid flow rate ($Q_{cit}$) through the anticoagulant infusion line (51),
  • a fluid flow rate ($Q_{PBP}$) through the PBP infusion line (52),
  • a fluid flow rate ($Q_{rep.pre}$) through the pre-dilution infusion line (29),
  • a fluid flow rate ($Q_{rep.post}$) through the post-dilution infusion line (63),
  • a fluid flow rate ($Q_{HCO3}$) through the post-dilution bicarbonate infusion line (23),
  • a fluid flow rate ($Q_{ca}$) through the ion balancing infusion line (74),
  • a blood fluid flow rate ($Q_b$) through the extracorporeal blood circuit (17),
  • a fluid flow rate ($Q_{syr}$) through a syringe fluid line (22),
  • a fluid flow rate ($Q_{dial}$) through the dialysis liquid supply line (8), and
  • a fluid flow rate ($Q_{eff}$) through the effluent fluid line (13),

wherein calculating the set value of one or more fluid flow rates is based at least on said target value for the parameter (nNBL; $Cp_{HCO3\_pat}$) indicative of a steady state acid-base balance in the blood.

2. The CRRT apparatus of claim 1, wherein receiving a patient prescription comprises allowing entry of a set value for a prescribed dialysis dose ($D_{set}$) to be delivered, and determining one or more operating parameters comprises determining at least two operating parameters, the determining of the operating parameters comprises calculating a set value of at least two fluid flow rates selected in the group, calculating the set value of at least two fluid flow rates is based at least on said set value of the prescribed dialysis dose ($D_{set}$) and on said target value for the parameter (nNBL; $Cp_{HCO3\_pat}$) indicative of a steady state acid-base balance in the blood, and
wherein the calculating the set value of at least two fluid flow rates is a step of calculating each set value of the at least two fluid flow rates is based at least on both said set value of the prescribed dialysis dose ($D_{set}$) and on said target value for the parameter (nNBL; $Cp_{HCO3\_pat}$) indicative of a steady state acid-base balance in the blood.

3. The CRRT apparatus of any one of the previous claims, wherein the parameter (nNBL) indicative of a steady state acid-base balance in the blood of the patient undergoing a CRRT treatment is a parameter function of a net buffer load (NBL) in the patient expected at a steady state, wherein the net buffer load is a sum of bicarbonate generation from bicarbonate precursor metabolism, such as citrate or lactate metabolism, bicarbonate balance in the extracorporeal blood circuit (17) and/or of bicarbonate infusions into the patient and/or of acid infusion in the extracorporeal blood circuit (17), such as citric acid infusion, in particular wherein the net buffer load is a normalized net buffer (nNBL) load in the patient expected at a steady state, particularly normalized over a patient body weight (BW), in more detail the parameter (nNBL) indicative of a steady state acid-base balance in the blood of the patient undergoing a CRRT treatment being the net buffer load (NBL) or the normalized net buffer load (nNBL):

$$\frac{J_{buffer\_load}}{BW} = nNBL = \frac{NBL}{BW}$$

4. The CRRT apparatus of any one of the previous claims, wherein the parameter (nNBL) indicative of a steady state acid-base balance in the blood of the patient is based on one or more of:

• an estimation of an amount per unit of time of bicarbonate generated from metabolism of the bicarbonate precursor infused to the patient, in particular of citrate ($J_{met\_cit}$) and/or lactate ($J_{met\_lact}$);
• a bicarbonate balance ($J_{HCO3\_bal}$) from the CRRT blood treatment to be delivered in terms of an amount per unit of time;
• a lactate balance ($J_{lact\_bal}$) from the CRRT blood treatment to be delivered in terms of an amount per unit of time;
• an acid infusion ($J_{H+}$) from citric acid contained in the fluid source in terms of an amount per unit of time;
wherein the parameter (nNBL) indicative of a steady state acid-base balance in the blood of the patient is an algebraic sum of the estimation of bicarbonate form precursor metabolism ($J_{met\_cit}$; $J_{lact}$), the bicarbonate balance ($J_{HCO3\_bal}$), and the acid infusion ($J_{H+}$), in particular the acid infusion ($J_{H+}$) being a negative term providing a loss in patient buffer,
in detail wherein the parameter (nNBL) indicative of a steady state acid-base balance in the blood of the patient is defined as follows:

$$nNBL = \frac{J_{buffer\_load}}{BW} = \frac{J_{met\_cit} + J_{HCO3\_bal} - J_{H+}}{BW}$$

alternatively, when also lactate balance is considered, the parameter (nNBL) indicative of a steady state acid-base balance in the blood of the patient is defined as follows:

$$nNBL = \frac{J_{buffer\_load}}{BW} = \frac{J_{met\_cit} + J_{HCO3\_bal} + J_{lact\_bal} - J_{H+}}{BW}$$

5. The CRRT apparatus of any one of the previous claims, wherein:

calculating the set value of the fluid flow rate ($Q_{cit}$) through the anticoagulant infusion line (51) is based at least on said target value for the parameter (nNBL; $Cp_{HCO3\_pat}$) indicative of a steady state acid-base balance in the blood and/or on said set value of the prescribed dialysis dose ($D_{set}$), particularly in case the set value for the blood fluid flow rate ($Q_b$) through the extracorporeal blood circuit (17) is determined by the control unit (12) as an operating parameter,
in particular wherein the control unit (12) calculates the set value of the fluid flow rate ($Q_{cit}$) through the anticoagulant infusion line (51) using said mathematical relations; and/or calculating the set value of the fluid flow rate ($Q_{PBP}$) through the PBP infusion line (52) is based at least on said target value for the parameter (nNBL; $Cp_{HCO3\_pat}$) indicative of a steady state acid-base balance in the blood and/or on said set value of the prescribed dialysis dose ($D_{set}$), in particular wherein the control unit (12) calculates the set value of the fluid flow rate ($Q_{PBP}$) through the PBP infusion line (52) using said mathematical relations; and/or
calculating the set value of the fluid flow rate ($Q_{rep.pre}$) through the pre-dilution infusion line (29) is based at least on said target value for the parameter (nNBL; $Cp_{HCO3\_pat}$) indicative of a steady state acid-base balance in the blood and/or on said set value of the prescribed dialysis dose ($D_{set}$), in particular wherein
the control unit (12) calculates the set value of the fluid flow rate ($Q_{rep.pre}$) through the pre-dilution infusion line (29) using said mathematical relations; and/or
calculating the set value of the fluid flow rate ($Q_{rep.post}$) through the post-dilution infusion line (63) is based at least on said target value for the parameter (nNBL; $Cp_{HCO3\_pat}$) indicative of a steady state acid-base balance in the blood and/or on said set value of the prescribed dialysis dose ($D_{set}$), in particular wherein
the control unit (12) calculates the set value of the fluid flow rate ($Q_{rep.post}$) through the post-dilution infusion line (63) using said mathematical relations; and/or
calculating the set value of the fluid flow rate ($Q_{HCO3}$) through the post-dilution bicarbonate infusion line (23) is based at least on said target value for the parameter (nNBL; $Cp_{HCO3\_pat}$) indicative of a steady state acid-base balance in the blood and/or on said set value of the prescribed dialysis dose ($D_{set}$), in particular wherein the control unit (12) calculates the set value of the fluid flow rate ($Q_{HCO3}$) through the post-dilution bicarbonate infusion line (23) using said mathematical relations; and/or calculating the set value of the fluid flow rate ($Q_{ca}$) through the ion balancing infusion line (74) is based at least on said target value for the parameter (nNBL; $Cp_{HCO3\_pat}$) indicative of a steady state acid-base balance in the blood and/or on said set value of the prescribed dialysis dose ($D_{set}$), in particular wherein
the control unit (12) calculates the set value of the fluid flow rate ($Q_{ca}$) through the ion balancing infusion line (74) using said mathematical relations; and/or

calculating the set value of the blood fluid flow rate ($Q_b$) through the extracorporeal blood circuit (17) is based at least on said target value for the parameter (nNBL; $Cp_{HCO3\_pat}$) indicative of a steady state acid-base balance in the blood and/or on said set value of the prescribed dialysis dose ($D_{set}$), in particular wherein the control unit (12) calculates the set value of the fluid flow rate ($Q_b$) through the extracorporeal blood circuit (17) using said mathematical relations; and/or

calculating the set value of the fluid flow rate ($Q_{dial}$) through the dialysis liquid supply line (8) is based at least on said target value for the parameter (nNBL; $Cp_{HCO3\_pat}$) indicative of a steady state acid-base balance in the blood and/or on said set value of the prescribed dialysis dose ($D_{set}$), in particular wherein

the control unit (12) calculates the set value of the fluid flow rate ($Q_{dial}$) through the dialysis liquid supply line (8) using said mathematical relations; and/or

calculating the set value of the fluid flow rate ($Q_{eff}$) through the effluent fluid line (13) is based at least on said target value for the parameter (nNBL; $Cp_{HCO3\_pat}$) indicative of a steady state acid-base balance in the blood and/or on said set value of the prescribed dialysis dose ($D_{set}$), in particular wherein the control unit (12) calculates the set value of the fluid flow rate ($Q_{eff}$) through the effluent fluid line (13) using said mathematical relations.

6. The CRRT apparatus of any one of the previous claims, wherein said one or more mathematical relations include a net buffer load mathematical relation linking the the parameter (nNBL; $Cp_{HCO3\_pat}$) indicative of a steady state acid-base balance in the blood with at least one, and particularly all, of:

   ◦ a bicarbonate balance ($J_{HCO3\_bal}$),
   ◦ a bicarbonate generated from metabolism of citrate ($J_{met\_cit}$), or a citrate load ($J_{cit\_load}$), in particular wherein the metabolism of citrate load leads to 3 moles of bicarbonate per mole of citrate at steady state, namely $J_{met\_cit} = 3 \cdot J_{cit\_load}$,
   ◦ a bicarbonate generated from metabolism of lactate ($J_{met\_lact}$), or a lactate balance ($J_{lact\_bal}$), in particular wherein the metabolism of lactate leads to 1 mole of bicarbonate per mole of lactate at steady state, namely $J_{met\_lact} = J_{lact\_bal}$,
   ◦ an acid infusion $J_{H+}$,

   optionally the net buffer load mathematical relation is:

$$J_{buffer\_load} = J_{met\_cit} + J_{HCO3_{bal}} + J_{met\_lact} - J_{H+}$$

   wherein the significance of the denotations is given in the Glossary; if any of the mass transfer rate is missing, the corresponding mass transfer term is absent from the mathematical relation, or the corresponding value is set to zero.

7. The CRRT apparatus of any one of the previous claims, wherein said one or more mathematical relations include a bicarbonate balance mathematical relation based on a bicarbonate balance ($J_{HCO3\_bal}$) from the CRRT blood treatment to be delivered in terms of an amount per unit of time, in particular wherein the bicarbonate balance ($J_{HCO3\_bal}$) is the difference between a bicarbonate infusion rate from the dialysis liquid supply line (8) and/or the infusion lines ($J_{HCO3\_inf}$) and the bicarbonate removal into dialysate ($J_{HCO3\_eff}$), in particular the bicarbonate balance mathematical relation is:

$$J_{HCO3\_bal} = J_{HCO3\_inf} - J_{HCO3\_eff}$$

   wherein the significance of the denotations is given in the Glossary; if any of the mass transfer rate is missing, the corresponding mass transfer term is absent from the mathematical relation, or the corresponding value is set to zero,

   optionally wherein bicarbonate infusion rate ($J_{HCO3\_inf}$) from the dialysis liquid supply line (8) is a function of the fluid flow rate ($Q_{dial}$) through the dialysis liquid supply line (8) and bicarbonate concentration ($C_{HCO3dial}$) in the dialysis fluid flowing in the dialysis liquid supply line (8), in particular the fluid flow rate ($Q_{dial}$) times the bicarbonate concentration ($C_{HCO3dial}$); and/or

   wherein bicarbonate infusion rate ($J_{HCO3\_inf}$) from the infusion lines is a function of the fluid flow rate/s ($Q_{rep.pre}$; $Q_{rep.post}$; $Q_{cit}$; $Q_{PBP}$; $Q_{HCO3}$; $Q_{ca}$) and the respective bicarbonate concentration ($C_{HCO3rep.pre}$; $C_{HCO3rep.post}$; $C_{HCO3cit}$; $C_{HCO3PBP}$; $C_{HCO3HCO3}$; $C_{HCO3ca}$), in particular the fluid flow rate/s ($Q_{rep.pre}$; $Q_{rep.post}$; $Q_{cit}$; $Q_{PBP}$; $Q_{HCO3}$; $Q_{ca}$) times the respective bicarbonate concentration ($C_{HCO3rep.pre}$; $C_{HCO3rep.post}$; $C_{HCO3cit}$; $C_{HCO3PBP}$; $C_{HCO3HCO3}$; $C_{HCO3ca}$).

8. The CRRT apparatus of the previous claim, wherein:

the bicarbonate removal into spent dialysate ($J_{HCO3\_eff}$) in the effluent line (13) is a function of the fluid flow rate ($Q_{dial}$) through the dialysis liquid supply line (8) and bicarbonate concentration ($C_{HCO3dial}$) in the dialysis fluid flowing in the dialysis liquid supply line (8), in particular the fluid flow rate ($Q_{dial}$) times the bicarbonate concentration ($C_{HCO3dial}$); and/or

wherein the bicarbonate removal into spent dialysate ($J_{HCO3\_eff}$) in the effluent line (13) is a function of bicarbonate concentration in the dialysis fluid ($C_{HCO3\_dial}$), in particular as a function of a difference between bicarbonate plasma water concentration at filter inlet ($Cpw_{HCO3\_inlet}$) and bicarbonate concentration in the dialysis fluid ($C_{HCO3\_dial}$); and/or

wherein the bicarbonate removal into spent dialysate ($J_{HCO3\_eff}$) in the effluent line (13) is a function of an ultrafiltration flow rate ($Q_{fil}$) in the filtration unit (2) and bicarbonate concentration ($C_{HCO3dial}$) in the dialysis fluid flowing in the dialysis liquid supply line (8), in particular the ultrafiltration flow rate ($Q_{fil}$) times the bicarbonate concentration ($C_{HCO3dial}$); and/or

wherein the bicarbonate removal into spent dialysate ($J_{HCO3\_eff}$) in the effluent line (13) is a function of a bicarbonate clearance ($K_{HCO3}$); and/or

wherein the bicarbonate removal into spent dialysate ($J_{HCO3\_eff}$) in the effluent line (13) is a function of the fluid flow rate ($Q_{eff}$) through the effluent fluid line (13), in particular wherein a bicarbonate clearance ($K_{HCO3}$) is estimated equal to said effluent fluid flow rate ($Q_{eff}$); and/or

wherein the bicarbonate removal into spent dialysate ($J_{HCO3\_eff}$) in the effluent line (13) is a function of a bicarbonate clearance ($K_{HCO3}$) and the bicarbonate clearance ($K_{HCO3}$) is a function of one or more flow rates, particularly including one or more of the fluid flow rate ($Q_{dial}$) through the dialysis liquid supply line (8), an ultrafiltration rate ($Q_{fil}$) in filtration unit (2), and a blood water flow rate ($Qbw_{inlet}$).

9. The CRRT apparatus of any one of the previous claims, wherein said one or more mathematical relations include a bicarbonate balance mathematical relation based on a bicarbonate balance ($J_{HCO3\_bal}$) from the CRRT blood treatment to be delivered in terms of an amount per unit of time, in particular the bicarbonate balance mathematical relation is:

$$J_{HCO3\_bal} = Q_{rep.pre} \cdot C_{HCO3_{rep.pre}} + Q_{rep.post} \cdot C_{HCO3_{rep.post}} + Q_{HCO3} \cdot C_{HCO3_{HCO3post}}$$
$$- K_{HCO3} \cdot \left( Cpw_{HCO3_{inlet}} - C_{HCO3_{dial}} \right) - Q_{fil} \cdot C_{HCO3\_dial}$$

wherein the significance of the denotations is given in the Glossary; if any of the infusion lines or the dialysis liquid supply line is missing, the corresponding flow rate is absent from the mathematical relation, or the corresponding flow rate is set to zero.

10. The CRRT apparatus of any one of the previous claims, wherein said one or more mathematical relations include a citrate load mathematical relation based on a citrate load ($J_{cit\_load}$) to the patient in terms of an amount per unit of time, in particular wherein the citrate load ($J_{cit\_load}$) is the difference between a citrate infusion rate ($J_{cit\_PBP}$) through the anticoagulant infusion line (51) and a citrate removal rate to effluent ($J_{cit\_dial}$) in the effluent line (13), in particular the citrate load mathematical relation is:

$$J_{citrate\_load} = J_{cit\_PBP} - J_{cit\_dial}$$

wherein the significance of the denotations is given in the Glossary; if any of the mass transfer rate is missing, the corresponding mass transfer term is absent from the mathematical relation, or the corresponding value is set to zero.

11. The CRRT apparatus of any one of the previous claims, wherein a citrate load ($J_{cit\_load}$) is alternatively a function of a citrate dose ($D_{cit}$) and the blood flow ($Qb$), namely according to $D_{cit} \cdot Q_b$, or a function of the citrate flow rate ($Q_{cit}$) in the anticoagulant line (51) and a total citrate concentration ($C_{cit\_pbp}$), namely according to $Q_{cit} \cdot C_{citPBP}$.

12. The CRRT apparatus of any one of the previous claims, wherein the control unit (12) is further configured to calculate the set values of at least two fluid flow rates by applying the clinical prescription parameters to said mathematical relations, wherein the clinical prescription parameters further comprises one or more of a fluid removal rate ($Q_{pfr}$) from the patient to said mathematical relations, a blood flow rate ($Q_b$) in the extracorporeal blood circuit (17), a prescribed dialysis dose ($D_{set}$) and the parameter (nNBL; $Cp_{HCO3\_pat}$) indicative of a steady state acid-base balance in the blood of the patient, a regional anticoagulation dose ($D_{cit}$) and an ion re-establishing solution parameter (CaComp; $D_{ca}$).

13. The CRRT apparatus of any one of the previous claims, wherein the parameter ($Cp_{HCO3\_pat}$) indicative of a steady state bicarbonate concentration in the blood of the patient is based on an estimated net buffer load ($J_{buffer\_load}/BW$) indicative of a steady state acid-base balance in the blood of the patient, wherein said one or more mathematical relations include steady state bicarbonate indicator mathematical relations, and wherein the steady state bicarbonate indicator mathematical relations link the parameter ($Cp_{HCO3\_pat}$) indicative of a steady state bicarbonate concentration in the blood of the patient with said at least two flow rates in the group.

14. The CRRT apparatus of any one of the previous claims, wherein said one or more mathematical relations include steady state bicarbonate indicator mathematical relations, and wherein the steady state bicarbonate indicator mathematical relations links the parameter ($Cp_{HCO3\_pat}$) indicative of a steady state bicarbonate concentration in the blood of the patient with one or more of the fluid flow rate ($Q_{rep.pre}$) through the pre-dilution infusion line (29), the fluid flow rate ($Q_{dial}$) through the dialysis liquid supply line (8), the fluid flow rate ($Q_{cit}$) through the anticoagulant infusion line (51), the fluid flow rate ($Q_{PBP}$) through the PBP infusion line (52), in particular links the parameter ($Cp_{HCO3\_pat}$) with the fluid flow rate ($Q_{rep.pre}$; $Q_{dial}$; $Q_{cit}$; $Q_{PBP}$) times the respective bicarbonate concentration ($C_{HCO3rep.pre}$; $C_{HCO3dial}$; $C_{HCO3cit}$; $C_{HCO3PBP}$) in the fluid flowing in the respective line.

15. The CRRT apparatus of any one of the previous claims, wherein said one or more mathematical relations include steady state bicarbonate indicator mathematical relations, and wherein the steady state bicarbonate indicator mathematical relations link the parameter ($Cp_{HCO3\_pat}$) indicative of a steady state bicarbonate concentration in the blood of the patient with:

    • the blood flow rate ($Q_b$), in particular a blood water flow rate ($Q_{bw}$) and/or a blood water flow rate ($Q_{bw\_inlet}$) at the inlet of the filtration unit (2); and/or
    • a bicarbonate concentration ($C_{HCO3dial}$) in a fluid flowing in the dialysis liquid supply line (8) and/or with a bicarbonate clearance ($K_{HCO3}$) of the filtration unit (2); and/or
    • a bicarbonate plasma water concentration $Cpw_{HCO3\_inlet}$ at filtration unit inlet; and/or
    • an ultrafiltration flow rate ($Q_{fil}$) in the filtration unit (2), in particular with the ultrafiltration flow rate ($Q_{fil}$) times a bicarbonate concentration ($C_{HCO3dial}$) in the fluid flowing in the dialysis liquid supply line (8); and/or
    • a bicarbonate removal ($J_{eff}$) into spent dialysate flowing in the effluent line (13).

EP 4 679 444 A2

FIG.1

94

FIG.2

FIG.3

EP 4 679 444 A2

FIG.4

FIG.5

# FIG.6

EP 4 679 444 A2

FIG.7

**REFERENCES CITED IN THE DESCRIPTION**

**Patent documents cited in the description**

- WO 2013030642 A
- EP 0678301 A
- US 8668825 B2
- EP 0547025 A
- EP 0658352 A
- EP 0920887 A